# EUROPÄISCHE PATENTSCHRIFT

(11) **EP 1 874 773 B1**
(45) Veröffentlichungstag und Bekanntmachung des Hinweises auf die Patenterteilung: **13.12.2017**
(21) Anmeldenummer: 06754738.0
(22) Anmeldetag: 13.04.2006
(51) Int. Cl.: C07D 471/06, C09B 5/62, C07D 221/18

(54) **HEXARYLEN- UND PENTARYLENTETRACARBONSÄUREDIIMIDE**
HEXARYLENE AND PENTARYLENE TETRACARBOXYLIC ACID DIIMIDES
DIIMIDES D'ACIDE HEXARYLENETETRACARBOXYLIQUE ET PENTARYLENETETRACARBOXYLIQUE

(30) Priorität: 19.04.2005 DE 102005018241
(43) Veröffentlichungstag der Anmeldung: 09.01.2008
(73) Patentinhaber: BASF SE, 67056 Ludwigshafen am Rhein (DE); Max-Planck-Gesellschaft zur Förderung der Wissenschaften e.V., 80539 München (DE)
(72) Erfinder: PSCHIRER, Neil Gregory, 55116 Mainz (DE); KOHL, Christopher, 55127 Mainz (DE); MÜLLEN, Klaus, 50939 Köln (DE)
(74) Vertreter: BASF IP Association
(86) Internationale Anmeldenummer: PCT/EP2006/061603
(87) Internationale Veröffentlichungsnummer: WO 2006/111511

(56) Entgegenhaltungen:
- EP-A- 0 596 292
- WO-A-96/22332
- QUANTE H ET AL: "QUATERRYLENEBIS(DICARBOXIMIDES)" ANGEWANDTE CHEMIE. INTERNATIONAL EDITION, WILEY VCH VERLAG, WEINHEIM, DE, Bd. 34, Nr. 12, 7. Juli 1995 (1995-07-07), Seiten 1323-1325, XP002002793 ISSN: 1433-7851
- KOHL C ET AL: "Bis(rylenedicarboximid)-a,d-1,5-diaminoan thraquinones as unique infrared absorbing dyes" CHEMICAL COMMUNICATIONS - CHEMCOM, ROYAL SOCIETY OF CHEMISTRY, GB, 2002, Seiten 2778-2779, XP002256670 ISSN: 1359-7345

## Beschreibung

Die vorliegende Erfindung betrifft neue Rylentetracarbonsäurediimide der allgemeinen Formel I 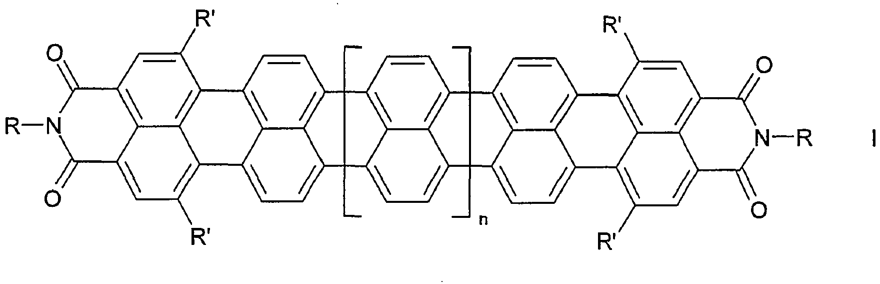 in der die Variablen folgende Bedeutung haben:
- R: gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, einoder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
- R': gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
- R¹: Wasserstoff oder C₁-C₁₈-Alkyl;
- R², R³: unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann;
- n: 1 oder 2,
sowie ihre Herstellung und ihre Verwendung zur Einfärbung von hochmolekularen organischen und anorganischen Materialien, zur Herstellung im nahinfraroten Bereich des elektromagnetischen Spektrums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-Laserstrahlenabsorbierende Materialien beim Schweißbehandeln von Kunststoffteilen sowie als Aktivkomponenten in der Photovoltaik.

Außerdem betrifft die Erfindung als Zwischenprodukte für die Rylentetracarbonsäurediimide I (R und R' haben in den folgenden Formeln, wenn nicht anders angegeben, jeweils die vorstehend genannte Bedeutung):
neue Bisterrylenderivate der allgemeinen Formel VII 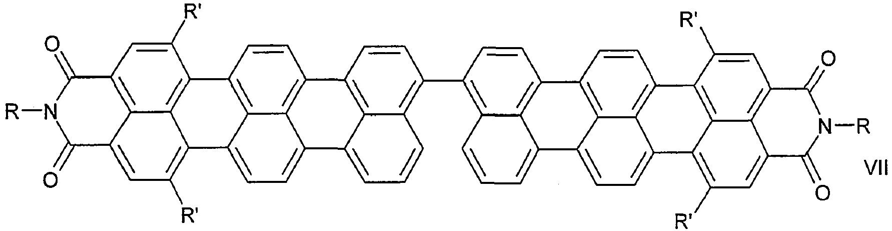
neue 11-(9-Perylen-3,4-dicarbonsäureimid)terrylen-3,4-dicarbonsäureimide der allgemeinen Formel VIII 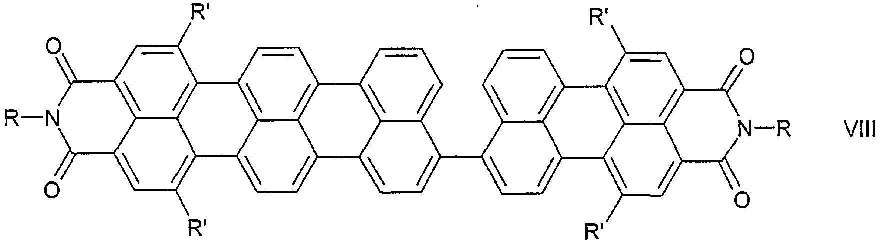
neue Terrylen-3,4-dicarbonsäureimide der allgemeinen Formel VI 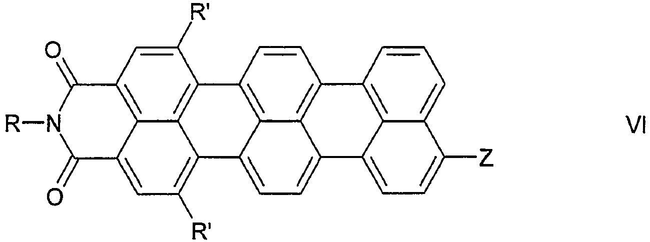 mit
   - Z: Brom, Iod, Amino, Nitro oder ein Rest

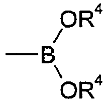

   - R⁴: gleiche oder verschiedene Reste:
   Wasserstoff, C₁-C₃₀-Alkyl, C₅-C₈-Cycloalkyl, Aryl oder Hetaryl, wobei die beiden Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können;
   - R': nicht Wasserstoff,
neue 9-(5-Nitronaphthyl)perylen-3,4-dicarbonsäureimide der allgemeinen Formel V 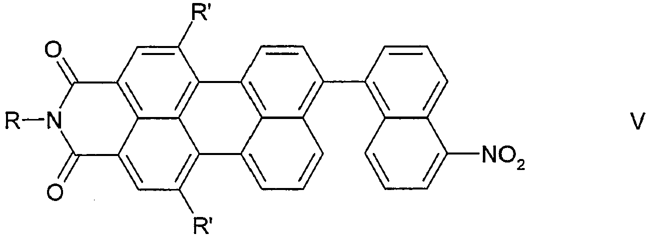
wobei R' nicht Wasserstoff bedeutet,
neue 13-(9-Perylen-3,4-dicarbonsäureimid)quaterrylen-3,4-dicarbonsäureimide der allgemeinen Formel XI 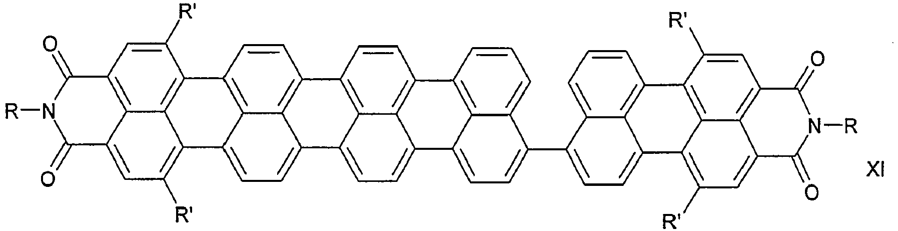
neue Perylen-3,9-bis(perylen-3,4-dicarbonsäureimide) der allgemeinen Formel Xa 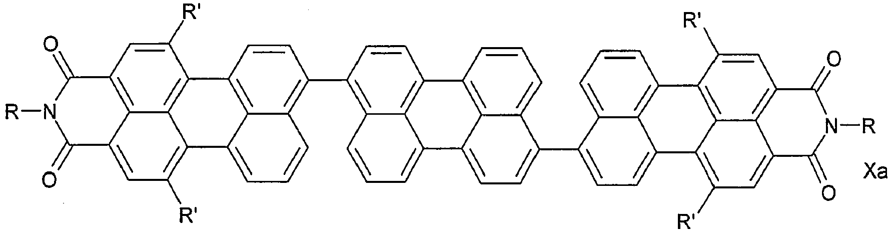
neue Perylen-3,10-bis(perylen-3,4-dicarbonsäureimide) der allgemeinen Formel Xb 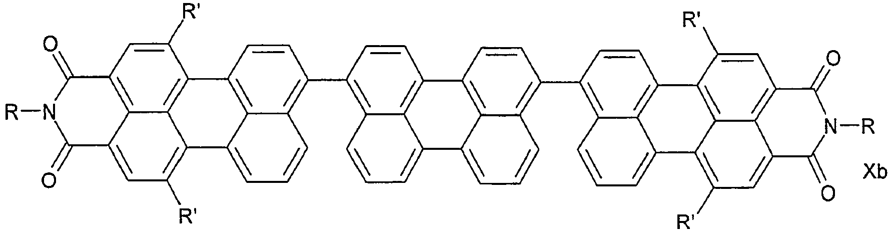
neue Bis(dioxaborolan-2-yl)perylene der allgemeinen Formel IXb 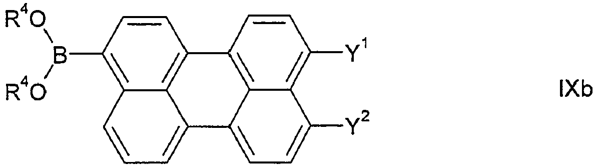
neue Naphthalin-1,5-bis(perylen-3,4-dicarbonsäureimide) der allgemeinen Formel Xlla 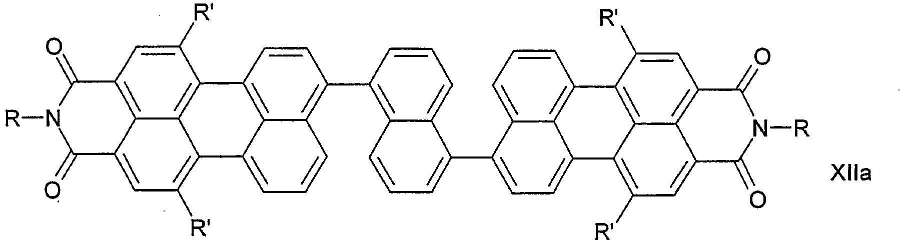
und neue Naphthalin-1,4-bis(perylen-3,4-dicarbonsäureimide) der allgemeinen Formel Xllb

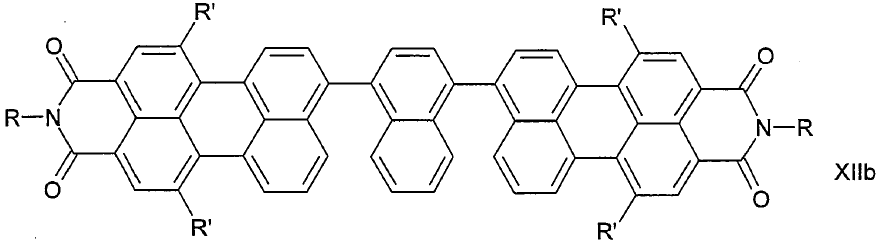

Höhere Rylentetracarbonsäurediimide sind bekanntermaßen aufgrund ihrer starken Absorption im Nahinfrarotbereich des elektromagnetischen Spektrums von besonderem anwendungstechnischen Interesse. So wird z.B. in der WO-A-02/77081 der Einsatz von Quaterrytentetracarbonsäurediimiden als Infrarotabsorber für den Wärmeschutz in Glaslaminaten beschrieben. WO 9622332 beschreibt Quaterrylentetracarbonsaürediimiden als Infrarotabsorber. Quaterrytentetracarbonsäurediimide weisen das größte bislang bekannte kondensierte Ringsystem auf.

Der Erfindung lag die Aufgabe zugrunde, Verbindungen bereitzustellen, deren Absorption in einem Bereich des elektromagnetischen Spektrums liegt, der noch längerwellig ist als der mit den bislang bekannten Rylentetracarbonsäurediimiden zugängliche Bereich.

Demgemäß wurden die Rylentetracarbonsäurediimide der eingangs definierten Formel I, nämlich die Hexarylentetracarbonsäurediimide der Formel Ia 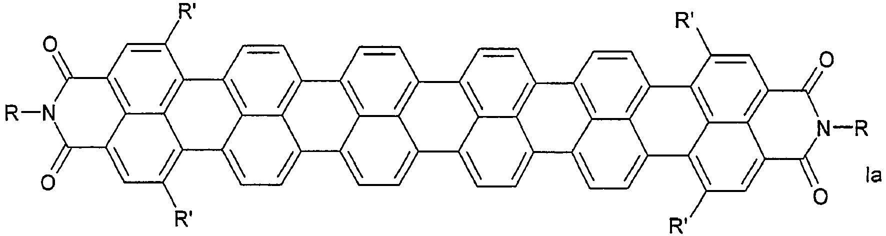 und die Pentarylentetracarbonsäurediimide der Formel Ib 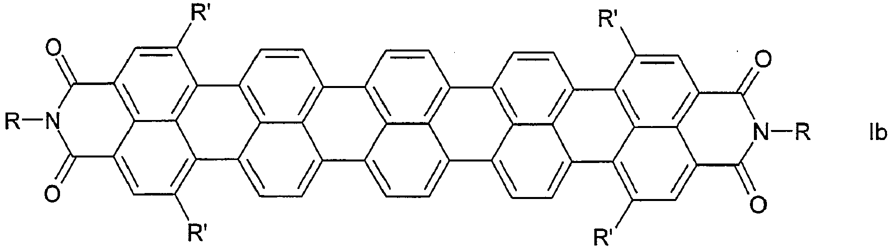 gefunden.

Bevorzugte Rylenimide I sind dem Unteranspruch zu entnehmen.

Weiterhin wurde ein Verfahren zur Herstellung der Hexarylentetracarbonsäurediimide der Formel Ia, in der R' nicht Wasserstoff bedeutet (im folgenden "Verfahren A1-Hexarylen" genannt), gefunden, welches dadurch gekennzeichnet ist, daß man
a) ein Diboran der allgemeinen Formel II 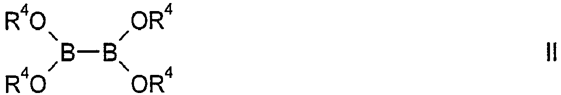 in der die Reste R⁴ die vorstehend genannte Bedeutung haben,
   in Gegenwart eines aprotischen organischen Lösungsmittels, eines Übergangsmetallkatalysators und einer Base mit
a1) einem 9-Bromperylen-3,4-dicarbonsäureimid der allgemeinen Formel IIIa 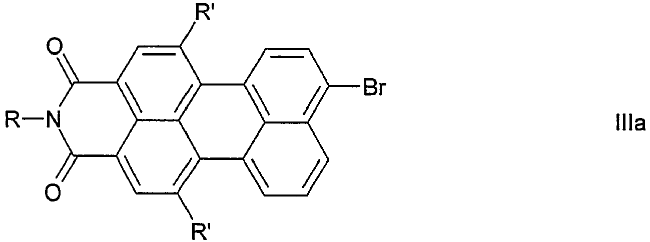 oder
a2) einem Naphthalinderivat der allgemeinen Formel IIIb 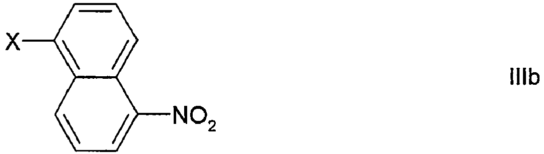 in der X Halogen, C₁-C₁₂-Alkylsulfonyl, dessen Alkylrest ein- oder mehrfach durch Halogen substituiert sein kann, oder C₆-C₁₈-Arylsulfonyl bedeutet, umsetzt,
b1) das in Schritt a1) gebildete 9-(Dioxaborolan-2-yl)perylen-3,4-dicarbonsäureimid der allgemeinen Formel IVa 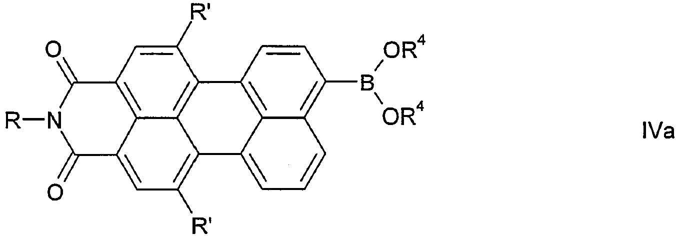 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Naphthalinderivat IIIb unterwirft
   oder
b2) das in Schritt a2) gebildete 1-(Dioxaborolan-2-yl)-5-nitronaphthalin der allgemeinen Formel IVb 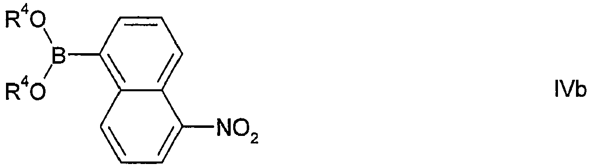 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid lila unterwirft,
c) das in Schritt b1) bzw. b2) gebildete 9-(5-Nitronaphthyl)perylen-3,4-dicarbonsäureimid der allgemeinen Formel V 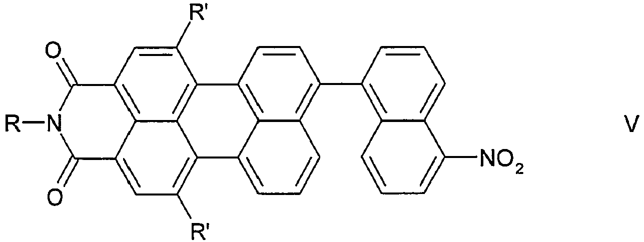 einer Cyclodehydrierung in Gegenwart eines basenstabilen, hochsiedenden, organischen Lösungsmittels sowie einer alkali- oder erdalkalimetallhaltigen Base und einer stickstoffhaltigen Hilfsbase unterzieht,
d) das in Schritt c) gebildete 11-Nitroterrylen-3,4-dicarbonsäureimid der allgemeinen Formel VIa 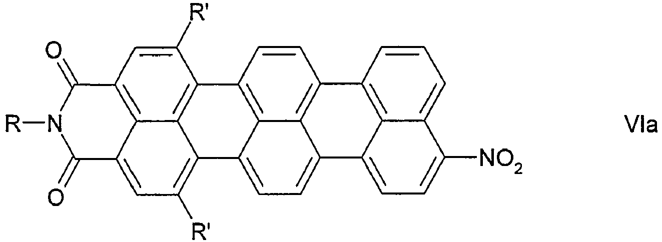 mit nascierendem Wasserstoff reduziert,
e) das in Schritt d) gebildete 11-Aminoterrylen-3,4-dicarbonsäureimid der allgemeinen Formel VIb 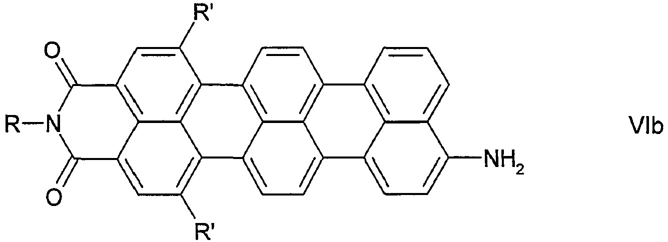 diazotiert und das gebildete Diazoniumsalz mit einem Metallbromid oder -iodid umsetzt,
f) das in Schritt e) gebildete 11-Halogenterrylen-3,4-dicarbonsäureimid der allgemeinen Formel VIc 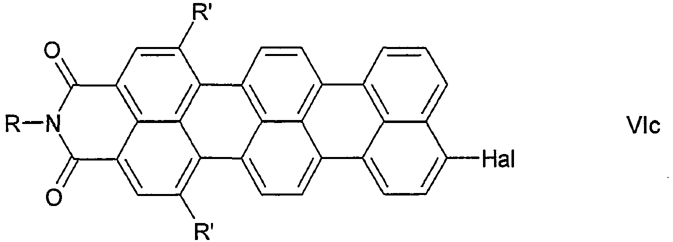 in der Hal Brom oder Iod bedeutet,
f1) in Gegenwart eines organischen Übergangsmetallkomplexes als Katalysator, freier Ligandmoleküle und eines aprotischen Verdünnungsmittels zu einem Bisterrylenderivat der allgemeinen Formel VII 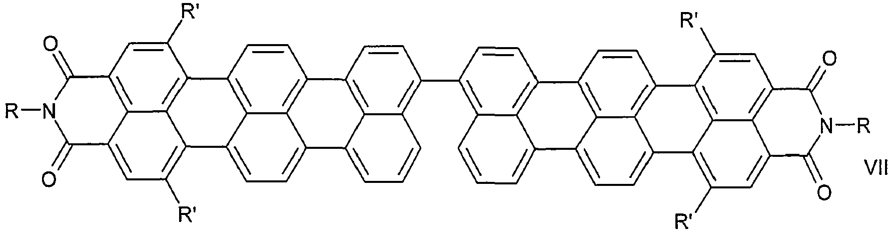 kuppelt
   oder
f2) in Gegenwart von 30 bis 70 mol-%, bezogen auf das 11-Halogenterrylen-3,4-dicarbonsäureimid VIc, eines Diborans II, eines Übergangsmetallkatalysators, einer Base und eines aprotischen Lösungsmittels ohne Zwischenisolierung des in situ gebildeten 11-(Dioxaborolan-2-yl)terrylen-3,4-dicarbonsäureimids der allgemeinen Formel VId 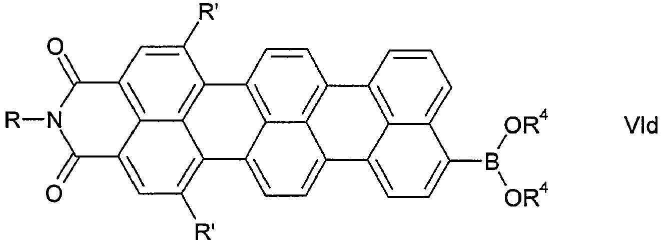 gemäß einer Suzuki-Kupplungsreaktion zum Bisterrylenderivat VII umsetzt und
g) das Bisterrylenderivat VII durch Cyclodehydrierung
g1) in einem Hydroxy- und Aminofunktionen aufweisenden und eine im wesentlichen ungelöste Base enthaltenden organischen Reaktionsmedium oder
g2) in Gegenwart eines basenstabilen, hochsiedenden, organischen Lösungsmittels sowie einer alkali- oder erdalkalimetallhaltigen Base und einer stickstoffhaltigen Hilfsbase
in das Hexarylentetracarbonsäurediimid Ia überführt.

Außerdem wurde ein Verfahren zur Herstellung der Hexarylentetracarbonsäurediimide der Formel Ia, in der R' nicht Wasserstoff bedeutet (im folgenden "Verfahren A2-Hexarylen" genannt), gefunden, welches dadurch gekennzeichnet ist, daß man
a) ein Diboran der allgemeinen Formel II 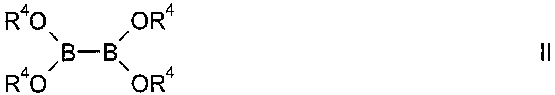 in der die Reste R⁴ die vorstehend genannte Bedeutung haben, in Gegenwart eines aprotischen organischen Lösungsmittels, eines Übergangsmetallkatalysators und einer Base mit einem 11-Halogenterrylen-3,4-dicarbonsäureimid der allgemeinen Formel Vlc 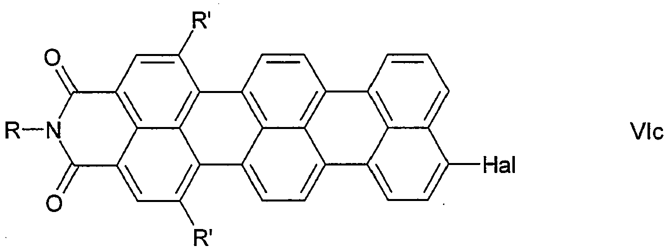 in der Hal Brom oder Iod bedeutet, umsetzt,
b) das in Schritt a) gebildete 11-(Dioxaborolan-2-yl)terrylen-3,4-dicarbonsäureimid der allgemeinen Formel VId 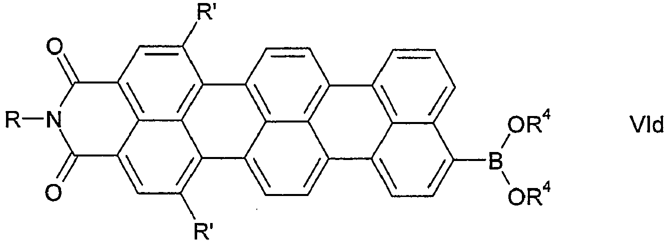 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 11-Halogenterrylen-3,4-dicarbonsäureimid VIc unterwirft und
c) das in Schritt b) gebildete Bisterrylenderivat der allgemeinen Formel VII 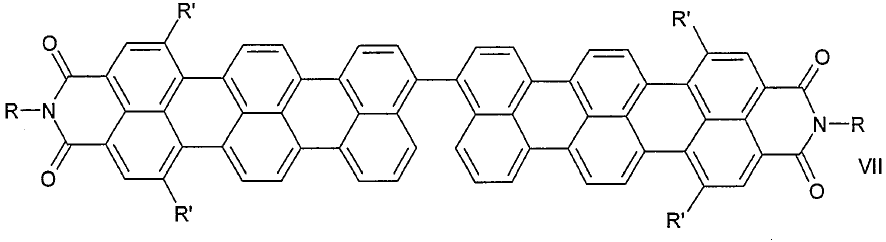 durch Cyclodehydrierung
c1) in einem Hydroxy- und Aminofunktionen aufweisenden und eine im wesentlichen ungelöste Base enthaltenden organischen Reaktionsmedium oder
c2) in Gegenwart eines basenstabilen, hochsiedenden, organischen Lösungsmittels sowie einer alkali- oder erdalkalimetallhaltigen Base und einer stickstoffhaltigen Hilfsbase
in das Hexarylentetracarbonsäurediimid la überführt.

Weiterhin wurde ein Verfahren zur Herstellung der Pentarylentetracarbonsäurediimide der Formel Ib, in der R' nicht Wasserstoff bedeutet (im folgenden "Verfahren A1-Pentarylen" genannt), gefunden, welches dadurch gekennzeichnet ist, daß man
a) das 11-Halogenterrylen-3,4-dicarbonsäureimid der Formel VIc 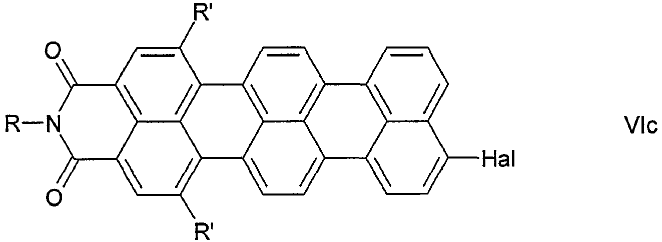 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-(Dioxaborolan-2-yl)perylen-3,4-dicarbonsäureimid der Formel IVa 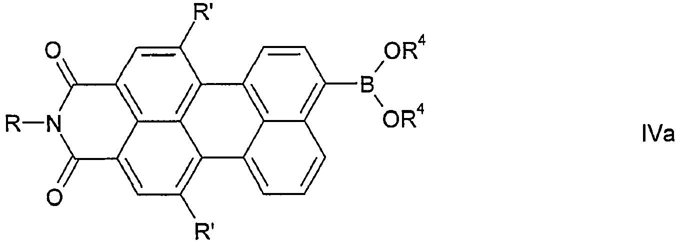 unterwirft und
b) das in Schritt a) gebildete 11-(9-Perylen-3,4-dicarbonsäureimid)terrylen-3,4-dicarbonsäureimid der allgemeinen Formel VIII 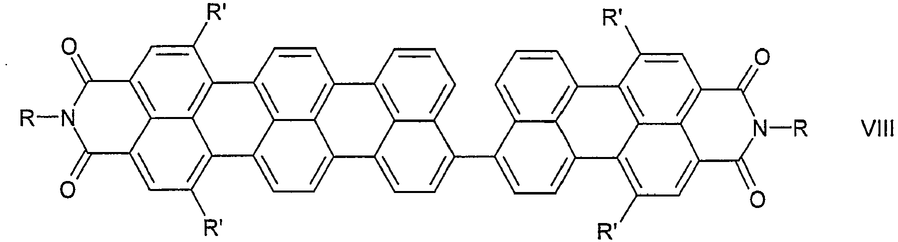 durch Cyclodehydrierung
b1) in einem Hydroxy- und Aminofunktionen aufweisenden und eine im wesentlichen ungelöste Base enthaltenden organischen Reaktionsmedium oder
b2) in Gegenwart eines basenstabilen, hochsiedenden, organischen Lösungsmittels sowie einer alkali- oder erdalkalimetallhaltigen Base und einer stickstoffhaltigen Hilfsbase
in das Pentarylentetracarbonsäurediimid Ib überführt.

Weiterhin wurde ein Verfahren zur Herstellung der Pentarylentetracarbonsäurediimide der Formel Ib, in der R' nicht Wasserstoff bedeutet (im folgenden "Verfahren A2-Pentarylen" genannt), gefunden, welches dadurch gekennzeichnet ist, daß man
a) ein 11-(Dioxaborolan-2-yl)terrylen-3,4-dicarbonsäureimid der Formel VId 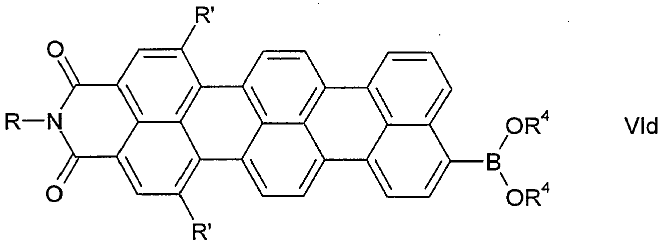 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid der Formel lila 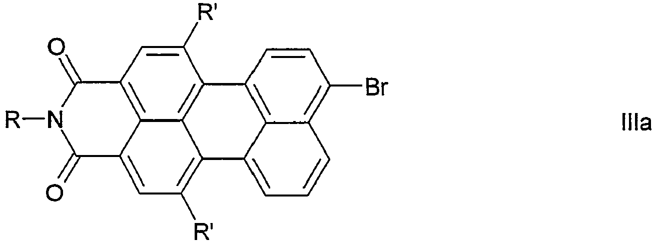 unterwirft und
b) das in Schritt a) gebildete 11-(9-Perylen-3,4-dicarbonsäureimid)terrylen-3,4-dicarbonsäureimid der Formel VIII 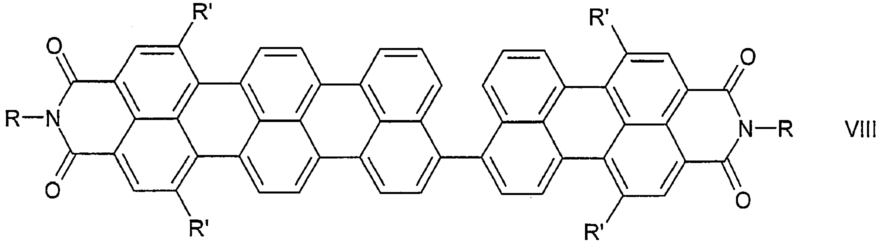 durch Cyclodehydrierung
b1) in einem Hydroxy- und Aminofunktionen aufweisenden und eine im wesentlichen ungelöste Base enthaltenden organischen Reaktionsmedium oder
b2) in Gegenwart eines basenstabilen, hochsiedenden, organischen Lösungsmittels sowie einer alkali- oder erdalkalimetallhaltigen Base und einer stickstoffhaltigen Hilfsbase
in das Pentarylentetracarbonsäurediimid Ib überführt.

Außerdem wurden die bei den Verfahren A als Zwischenprodukte auftretenden Bisterrylenderivate der eingangs definierten Formel VII 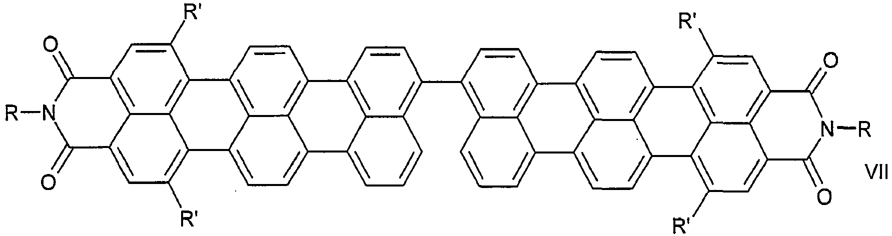 die 11-(9-Perylen-3,4-dicarbonsäureimid)terrylen-3,4-dicarbonsäureimide der eingangs definierten Formel VIII 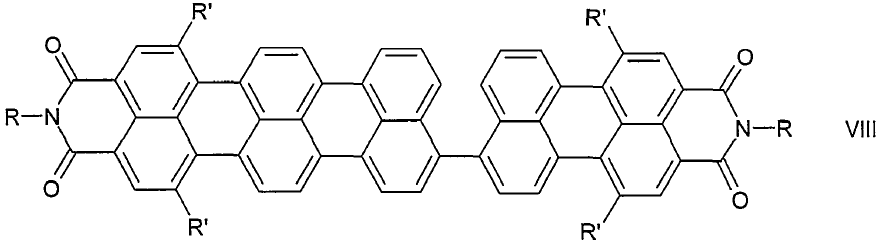 die Terrylen-3,4-dicarbonsäureimide der eingangs definierten Formel VI 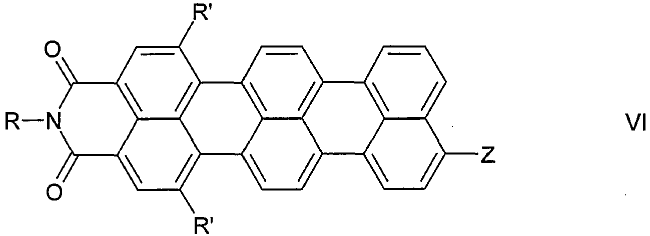 und die 9-(5-Nitronaphthyl)perylen-3,4-dicarbonsäureimide der eingangs definierten Formel V 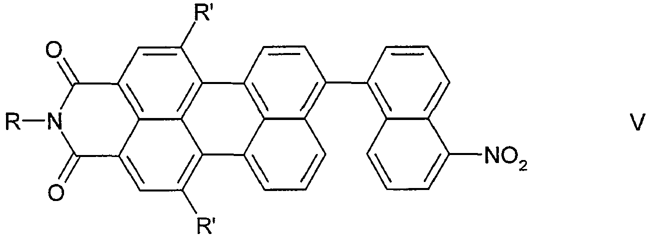 gefunden.

Schließlich wurde ein weiteres Verfahren zur Herstellung der Hexarylentetracarbonsäurediimide Ia (im folgenden "Verfahren B-Hexarylen" genannt) gefunden, welches dadurch gekennzeichnet ist, daß man
a) ein Diboran der Formel II 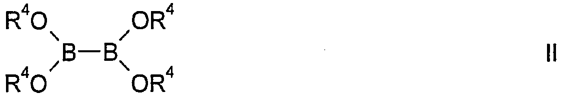 in Gegenwart eines aprotischen organischen Lösungsmittels, eines Übergangsmetallkatalysators und einer Base mit
a1) einem 9-Bromperylen-3,4-dicarbonsäureimid der Formel lila 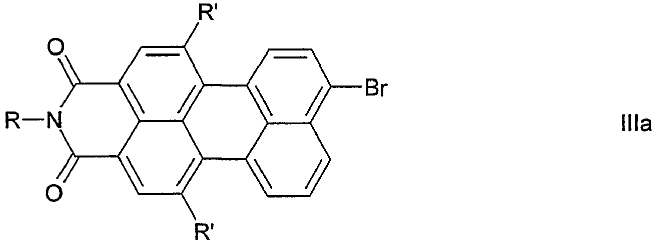 oder
a2) einem Dihalogenperylen der allgemeinen Formel IXa 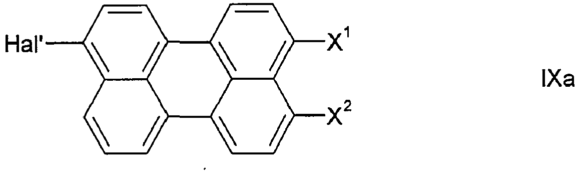 in der Hal' für Chlor oder Brom steht und einer der beiden Reste X¹ oder X² ebenfalls Hal' und der andere Rest Wasserstoff bedeutet, umsetzt,
b1) das in Schritt a1) gebildete 9-(Dioxaborolan-2-yl)perylen-3,4-dicarbonsäureimid der Formel IVa 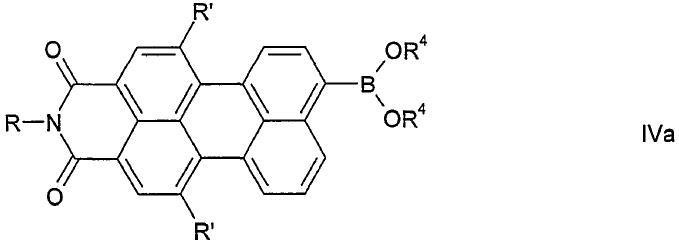 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Dihalogenperylen IXa im Molverhältnis 2 : 1 bis 6 : 1 unterwirft
   oder
b2) das in Schritt a2) gebildete Bis(dioxaborolan-2-yl)perylen der allgemeinen Formel IXb 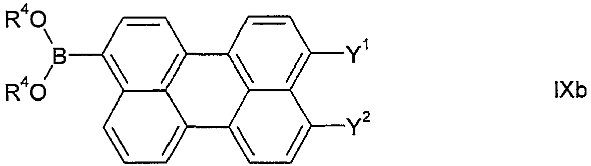 in der einer der beiden Reste Y¹ oder Y² ebenfalls einen Rest 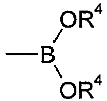 und der andere Rest Wasserstoff bedeutet,
   in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid IIIa im Molverhältnis 1 : 2 bis 1 : 6 unterwirft und
c) das in Schritt b1) bzw. b2) gebildete Perylen-3,9-bis(perylen-3,4-dicarbonsäureimid) der allgemeinen Formel Xa 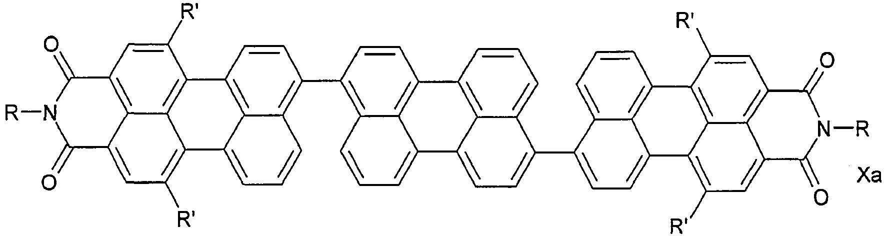 oder Perylen-3,10-bis(perylen-3,4-dicarbonsäureimid) der allgemeinen Formel Xb 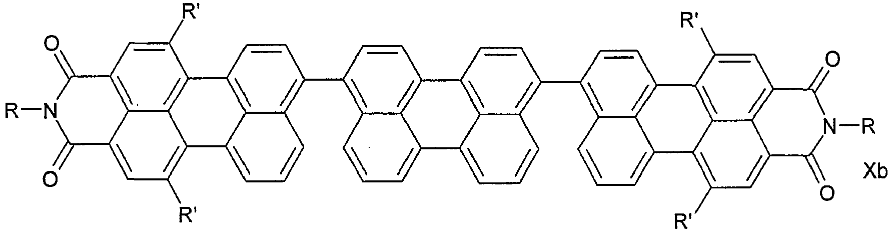
c1) einer einstufigen Cyclodehydrierung in Gegenwart einer starken Lewis-Säure und eines inerten organischen Lösungsmittels direkt zum Hexarylentetracarbonsäurediimid la unterzieht
   oder
c2a) in einem ersten Schritt bei Raumtemperatur in Gegenwart eines inerten organischen Lösungsmittels mit einer schwachen Lewis-Säure in Kontakt bringt
   und
c2b) das dabei gebildete 13-(9-Perylen-3,4-dicarbonsäureimid)quaterrylen-3,4-dicarbonsäureimid der allgemeinen Formel XI 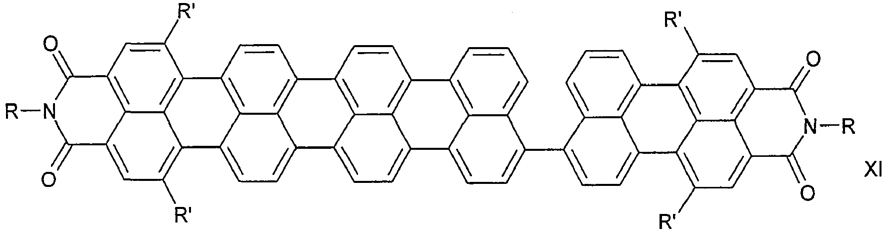 dann nach Zwischenisolierung in einem zweiten Schritt
c2bα)in einem Hydroxy- und Amionofunktionen aufweisenden und eine im wesentlichen ungelöste Base enthaltenden organischen Reaktionsmedium oder
c2bβ)in Gegenwart eines basenstabilen, hochsiedenden, organischen Lösungsmittels sowie einer alkali- oder erdalkalimetallhaltigen Base und einer stickstoffhaltigen Hilfsbase
weiter zum Hexarylentetracarbonsäurediimid Ia cyclodehydriert.

Außerdem wurde ein zu diesem Verfahren analoges Verfahren zur Herstellung der Pentarylentetracarbonsäurediimide Ib (im folgenden "Verfahren B-Pentarylen" genannt) gefunden, welches dadurch gekennzeichnet ist, daß man
a) ein Diboran der Formel II 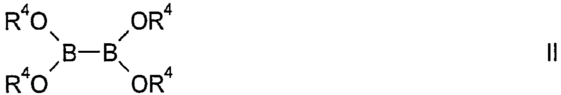 in Gegenwart eines aprotischen organischen Lösungsmittels, eines Übergangsmetallkatalysators und einer Base mit
a1) einem 9-Bromperylen-3,4-dicarbonsäureimid der Formel lila 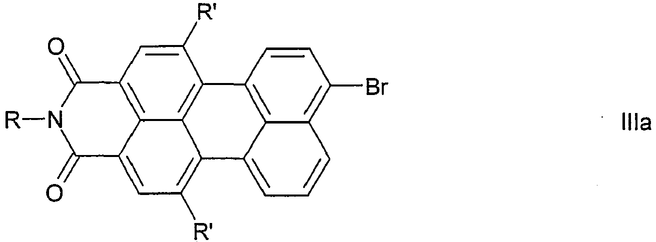 oder
a2) einem Dihalogennaphthalin der allgemeinen Formel IXc 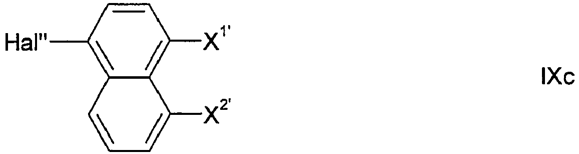 in der Hal" für Chlor, Brom oder Iod steht und einer der beiden Reste X¹' oder X²' ebanfalls Hal" und der andere Rest Wasserstoff bedeutet, umsetzt,
b1) das in Schritt a1) gebildete 9-(Dioxaborolan-2-yl)perylen-3,4-dicarbonsäureimid der Formel IVa 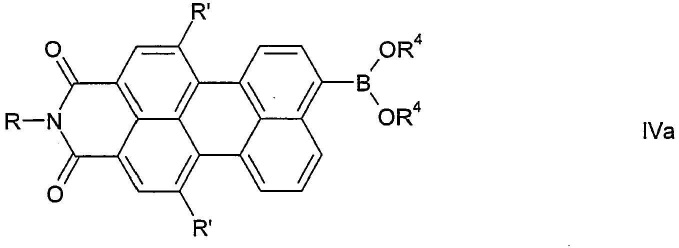 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Dihalogennaphthalin IXc im Molverhältnis 2 : 1 bis 6 : 1 unterwirft
   oder
b2) das in Schritt a2) gebildete Bis(dioxaborolan-2-yl)naphthalin der allgemeinen Formel IXd 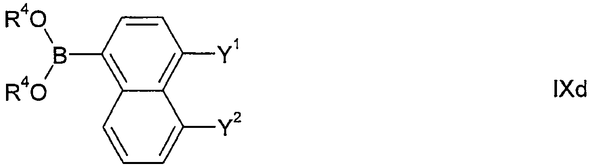 in der einer der beiden Reste Y¹ oder Y² ebenfalls einen Rest 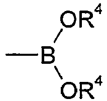 und der andere Rest Wasserstoff bedeutet, in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid lila im Molverhältnis 1 : 2 bis 1 : 6 unterwirft und
c) das in Schritt b1) bzw. b2) gebildete Naphthalin-1,5-bis(perylen-3,4-dicarbonsäureimid) der allgemeinen Formel Xlla 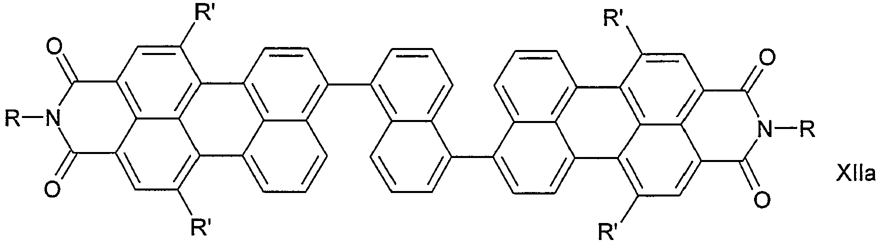 oder Naphthalin-1,4-bis(perylen-3,4-dicarbonsäureimid) der allgemeinen Formel Xllb 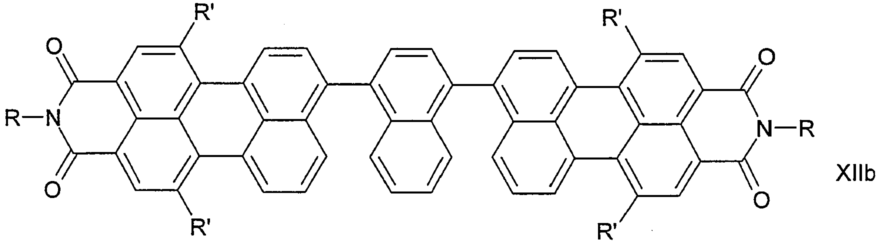
c1) einer einstufigen Cyclodehydrierung in Gegenwart einer starken Lewis-Säure und eines inerten organischen Lösungsmittels direkt zum Pentarylentetracarbonsäurediimid Ib unterzieht
   oder
c2a) in einem Schritt ohne Erhitzen bei Raumtemperatur in Gegenwart eines inerten organischen Lösungsmittels mit einer schwachen Lewis-Säure in Kontakt bringt
   und
c2b) das dabei gebildete 11-(9-Perylen-3,4-dicarbonsäureimid)terrylen-3,4-dicarbonsäureimid der Formel VIII 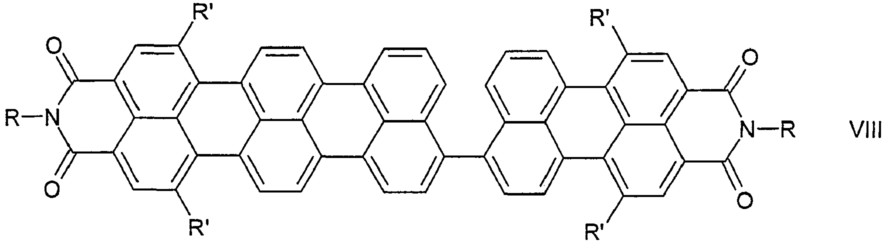 dann nach Zwischenisolierung in einem zweiten Schritt
c2bα)in einem Hydroxy- und Amionofunktionen aufweisenden und eine im wesentlichen ungelöste Base enthaltenden organischen Reaktionsmedium oder
c2bβ)in Gegenwart eines basenstabilen, hochsiedenden, organischen Lösungsmittels sowie einer alkali- oder erdalkalimetallhaltigen Base und einer stickstoffhaltigen Hilfsbase
   weiter zum Pentarylentetracarbonsäurediimid Ib cyclodehydriert.

Außerdem wurden die bei den Verfahren B als Zwischenprodukte auftretenden 13-(9-Perylen-3,4-dicarbonsäureimid)quaterrylen-3,4-dicarbonsäureimide der eingangs definierten Formel XI 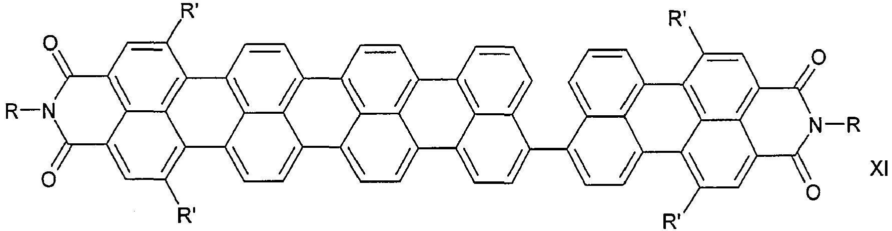 die Perylen-3,9-bis(perylen-3,4-dicarbonsäureimide) der eingangs definierten Formel Xa 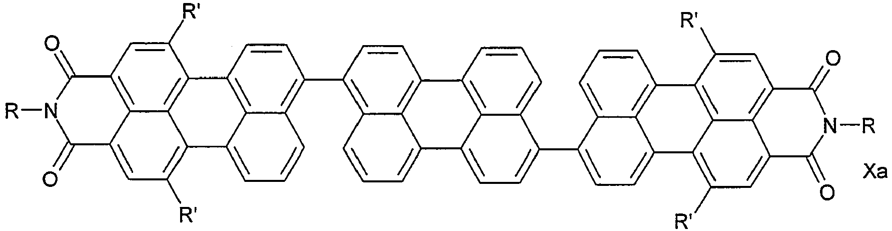 die Perylen-3,10-bis(perylen-3,4-dicarbonsäureimide) der eingangs definierten Formel Xb 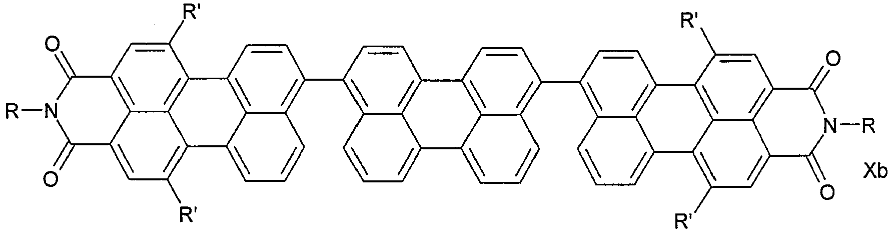 die Bis(dioxaborolan-2-yl)perylene der eingangs definierten Formel IXb 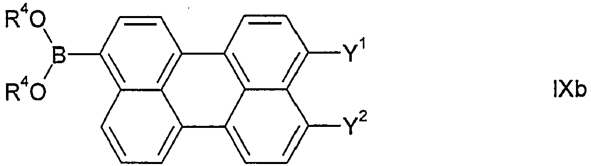 die Naphthalin-1,5-bis(perylen-3,4-dicarbonsäureimide) der eingangs definierten Formel Xlla 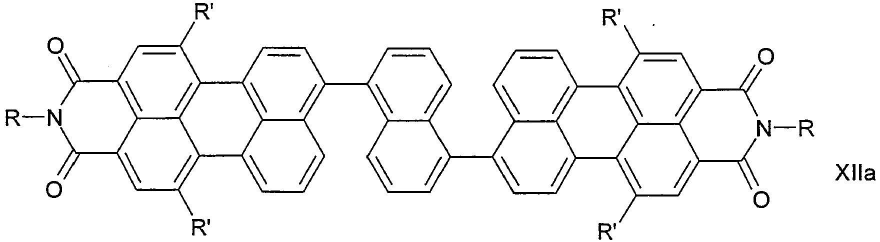 und die Naphthalin-1,4-bis(perylen-3,4-dicarbonsäureimide) der eingangs definierten Formel Xllb 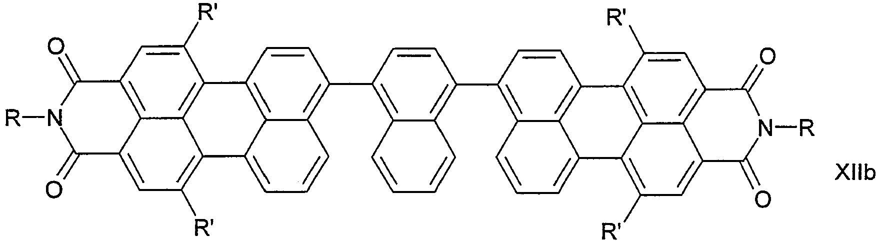 gefunden.

Nicht zuletzt wurde die Verwendung der Rylentetracarbonsäurediimide I zur Einfärbung von hochmolekularen organischen und anorganischen Materialien, zur Herstellung im nahinfraroten Bereich des elektromagnetischen Spektrums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-Laserstrahlenabsorbierende Materialien beim Schweißbehandeln von Kunststoffteilen sowie als Aktivkomponenten in der Photovoltaik gefunden.

Als Beispiele für die in den Formeln genannten Reste R, R', R¹ bis R⁵ sowie deren Substituenten seien im einzelnen genannt:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3-Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3-Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3- Dimethylaminopropyl, 3-Monoisopropylaminopropyl, 2- und 4-Monopropylaminobutyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazadecyl, 3,6,9-Triazaundecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylproypl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
2-Hydroxyethyl, 2- und 3-Hydroxypropyl, 1-Hydroxyprop-2-yl, 3- und 4-Hydroxybutyl, 1-Hydroxybut-2-yl und 8-Hydroxy-4-oxaoctyl;
2-Cyanoethyl, 3-Cyanopropyl, 3- und 4-Cyanobutyl, 2-Methyl-3-ethyl-3-cyanopropyl, 7-Cyano-7-ethylheptyl und 4-Methyl-7-methyl-7-cyanoheptyl;
2-Chlorethyl, 2- und 3-Chlorpropyl, 2-, 3- und 4-Chlorbutyl, 2-Bromethyl, 2- und 3-Brompropyl und 2-, 3- und 4-Brombutyl;
2-Nitroethyl, 2- und 3-Nitropropyl und 2-, 3- und 4-Nitrobutyl;
Methoxy, Ethoxy, Propoxy, Isopropoxy, Butoxy, Isobutoxy, sec.-Butoxy, tert.-Butoxy, Pentoxy, Isopentoxy, Neopentoxy, tert.-Pentoxy und Hexoxy;
Methylamino, Ethylamino, Propylamino, Isopropylamino, Butylamino, Isobutylamino, Pentylamino, Hexylamino, Dimethylamino, Methylethylamino, Diethylamino, Dipropylamino, Diisopropylamino, Dibutylamino, Diisobutylamino, Dipentylamino, Dihexylamino, Dicyclopentylamino, Dicyclohexylamino, Dicycloheptylamino, Diphenylamino und Dibenzylamino;
Carbamoyl, Methylaminocarbonyl, Ethylaminocarbonyl, Propylaminocarbonyl, Butylaminocarbonyl, Pentylaminocarbonyl, Hexylaminocarbonyl, Heptylaminocarbonyl, Octylaminocarbonyl, Nonylaminocarbonyl, Decylaminocarbonyl und Phenylaminocarbonyl;
Formylamino, Acetylamino, Propionylamino und Benzoylamino;
Chlor, Brom und Iod;
Phenylazo, 2-Napthylazo, 2-Pyridylazo und 2-Pyrimidylazo;
Cyclopropyl, Cyclobutyl, Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl und 3-, 4- und 5-Propylcyclooctyl; 3- und 4-Hydroxycyclohexyl, 3- und 4- Nitrocyclohexyl und 3- und 4-Chlorcyclohexyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl, und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4- und 5-Pyrazolyl, 2-, 4- und 5-Imidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
2-, 3- und 4-Methylphenyl, 2,4-, 3,5- und 2,6-Dimethylphenyl, 2,4,6-Trimethylphenyl, 2-, 3- und 4-Ethylphenyl, 2,4-, 3,5- und 2,6-Diethylphenyl, 2,4,6-Triethylphenyl, 2-, 3- und 4-Propylphenyl, 2,4-, 3,5- und 2,6-Dipropylphenyl, 2,4,6-Tripropylphenyl, 2-, 3- und 4-Isopropylphenyl, 2,4-, 3,5- und 2,6-Diisopropylphenyl, 2,4,6-Triisopropylphenyl, 2-, 3- und 4-Butylphenyl, 2,4-, 3,5- und 2,6-Dibutylphenyl, 2,4,6-Tributylphenyl, 2-, 3- und 4-Isobutylphenyl, 2,4-, 3,5- und 2,6-Diisobutylphenyl, 2,4,6-Triisobutylphenyl, 2-, 3- und 4-sec.-Butylphenyl, 2,4-, 3,5- und 2,6-Di-sec.-butylphenyl und 2,4,6-Tri-sec.-butylphenyl; 2-, 3- und 4-Methoxyphenyl, 2,4-, 3,5- und 2,6-Dimethoxyphenyl, 2,4,6-Trimethoxyphenyl, 2-, 3- und 4-Ethoxyphenyl, 2,4-, 3,5- und 2,6-Diethoxyphenyl, 2,4,6-Triethoxyphenyl, 2-, 3- und 4-Propoxyphenyl, 2,4-, 3,5- und 2,6-Dipropoxyphenyl, 2-, 3- und 4-Isopropoxyphenyl, 2,4- und 2,6-Diisopropoxyphenyl und 2-, 3- und 4-Butoxyphenyl; 2-, 3- und 4-Chlorphenyl und 2,4-, 3,5- und 2,6-Dichlorphenyl; 2-, 3- und 4-Hydroxyphenyl und 2,4-, 3,5- und 2,6-Dihydroxyphenyl; 2-, 3- und 4-Cyanophenyl; 3- und 4-Carboxyphenyl; 3- und 4-Carboxamidophenyl, 3- und 4-N-Methylcarboxamidophenyl und 3- und 4-N-Ethylcarboxamidophenyl; 3- und 4-Acetylaminophenyl, 3- und 4-Propionylaminophenyl und 3- und 4-Butyrylaminophenyl; 3- und 4-N-Phenylaminophenyl, 3- und 4-N-(o-Tolyl)aminophenyl, 3- und 4-N-(m-Tolyl)aminophenyl und 3- und 4-(p-Tolyl)aminophenyl; 3- und 4-(2-Pyridyl)aminophenyl, 3- und 4-(3-Pyridyl)aminophenyl, 3- und 4-(4-Pyridyl)aminophenyl, 3- und 4-(2-Pyrimidyl)aminophenyl und 4-(4-Pyrimidyl)aminophenyl;
4-Phenylazophenyl, 4-(1-Naphthylazo)phenyl, 4-(2-Naphthylazo)phenyl, 4-(4-Naphthylazo)phenyl,4-(2-Pyriylazo)phenyl, 4-(3-Pyridylazo)phenyl, 4-(4-Pyridylazo)phenyl, 4-(2-Pyrimidylazo)phenyl, 4-(4-Pyrimidylazo)phenyl und 4-(5-Pyrimidylazo)phenyl;
Phenoxy, Phenylthio, 2-Naphthoxy, 2-Naphthylthio, 2-, 3- und 4-Pyridyloxy, 2-, 3- und 4-Pyridylthio, 2-, 4- und 5-Pyrimidyloxy und 2-, 4- und 5-Pyrimidylthio.

Im folgenden werden die erfindungsgemäßen Herstellungsverfahren näher erläutert.

### Verfahren A1-Hexarvlen:

### Schritt a) - Verfahren A1-Hexarylen:

Beim erfindungsgemäßen Verfahren A1 zur Herstellung der im Rylenkern (het)aryloxy- und (het)arylthiosubstituierten Hexarylentetracarbonsäurediimide Ia wird in Schritt a) ein Diboran II mit a1) einem 9-Bromperylen-3,4-dicarbonsäureimid IIIa oder a2) einem 1,5-disubstituierten Naphthalinderivat IIIb umgesetzt. Das Naphthalinderivat IIIb trägt einen Rest X (Halogenatom oder Alkyl- oder Arylsulfonylrest), der mit dem Diboran II reagieren kann, sowie eine Nitrogruppe, die die Folgeumsetzungen des in Schritt d) gebildeten Terrylen-3,4-dicarbonsäureimids ermöglicht.

Im folgenden werden 9-Bromperylen-3,4-dicarbonsäureimid IIIa und Naphthalinderivat IIIb zusammen als "Edukt III" bezeichnet.

Die Umsetzung des Diborans II mit dem Edukt III wird in Gegenwart eines aprotischen organischen Lösungsmittels, eines Übergangsmetallkatalysators und einer Base vorgenommen.

Das Molverhältnis Diboran II zu Edukt III liegt dabei im allgemeinen bei 0,8 : 1 bis 3 : 1, insbesondere bei 1,5 : 1 bis 2 : 1.

Als Lösungsmittel sind für Schritt a) grundsätzlich alle unter den Reaktionsbedingungen gegen Basen stabilen aprotischen Lösungsmittel mit einem Siedepunkt oberhalb der gewählten Reaktionstemperatur geeignet, in denen sich die Edukte III bei Reaktionstemperatur vollständig und die verwendeten Katalysatoren und Basen zumindest partiell lösen, so daß weitgehend homogene Reaktionsbedingungen vorliegen. Es können sowohl unpolar-aprotische als auch polar-aprotische Lösungsmittel eingesetzt werden, wobei die unpolar-aprotischen Lösungsmittel bevorzugt sind.

Beispiele für bevorzugte unpolar-aprotische Lösungsmittel sind bei > 100°C siedende Lösungsmittel aus den folgenden Gruppen: Aliphaten (insbesondere C₈-C₁₈-Alkane), unsubstituierte, alkylsubstituierte und kondensierte Cycloaliphaten (insbesondere unsubstituierte C₇-C₁₀-Cycloalkane, C₆-C₈-Cycloalkane, die durch ein bis drei C₁-C₆-Alkylgruppen substituiert sind, polycyclische gesättigte Kohlenwasserstoffe mit 10 bis 18 C-Atomen), alkyl- und cycloalkylsubstituierte Aromaten (insbesondere Benzol, das durch ein bis drei C₁-C₆-Alkylgruppen oder einen C₅-C₈-Cycloalkylrest substituiert ist) und kondensierte Aromaten, die alkylsubstituiert und/oder teilhydriert sein können (insbesondere Naphthalin, das durch ein bis vier C₁-C₆-Alkylgruppen substituiert ist) sowie Mischungen dieser Lösungsmittel.

Als Beispiele für besonders bevorzugte Lösungsmittel seien im einzelnen genannt: Octan, Isooctan, Nonan, Isononan, Decan, Isodecan, Undecan, Dodecan, Hexadecan und Octadecan; Cycloheptan, Cyclooctan, Methylcyclohexan, Dimethylcyclohexan, Trimethylcyclohexan, Ethylcyclohexan, Diethylcyclohexan, Propylcyclohexan, Isopropylcyclohexan, Dipropylcyclohexan, Butylcyclohexan, tert.-Butylcyclohexan, Methylcycloheptan und Methylcyclooctan; Toluol, o-, m- und p-Xylol, 1,3,5-Trimethylbenzol (Mesitylen), 1,2,4- und 1,2,3-Trimethylbenzol, Ethylbenzol, Propylbenzol, Isopropylbenzol, Butylbenzol, Isobutylbenzol, tert.-Butylbenzol und Cyclohexylbenzol; Naphthalin, Decahydronaphthalin (Dekalin), 1- und 2-Methylnaphthalin und 1- und 2-Ethylnaphthalin; Kombinationen aus den zuvor genannten Lösungsmitteln, wie sie aus den hochsiedenden, teil- oder durchhydrierten Fraktionen thermischer und katalytischer Crackprozesse bei der Rohöl- oder Naphthaverarbeitung gewonnen werden können, z.B. Gemische vom Exxsol® Typ und Alkylbenzolgemische vom Solvesso® Typ.

Ganz besonders bevorzugte Lösungsmittel sind Xylol (alle Isomeren), Mesitylen und vor allem Toluol.

Beispiele für geeignete polar-aprotische Lösungsmittel sind N,N-disubstituierte aliphatische Carbonsäureamide (insbesondere N,N-Di-C₁-C₄-alkyl-C₁-C₄-carbonsäureamide), stickstoffhaltige Heterocyclen und aprotische Ether (insbesondere cyclische Ether, Diarylether und Di-C₁-C₆-alkylether von monomeren und oligomeren C₂-C₃-Alkylenglykolen, die bis zu 6 Alkylenoxideinheiten enthalten können, vor allem Diethylenglykoldi-C₁-C₄-alkylether).

Als Beispiele für besonders geeignete Lösungsmittel seien im einzelnen genannt: N,N-Dimethylformamid, N,N-Diethylformamid, N,N-Dimethylacetamid und N,N-Dimethylbutyramid; N-Methyl-2-pyrrolidon, Chinolin, Isochinolin, Chinaldin, Pyrimidin, N-Methylpiperidin und Pyridin; Tetrahydrofuran, Dioxan, Diphenylether, Diethylenglykoldimethyl-, -diethyl-, -dipropyl-, -diisopropyl-, -di-n-butyl-, -di-sec.-butyl- und -di-tert.-butylether, Diethylenglykolmethylethylether, Triethylenglykoldimethyl- und -diethylether und Triethylenglykolmethylethylether.

Im Fall der Edukte IIIa sind die unpolar-aprotischen Lösungsmittel, vor allem Toluol, besonders bevorzugt, im Fall der Edukte IIIb sind polar-aprotische Lösungsmittel, insbesondere Dioxan, besonders bevorzugt.

Die Lösungsmittelmenge beträgt in der Regel 10 bis 1000 ml, bevorzugt 20 bis 300 ml, je g Edukt III.

Als Übergangsmetallkatalysator eignen sich insbesondere Palladiumkomplexe, wie Tetrakis(triphenylphosphin)palladium(0), Tetrakis(tris-o-tolylphosphin)palladium(0), [1,2-Bis(diphenylphosphino)ethan]palladium(II)chlorid, [1,1'-Bis(diphenylphosphino)-ferrocen]palladium(II)chlorid, Bis(triethylphosphin)palladium(II)chlorid, Bis(tricyclohexylphosphin)palladium(II)acetat, (2,2'-Bipyridyl)palladium(II)chlorid, Bis(triphenylphosphin)palladium(II)chlorid, Tris(dibenzylidenaceton)dipalladium(0), 1,5-Cyclooctadienpalladium(II)chlorid, Bis(acetonitril)palladium(II)chlorid und Bis(benzonitril)palladium(II)chlorid, wobei [1,1'-Bis(diphenylphosphino)ferrocen]palladium(II)chlorid und Tetrakis(triphenylphosphin)palladium(0) bevorzugt sind.

Üblicherweise wird der Übergangsmetallkatalysator in einer Menge von 1 bis 20 mol-%, vor allem 2 bis 10 mol-%, bezogen auf das Edukt III, eingesetzt.

Als Base kommen vorzugsweise die Alkalimetallsalze, insbesondere die Natrium- und vor allem die Kaliumsalze, schwacher organischer und anorganischer Säuren, wie Natriumacetat, Kaliumacetat, Natriumcarbonat, Natriumhydrogencarbonat, Kaliumcarbonat und Kaliumhydrogencarbonat, zum Einsatz. Bevorzugte Basen sind die Acetate, vor allem Kaliumacetat.

Im allgemeinen werden 1 bis 5 mol, bevorzugt 2 bis 4 mol, Base je mol Edukt III verwendet.

Die Reaktionstemperatur liegt üblicherweise bei 20 bis 180°C, vor allem bei 60 bis 120°C.

Die Reaktionszeit beträgt in der Regel 0,5 bis 30 h, insbesondere 1 bis 20 h.

Verfahrenstechnisch geht man in Schritt a) zweckmäßigerweise wie folgt vor:
Man legt Edukt III und Lösungsmittel vor, gibt Diboran II, den Übergangsmetallkatalysator und die Base nacheinander zu und erhitzt die Mischung 0,5 bis 30 h unter Schutzgas auf die gewünschte Reaktionstemperatur. Nach Abkühlen auf Raumtemperatur filtriert man die festen Bestandteile aus dem Reaktionsgemisch ab und destilliert das Lösungsmittel unter vermindertem Druck ab.

Die Reinheit des so hergestellten 9-(Dioxaborolan-2-yl)perylen-3,4-dicarbonsäureimids IVa bzw. 1-(Dioxaborolan-2-yl)-5-nitronaphthalins IVb (im folgenden zusammen kurz als "Dioxaborolanylderivat IV" bezeichnet) reicht im allgemeinen für die Weiterverarbeitung aus. Gegebenenfalls kann das Rohprodukt durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens weiter aufgereinigt werden.

Die Ausbeute in Schritt a) liegt üblicherweise bei 80 bis 100%.

### Schritt b) - Verfahren A1-Hexarylen:

Das in Schritt a) erhaltene Dioxaborolanylderivat IV wird in Schritt b) einer Suzuki-Kupplungsreaktion mit einem Edukt III unterworfen. Dabei wird entweder das in Schritt a1) erhaltene 9-(Dioxaborolan-2-yl)perylen-3,4-dicarbonsäureimid IVa mit einem Naphthalinderivat IIIb (Schritt b1)) oder das in Schritt a2) erhaltene 1-(Dioxaborolan-2-yl)-5-nitronaphthalin IVb mit einem 9-Bromperylen-3,4-dicarbonsäureimid lila (Schritt b2)) umgesetzt.

Die Umsetzung des Dioxaborolanylderivats IV mit dem Edukt III wird in Gegenwart eines organischen Lösungsmittels, gewünschtenfalls im Gemisch mit Wasser, sowie eines Übergangsmetallkatalysators und einer Base durchgeführt.

Das Molverhältnis von 9-(Dioxaborolan-2-yl)perylen-3,4-dicarbonsäureimid IVa zu Naphthalinderivat IIIb beträgt dabei in der Regel 0,8 : 1 bis 3 : 1, vorzugsweise 0,9 : 1 bis 2 : 1. Das Molverhältnis von 1-(Dioxaborolan-2-yl)-5-nitronaphthalin IVb zu 9-Bromperylen-3,4-dicarbonsäureimid IIIa liegt im allgemeinen bei 0,8 : 1 bis 3 : 1, bevorzugt bei 1,5: 1 bis 2,5: 1.

Als Lösungsmittel eignen sich für Schritt b) alle Lösungsmittel, in denen sich die Dioxaborolanylderivate IV und die Edukte III bei Reaktionstemperatur vollständig und die verwendeten Katalysatoren und Basen zumindest partiell lösen, so daß weitgehend homogene Reaktionsbedingungen vorliegen. Insbesondere geeignet sind die bereits für Schritt a) genannten Lösungsmittel, wobei auch hier die alkylsubstituierten Benzole bevorzugt sind. Die Lösungsmittelmenge liegt üblicherweise bei 10 bis 1000 ml, vorzugsweise bei 20 bis 100 ml, je g Dioxaborolanylderivat IV.

Vorzugsweise setzt man in Schritt b) Wasser als zusätzliches Lösungsmittel ein. In diesem Fall werden in der Regel 10 bis 1000 ml, insbesondere 250 bis 500 ml, Wasser je I organisches Lösungsmittel verwendet.

Als Übergangsmetallkatalysatoren werden in Schritt b) ebenfalls vorzugsweise Palladiumkomplexe eingesetzt, wobei hier die gleichen Bevorzugungen wie in Schritt a) gelten. Die Einsatzmenge Katalysator beträgt üblicherweise 1 bis 20 mol-%, insbesondere 1,5 bis 5 mol-%, bezogen auf das Dioxaborolanylderivat IV.

Als Base sind in Schritt b) wie in Schritt a) die Alkalimetallsalze schwacher Säuren bevorzugt, wobei die Carbonate, wie Natriumcarbonat und vor allem Kaliumcarbonat, besonders bevorzugt sind. In der Regel liegt die Basenmenge bei 0,1 bis 10 mol, insbesondere bei 0,2 bis 5 mol, je mol Dioxaborolanylderivat IV.

Die Reaktionstemperatur beträgt im allgemeinen 20 bis 180°C, bevorzugt 60 bis 120°C. Wird in Schritt b) Wasser eingesetzt, so empfiehlt es sich, die Umsetzung nicht bei Temperaturen über 100°C vorzunehmen, da ansonsten unter Druck gearbeitet werden müßte.

Die Reaktion ist üblicherweise in 0,5 bis 48 h, insbesondere in 5 bis 20 h, beendet.

Verfahrenstechnisch geht man in Schritt b) zweckmäßigerweise wie folgt vor:
Man legt Dioxaborolanylderivat IV und Edukt III sowie Lösungsmittel vor, gibt Übergangsmetallkatalysator und die vorzugsweise in Wasser oder einem Wasser/AlkoholGemisch gelöste Base zu und erhitzt die Mischung 0,5 bis 48 h unter Schutzgas auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Die Reinheit des so hergestellten 9-(5-Nitronaphthyl)perylen-3,4-dicarbonsäureimids V reicht im allgemeinen für die Weiterverarbeitung aus. Gegebenenfalls kann das Rohprodukt durch Waschen mit Wasser und gewünschtenfalls einem geeigneten organischen Lösungsmittel, insbesondere einem chlorierten aliphatischen oder aromatischen Kohlenwasserstoff, oder durch Säulenchromatographie an Kieselgel mit einem Gemisch von Methylenchlorid und Hexan oder Pentan oder mit Toluol als Eluens weiter aufgereinigt werden.

Die Ausbeute in Schritt b) liegt üblicherweise bei 90 bis 95%.

### Schritt c) - Verfahren A1-Hexarvlen:

Das in Schritt b) erhaltene 9-(5-Nitronaphthyl)perylen-3,4-dicarbonsäureimid V wird dann in Schritt c) durch Cyclodehydrierung in das 11-Nitroterrylen-3,4-dicarbonsäureimid VIa überführt.

Die Cyclodehydrierung zum Terrylen-3,4-dicarbonsäureimid VIa wird in Gegenwart eines basenstabilen, hochsiedenden, organischen Lösungsmittels sowie einer alkali- oder erdalkalimetallhaltigen Base und einer stickstoffhaltigen Hilfsbase vorgenommen.

Als Lösungsmittel sind grundsätzlich die für Schritt a) genannten unpolar-aprotischen und polar-aprotischen Lösungsmittel geeignet.

Bevorzugte unpolar-aprotische Lösungsmittel sind Xylol (alle Isomere), Mesitylen und vor allem Toluol und Dekalin, bevorzugte polar-aprotische Lösungsmittel sind Diphenylether und die Dialkylether von monomerem und oligomerem Ethylenglykol, insbesondere Diethylenglykoldimethyl- und -diethylether.

Besonders bevorzugtes Lösungsmittel ist Diethylenglykoldimethylether.

Neben den aprotischen organischen Lösungsmitteln können auch protische Lösungsmittel, die Amino- und Hydroxyfunktionen enthalten, eingesetzt werden. Geeignete Beispiele sind hier Alkoholamine, insbesondere Mono-, Di- und Tri-C₂-C₄-alkoholamine, wie Mono-, Di- und Triethanolamin, wobei Ethanolamin besonders bevorzugt ist.

Die Lösungsmittelmenge beträgt in der Regel 50 bis 250 ml unpolar-aprotisches Lösungsmittel, 10 bis 50 ml polar-aprotisches Lösungsmittel bzw. 3 bis 50 ml protisches Lösungsmittel je g 9-(5-Nitronaphthyl)perylen-3,4-dicarbonsäureimid V.

Als Base sind starke anorganische und organische alkali- oder erdalkalimetallhaltige Basen geeignet, wobei die alkalimetallhaltigen Basen besonders geeignet sind. Bevorzugte anorganische Basen sind Alkali- und Erdalkalimetallhydroxide und -amide, bevorzugte organische Basen sind Alkali- und Erdalkalimetallalkoholate (insbesondere die C₁-C₁₀-Alkoholate, vor allem tert.-C₄-C₆-Alkoholate), Alkali- und Erdalkalimetall-(phenyl)alkylamide (insbesondere die Bis(C₁-C₄-alkyl)amide) und Triphenylmethylmetallate. Besonders bevorzugt sind die Alkalimetallalkoholate. Bevorzugte Alkalimetalle sind Lithium, Natrium und Kalium, wobei Kalium ganz besonders bevorzugt ist. Besonders geeignete Erdalkalimetalle sind Magnesium und Calcium. Selbstverständlich können auch Mischungen verschiedener Basen eingesetzt werden.

Als Beispiele für besonders bevorzugte Basen seien im einzelnen genannt: Lithiumhydroxid, Natriumhydroxid und Kaliumhydroxid; Lithiumamid, Natriumamid und Kaliumamid; Lithiummethylat, Natriummethylat, Kaliummethylat, Lithiumethylat, Natriumethylat, Kaliumethylat, Natriumisopropylat, Kaliumisopropylat, Natrium-tert.-butylat, Kalium-tert.-butylat, Lithium-(1,1-dimethyl)octylat, Natrium-(1,1-dimethyl)octylat und Kalium-(1,1-dimethyl)octylat; Lithiumdimethylamid, Lithiumdiethylamid, Lithiumdiisopropylamid, Natriumdiisopropylamid, Triphenylmethyllithium, Triphenylmethylnatrium und Triphenylmethylkalium.

Ganz besonders bevorzugte Basen sind Lithiumdiisopropylamid, Natriummethylat, Natrium-tert.-butylat, vor allem Kaliummethylat und Kaliumhydroxid und insbesondere Natrium- und Kalium-tert.-butylat.

Insbesondere bei Verwendung der Alkylate und der Hydroxide wird zur Erhöhung der Reaktivität eine stickstoffhaltige Hilfsbase mit geringer nucleophiler Wirkung zugesetzt. Geeignete Basen sind bei den Reaktionstemperaturen flüssige Alkylamine, insbesondere Tri(C₃-C₆-alkyl)amine, wie Tripropylamin und Tributylamin, Alkoholamine, insbesondere Mono-, Di- und Tri(C₂-C₄-alkohol)amine, wie Mono-, Di- und Triethanolamin, und insbesondere heterocyclische Basen, wie Pyridin, N-Methylpiperidin, N-Methylpiperidon, N-Methylmorpholin, N-Methyl-2-pyrrolidon, Pyrimidin, Chinolin, Isochinolin, Chinaldin und vor allem Diazabicyclononen (DBN) und Diazabicycloundecen (DBU). Selbstverständlich können auch Mischungen dieser Hilfsbasen verwendet werden.

Geeignete Einsatzmengen für die Hilfsbase liegen im allgemeinen bei 1 bis 60 g, vorzugsweise bei 5 bis 30 g, je g 9-(5-Nitronaphthyl)perylen-3,4-dicarbonsäureimid V. Von der Alkalimetallbase werden 2 bis 20 mol, vorzugsweise 8 bis 20 mol, je mol 9-(5-Nitronaphthyl)perylen-3,4-dicarbonsäureimid V eingesetzt.

Die Alkalimetallbase kann in fester oder in gelöster Form eingesetzt werden. Wenn die Alkalimetallbase in Kombination mit einem unpolar-aprotischen Reaktionslösungsmittel verwendet wird, in dem sie nicht ausreichend löslich ist, kann sie in einem Alkohol, der eine höhere Basenstärke als die Alkalimetallbase hat, gelöst werden. Geeignet sind vor allem tertiäre aliphatische Alkohole, die Arylsubstituenten enthalten können und insgesamt vier bis zwölf C-Atome aufweisen, z.B. tert.-Butanol, 2-Methyl-2-butanol (tert.-Amylalkohol), 3-Methyl-3-pentanol, 3-Ethyl-3-pentanol, 2-Phenyl-2-pentanol, 2,3-Dimethyl-3-pentanol, 2,4,4-Trimethyl-2-pentanol und 2,2,3,4,4-Pentamethyl-3-pentanol.

Die Reaktionstemperatur liegt üblicherweise bei 50 bis 210°C, bevorzugt bei 20 bis 100°C.

Die Reaktionszeit beträgt in der Regel 0,5 bis 24 h, insbesondere 0,5 bis 4 h.

Verfahrenstechnisch geht man zweckmäßigerweise wie folgt vor, daß man 9-(5-Nitronaphthyl)perylen-3,4-dicarbonsäureimid V und Base vorlegt, Lösungsmittel und Hilfsbase unter Schutzgas zugibt, die Mischung die gewünschte Zeit unter Rühren und unter Schutzgas auf die gewünschte Reaktionstemperatur erhitzt und die gebildeten 11-Nitroterrylen-3,4:11,12-tetracarbonsäurediimide VIa nach Abkühlen auf Raumtemperatur durch Verdünnen mit einem protischen Lösungsmittel, das die anderen Komponenten löst, z.B. mit C₁-C₃-Alkoholen und insbesondere Wasser, ausfällt, abfiltriert, mit einem der genannten Lösungsmittel, insbesondere mit einem der Alkohole, wäscht und anschließend im Vakuum trocknet.

Gelegentlich kann es zweckmäßig sein, das Reaktionsprodukt einer Oxidation zu unterziehen. Dies kann am einfachsten durch Einblasen von Luftsauerstoff in die noch warme Reaktionsmischung geschehen. Es ist jedoch auch möglich, Oxidationsmittel, wie vorzugsweise Wasserstoffperoxid, aber auch aldehydgruppenhaltige Zucker, z.B. Glukose, insbesondere nach der Reaktion zuzugeben.

Die Reinheit des so hergestellten 11-Nitroterrylen-3,4-dicarbonsäureimids VIa reicht im allgemeinen für die Weiterverarbeitung aus. Gegebenenfalls kann man das Rohprodukt aus einem Gemisch von halogenierten Lösungsmitteln, wie Chloroform und Methylenchlorid, und Alkoholen, wie Methanol, Ethanol und Isopropanol, oder aus einem Carbonsäureamid, wie N-Methylpyrrolidon, umkristallisieren. Alternativ kann man auch eine Säulenchromatographie an Kieselgel unter Verwendung von Methylenchlorid oder Hexan oder Pentan oder mit Toluol als Eluens vornehmen.

Die Ausbeute in Schritt c) liegt üblicherweise bei 25 bis 75%.

### Schritt d) - Verfahren A1-Hexarylen:

Das in Schritt c) erhaltene 11-Nitroterrylen-3,4-dicarbonsäureimid VIa wird in Schritt d) mit nascierendem Wasserstoff zum entsprechenden 11-Aminoterrylen-3,4-dicarbonsäureimid VIb reduziert.

Die Reduktion erfolgt vorzugsweise in saurem, alkoholisch-wäßrigem Medium mit einem metallischen Reduktionsmittel.

Beispiele für geeignete metallische Reduktionsmittel sind unedle Metalle, die in möglichst feinverteilter Form eingesetzt werden, wie Eisenpulver und Zinn, sowie Salze von Metallen in niedriger Oxidationsstufe, wie Zinn(II)chlorid. Bevorzugtes Reduktionsmittel ist Eisen.

Die Reduktion erfolgt in Gegenwart von Säuren, insbesondere anorganischen Säuren, vor allem von Salzsäure.

In der Regel werden 0,2 bis 1,5 g, insbesondere 0,8 bis 1,5 g, metallisches Reduktionsmittel und 40 bis 80 ml, vor allem 20 bis 40 ml, konzentrierte anorganische Säure je g 11-Nitroterrylen-3,4-dicarbonsäureimid VIa eingesetzt.

Als alkoholisches Lösungsmittel eignen sich beispielsweise C₁-C₄-Alkohole, wie Methanol, Ethanol, Propanol, Isopropanol und Butanol, wobei Ethanol bevorzugt ist.

Üblicherweise werden 100 bis 500 ml, vor allem 100 bis 200 ml, Alkohol je g 11-Nitroterrylen-3,4-dicarbonsäureimid VIa eingesetzt.

Die Reaktionstemperatur liegt im allgemeinen bei 25 bis 90°C, vorzugsweise bei 75 bis 85°C.

Die Reaktionszeit beträgt üblicherweise 0,5 bis 24 h, bevorzugt 1 bis 10 h.

Verfahrenstechnisch geht man in Schritt d) zweckmäßigerweise so vor, daß man eine Mischung von 11-Nitroterrylen-3,4-dicarbonsäureimid VIa, Metall, Säure und Alkohol 0,5 bis 24 h bei der gewünschten Reaktionstemperatur rührt, dann auf Raumtemperatur abkühlt und das ungelöste Metall durch Filtration vom gelösten 11-Aminoterrylen-3,4-dicarbonsäureimid VIb abtrennt und mehrfach mit einem halogenierten aliphatischen Kohlenwasserstoff, z.B. Methylenchlorid, wäscht. Anschließend trennt man die organische Phase aus dem Filtrat ab und destilliert das Lösungsmittel ab.

Die Reinheit des so hergestellten 11-Aminoterrylen-3,4-dicarbonsäureimids VIb reicht üblicherweise für die Weiterverarbeitung aus.

Die Ausbeute in Schritt d) liegt im allgemeinen bei 75 bis 95%.

Anstelle der bevorzugten Reduktion mit Metall/Säure kann das 11-Nitroterrylen-3,4-dicarbonsäureimid VIa auch katalytisch an Palladium/Aktivkohle hydriert werden.

Diese Hydrierung wird vorzugsweise in polarem protischen Reaktionsmedium, insbesondere in einer Mischung aus einem halogenierten aliphatischen Kohlenwasserstoff und einem Alkohol, z.B. einer 2:1-Mischung von Chloroform und Ethanol, vorgenommen.

In der Regel werden 10 bis 150 ml, vor allem 20 bis 80 ml, Reaktionsmedium je g 11-Nitroterrylen-3,4-dicarbonsäureimid VIa eingesetzt.

Die Katalysatormenge beträgt üblicherweise 2 bis 20 g, vorzugsweise 2 bis 5 g, je g 11-Nitroterrylen-3,4-dicarbonsäureimid VIa.

Die katalytische Hydrierung wird üblicherweise bei Raumtemperatur vorgenommen und dauert im allgemeinen 2 bis 24 h, insbesondere 10 bis 16 h.

Die Isolierung des hergestellten 11-Aminoterrylen-3,4-dicarbonsäureimids Vlb kann wie oben beschrieben erfolgen.

### Schritt e) - Verfahren A1-Hexarvlen:

Das in Schritt d) erhaltene 11-Aminoterrylen-3,4-dicarbonsäureimid VIb wird in Schritt e) in das entsprechende 11-lod- oder 11-Bromterrylen-3,4-dicarbonsäureimid VIc überführt.

Dazu wird das 11-Aminoterrylen-3,4-dicarbonsäureimid VIb zunächst in ein Diazoniumsalz überführt, das dann mit einem Metalliodid oder -bromid umgesetzt wird.

Die Diazotierung wird auf übliche Weise mit einer Kombination von Alkalimetallnitrit, insbesondere Natriumnitrit, und wäßriger Mineralsäure, vor allem Salzsäure, in Gegenwart eines polar-aprotischen organischen Lösungsmittels durchgeführt.

Im allgemeinen werden 1 bis 20 g, vorzugsweise 5 bis 10 g, Alkalimetallnitrit und 10 bis 100 ml, bevorzugt 30 bis 60 ml, Salzsäure je g 11-Aminoterrylen-3,4-dicarbonsäureimid Vlb eingesetzt.

Als polar-aprotisches organisches Lösungsmittel eignen sich insbesondere die in Schritt a) genannten protischen Ether, wobei Diethylether und Tetrahydrofuran besonders geeignet sind, und Nitrile, wie Acetonitril und Propionitril, wobei Acetonitril besonders bevorzugt ist. Vorzugsweise werden Mischungen dieser Lösungsmittel verwendet.

In der Regel kommen 10 bis 80 ml, insbesondere 20 bis 40 ml, organisches Lösungsmittel je g 11-Aminoterrylen-3,4-dicarbonsäureimid VIb zum Einsatz.

Die Diazotierung wird in der Regel bei -20 bis 0°C, insbesondere bei -10 bis -5°C, vorgenommen.

Die Diazoniumgruppe wird anschließend in dem angefallenen Reaktionsgemisch durch Umsetzung mit einem im Reaktionsmedium löslichen Metalliodid oder -bromid substituiert.

Als Metallhalogenide eignen sich vor allem die Alkalimetallhalogenide, vorzugsweise die Natrium- und Kaliumhalogenide, wobei Natrium- und Kaliumiodid bevorzugt sind.

Im allgemeinen werden 1 bis 100 g, vor allem 5 bis 30 g, Metallhalogenid je g Diazoniumsalz eingesetzt.

Die Substitution wird üblicherweise bei -10 bis 20°C, vorzugsweise bei -5 bis 5°C, vorgenommen.

Verfahrenstechnisch geht man in Schritt e) zweckmäßigerweise wie folgt vor:
Man legt eine Lösung von 11-Aminoterrylen-3,4-dicarbonsäureimid VIb im organischen Lösungsmittel vor und tropft zunächst langsam die anorganische Säure und dann unter Kühlen eine Lösung des Nitrits in wäßrig-organischem Medium, insbesondere einer Mischung von Wasser und Acetonitril, so langsam zu, daß die Temperatur nicht über -5°C steigt. Nach einer Nachrührzeit von etwa 0,25 bis 0,5 h bei dieser Temperatur gibt man die Reaktionslösung in eine gekühlte Mischung von Metallhalogenid und wäßrig-organischem Medium, insbesondere Wasser und Acetonitril, rührt etwa 0,5 bis 2 h bei etwa 0°C und läßt die Mischung dann langsam auf Raumtemperatur erwärmen. Man isoliert das Reaktionsprodukt durch Extraktion mit einem halogenierten aliphatischen Kohlenwasserstoff, z.B. Methylenchlorid, wäscht den Extrakt mit Wasser und destilliert das Lösungsmittel ab.

Zur Reinigung wäscht man das erhaltene 11-Halogenterrylen-3,4-dicarbonsäureimid VIc mit der wäßrigen Lösung eines Reduktionsmittels, vorzugsweise mit einer Natriumsulfitlösung, und abschließend mit Wasser und trocknet es anschließend im Vakuum. Gewünschtenfalls kann man zusätzlich eine Säulenchromatographie an Kieselgel mit einem Gemisch von Methylenchlorid und Hexan oder Pentan oder mit Toluol als Eluens vornehmen. In der Regel kann das 11-Halogenterrylen-3,4-dicarbonsäureimid VIc jedoch auch ohne Reinigung für den Folgeschritt f) eingesetzt werden.

Die Ausbeute in Schritt e) liegt im allgemeinen bei 20 bis 25%.

### Schritt f) - Verfahren A1-Hexarylen:

Das in Schritt e) erhaltene 11-Halogenterrylen-3,4-dicarbonsäureimid VIc wird in Schritt f) zum Bisterrylenderivat VII gekuppelt.

Die Kupplung kann dabei f1) in Gegenwart eines organischen Übergangsmetallkomplexes als Katalysator, freier Ligandmoleküle und eines aprotischen Lösungsmittels im Sinne einer Homokupplung oder f2) in Gegenwart von 30 bis 70 mol-%, bezogen auf das 11-Halogenterrylen-3,4-dicarbonsäureimid VIc, eines Diborans II, eines Übergangsmetallkatalysators, einer Base und eines aprotischen Lösungsmittels gemäß einer Suzuki-Kupplungsreaktion erfolgen, wobei das in situ gebildete 11-(Dioxaborolan-2-yl)terrylen-3,4-dicarbonsäureimid VId nicht zwischenisoliert, sondern direkt mit dem verbleibenden 11-Halogenterrylen-3,4-dicarbonsäureimid VIc umgesetzt wird.

Selbstverständlich kann das 11-(Dioxaborolan-2-yl)terrylen-3,4-dicarbonsäureimid Vld auch separat aus dem 11-Halogenterrylen-3,4-dicarbonsäureimid Vlc hergestellt und zwischenisoliert und anschließend in einem weiteren Reaktionsschritt mit 11-Halogenterrylen-3,4-dicarbonsäureimid VIc gekuppelt werden. Diese Vorgehensweise ist Gegenstand des Verfahrens A2-Hexarylen und ist insbesondere zur Herstellung von Hexarylentetracarbonsäurediimiden Ia geeignet, die unsymmetrisch substituiert sind (verschiedene Reste R an den beiden Imidstickstoffatomen und/oder verschiedene Reste R' in den beiden Terrylenbausteinen).

Für die Verfahrensvariante f1) insbesondere geeignete inerte Verdünnungsmittel sind z.B. aliphatische Carbonsäureamide, wie N,N-Dimethylformamid und N,N-Dimethylacetamid, aliphatische und cycloaliphatische Ether, wie 1,2-Dimethoxyethan und Tetrahydrofuran, und Aromaten, wie Benzol, Toluol und Xylol, wobei N,N-Dimethylformamid und N,N-Dimethylacetamid bevorzugt sind.

Die Verdünnungsmittelmenge beträgt in der Regel 20 bis 100 g, vorzugsweise 25 bis 45 g, je g 11-Halogenterrylen-3,4-dicarbonsäureimid VIc.

Bei den als Katalysator dienenden organischen Übergangsmetallkomplexen kommen neben den bereits für Schritt a) genannten Palladiumkomplexen, von denen Tetrakis(triphenylphosphin)palladium(0) bevorzugt ist, insbesondere Nickelkomplexe in Betracht, z.B. Bis(triphenylphosphin)nickel(II)chlorid, Tetrakis(triphenylphosphin)nickel(0), [1,2-Bis(diphenylphosphino)ethan]nickel(II)chlorid und bevorzugt Bis(1,5-cyclooctadien)nickel(0).

Man kann die Katalysatoren auch in situ durch Zugabe von Übergangsmetallsalzen oder -verbindungen, freien Liganden, wie Cyclooctadien, Bipyridyl, Triphenylphosphin, Trifluorphosphin, η-, δ- und π-gebundenen Olefinen, Cycloolefinen, Aromaten und Antiaromaten, Carbonylen, Wasserstoff und Halogen sowie auch deren Mischungen, und erforderlichenfalls Oxidations- oder Reduktionsmitteln erzeugen.

Im allgemeinen werden 40 bis 150 mol-%, vorzugsweise 50 bis 100 mol-%, organischer Übergangsmetallkomplex, bezogen auf das 11-Halogenterrylen-3,4-dicarbonsäureimid Vlc, eingesetzt.

In der Regel empfiehlt sich stets die gleichzeitige Anwesenheit von freien Ligandmolekülen, wie insbesondere Mischungen von Cyclooctadien und Bipyridyl im molaren Verhältnis von 1 : 1 bis 8 : 1. Hierbei sind Mengen von üblicherweise 80 bis 900 mol-%, bevorzugt 80 bis 200 mol-%, bezogen auf das 11-Halogenterrylen-3,4-dicarbonsäureimid VIc, geeignet.

Die Kupplungstemperatur beträgt im allgemeinen 40 bis 80°C, vorzugsweise 60 bis 70°C.

Die Reaktionszeit liegt in der Regel bei 24 bis 48 h, insbesondere bei 36 bis 48 h.

Verfahrenstechnisch geht man in Schritt f1) zweckmäßigerweise so vor, daß man 11-Halogenterrylen-3,4-dicarbonsäureimid VIc, metallorganischen Katalysator sowie freie Ligandmoleküle im inerten Verdünnungsmittel vorlegt und, gegebenenfalls unter Schutzgas, 24 bis 48 h auf die gewünschte Reaktionstemperatur erhitzt. Nach Abkühlen trägt man das Reaktionsgemisch in Wasser, das gegebenenfalls Methanol enthalten kann, ein, gibt verdünnte anorganische Säure, z.B. verdünnte Salzsäure, zu und filtriert den gebildeten Niederschlag ab, wäscht mit Wasser und trocknet im Vakuum.

Die Reinheit des so hergestellten Bisterrylenderivats VII reicht im allgemeinen für die nachfolgende Cyclodehydrierung aus. Gegebenenfalls kann das Produkt noch zusätzlich durch eine Säulenchromatographie an Kieselgel mit einem Gemisch von Methylenchlorid und Hexan oder Pentan als Eluens weiter aufreinigt werden.

Die Ausbeute in Schritt f1) liegt im allgemeinen bei 70 bis 90%.

Bei der Verfahrensvariante f2) wird analog Schritt a) und b) vorgegangen, wobei jedoch nur 30 bis 70 mol-% Diboran II, bezogen auf das 11-Halogenterrylen-3,4-dicarbonsäureimid VIc, für die in situ-Bildung des 11-(Dioxaborolan-2-yl)terrylen-3,4-dicarbonsäureimids VId eingesetzt werden.

In der Regel werden 1 bis 20 mol-%, bevorzugt 5 bis 10 mol-%, Übergangsmetallkatalysator und 1 bis 5 mol, vorzugsweise 2 bis 3 mol, Base je mol 11-Halogenterrylen-3,4-dicarbonsäureimid VIc verwendet. Das aprotische organische Lösungsmittel kommt üblicherweise in Mengen von 10 bis 100 ml, insbesondere 20 bis 50 ml, je g 11-Halogenterrylen-3,4-dicarbonsäureimid VIc zum Einsatz.

Die Reaktionstemperatur liegt im allgemeinen bei 20 bis 100°C, vorzugsweise bei 60 bis 80°C, und die Reaktionsdauer bei 12 bis 72 h, bevorzugt bei 24 bis 48 h.

Verfahrenstechnisch geht man in Schritt f2) zweckmäßigerweise wie folgt vor:
Man legt 11-Halogenterrylen-3,4-dicarbonsäureimid VIc und Lösungsmittel vor, gibt Diboran II, den Übergangsmetallkatalysator und die Base nacheinander zu und erhitzt die Mischung 12 bis 72 h auf die gewünschte Reaktionstemperatur. Nach Abkühlen auf Raumtemperatur trennt man die organische Phase aus dem Reaktionsgemisch ab und destilliert das Lösungsmittel unter vermindertem Druck ab.

Auch hier reicht die Reinheit des erhaltenen Bisterrylenderivats VII in der Regel für die nachfolgende Cyclodehydrierung aus. Wie in Schritt f1) ist jedoch eine weitere Aufreinigung durch Säulenchromatographie möglich.

Die Ausbeute in Schritt f2) liegt üblicherweise bei 80 bis 95%.

### Schritt g) - Verfahren A1-Hexarvlen:

Das in Schritt f) erhaltene Bisterrylenderivat VII wird in Schritt g) zum Hexarylentetracarbonsäurediimid Ia cyclodehydriert.

Die Cyclodehydrierung kann g1) in einem Hydroxy- und Aminofunktionen aufweisenden und eine im wesentlichen ungelöste Base enthaltenden organischen Reaktionsmedium oder g2) wie in Schritt c) in Gegenwart eines basenstabilen, hochsiedenden, organischen Lösungsmittels sowie einer alkali- oder erdalkalimetallhaltigen Base und einer stickstoffhaltigen Hilfsbase vorgenommen werden.

Bevorzugt ist die Verfahrensvariante g1), die im folgenden näher beschrieben wird.

Als organisches Reaktionsmedium sind dabei vor allem Aminoalkohole geeignet, die 2 bis 20, vorzugsweise 2 bis 10 Kohlenstoffatome aufweisen. Die Kohlenstoffkette dieser Alkohole kann durch Sauerstoffatome in Etherfunktion unterbrochen sein. Beispiele für besonders geeignete Lösungsmittel sind Ethanolamin, Triethanolamin und Diethanolamin, wobei Ethanolamin bevorzugt ist. Es ist auch möglich, Mischungen von Alkoholen und Aminen zu verwenden, die jeweils einen Siedepunkt von mindestens 70°C haben und bei der Reaktionstemperatur flüssig sind.

Üblicherweise werden 1,5 bis 150 ml, bevorzugt 5 bis 50 ml, Reaktionsmedium je g Bisterrylenderivat VII eingesetzt.

Als im Reaktionsmedium im wesentlichen unlösliche Base eignen sich die Alkalimetallsalze, insbesondere die Natriumsalze und vor allem die Kaliumsalze, schwacher organischer und bevorzugt schwacher anorganischer Säuren, wie Formiate, Acetate, Propionate, Hydrogencarbonate und besonders bevorzugt Carbonate, insbesondere Natriumcarbonat und vor allem Kaliumcarbonat.

In der Regel beträgt die Basenmenge 1 bis 10 mol, bevorzugt 2 bis 5 mol, je mol Bisterrylenderivat VII.

Die Reaktionstemperatur liegt im allgemeinen bei 40 bis 200°C, insbesondere bei 80 bis 160°C.

Die Reaktionszeit beträgt üblicherweise 0,5 bis 24 h, vorzugsweise 1 bis 12 h.

Verfahrenstechnisch geht man in Schritt g) zweckmäßigerweise so vor, daß man eine Mischung von Bisterrylenderivat VII, Lösungsmittel und Base 0,5 bis 24 h unter Schutzgas bei der gewünschten Reaktionstemperatur rührt und das gebildete Hexarylentetracarbonsäurediimid la nach Abkühlen auf Raumtemperatur durch Zugabe eines Alkohols, wie Ethanol, oder von Wasser aus dem Reaktionsgemisch ausfällt, abfiltriert und mit Wasser wäscht.

Die Reinigung des erhaltenen Hexarylentetracarbonsäurediimids la kann wie folgt vorgenommen werden: Katalysatorrückstände können durch eine schnelle Filtration über Kieselgel unter Waschen mit einem halogenierten aliphatischen Kohlenwasserstoff, wie Methylenchlorid, entfernt werden. Rückstände nichtumgesetzter Edukte auf Perylen- und Terrylenbasis können durch Säulenchromatographie an Kieselgel mit Methylenchlorid als Eluens oder durch wiederholtes Waschen mit Hexan oder Pentan entfernt werden.

Die Ausbeute in Schritt g1) liegt im allgemeinen bei 90 bis 100%.

Bei der Verfahrensvariante g2) wird analog Schritt c) vorgegangen.

Isolierung und Reinigung des erhaltenen Hexarylentetracarbonsäurediimids Ia kann wie in Schritt g1) erfolgen.

Die Ausbeute in Schritt g2) liegt üblicherweise bei 90 bis 95%.

### Verfahren A2-Hexarylen:

### Schritt a) - Verfahren A2-Hexarylen:

Beim erfindungsgemäßen Verfahren A2 zur Herstellung von im Rylenkern (het)aryloxy- und (het)arylthiosubstituierten Hexarylentetracarbonsäurediimiden la wird in Schritt a) ein Diboran II mit einem 11-Halogenterrylen-3,4-dicarbonsäureimid VIc, das wie beim Verfahren A1-Hexarylen beschrieben herzustellen ist, analog Schritt a) des Verfahrens A1-Hexarylen in Gegenwart eines aprotischen organischen Lösungsmittels, eines Übergangsmetallkatalysators und einer Base umgesetzt.

Verfahrenstechnisch geht man zweckmäßigerweise ebenfalls wie für Schritt a) des Verfahrens A1-Hexarylen beschrieben vor. Eine weitere Reinigung des auch hier durch Abfiltrieren der festen Bestandteile und Abdestillieren des Lösungsmittels isolierten 11-(Dioxaborolan-2-yl)terrylen-3,4-dicarbonsäureimids VId ist üblicherweise wiederum nicht erforderlich, kann aber ebenfalls durch Säulenchromatographie an Kieselgel mit einem Gemisch von Methylenchlorid und Hexan oder Pentan oder mit Toluol als Eluens erfolgen.

Die Ausbeute in Schritt a) liegt üblicherweise bei 80 bis 100%.

### Schritt b) - Verfahren A2-Hexarylen:

Das in Schritt a) erhaltene 11-(Dioxaborolan-2-yl)terrylen-3,4-dicarbonsäureimid Vld wird in Schritt b) einer Suzuki-Kupplungsreaktion mit einem 11-Halogenterrylen-3,4-dicarbonsäureimid VIc unterworfen.

Hierbei kann ein 11-Halogenterrylen-3,4-dicarbonsäureimid VIc eingesetzt werden, das entweder mit dem für die Herstellung des 11-(Dioxaborolan-2-yl)terrylen-3,4-dicarbonsäureimids VId eingesetzten 11-Halogenterrylen-3,4-dicarbonsäureimid Vlc identisch oder von diesem verschieden ist (andere Reste R und/oder R' trägt). Im ersten Fall werden symmetrische Hexarylentetracarbonsäurediimide erhalten, während im zweiten Fall unsymmetrische Hexarylentetracarbonsäurediimide zu erhalten sind.

Die Umsetzung wird wie in Schritt b) des Verfahrens A1-Hexarylen beschrieben in Gegenwart eines organischen Lösungsmittels, gewünschtenfalls im Gemisch mit Wasser, sowie eines Übergangsmetallkatalysators und einer Base durchgeführt.

Verfahrenstechnisch geht man zweckmäßigerweise ebenfalls wie für Schritt b) des Verfahrens A1-Hexarylen beschrieben vor. Eine weitere Reinigung des auch hier durch Abtrennen der organischen Phase und Abdestillieren des Lösungsmittels isolierten Bisterrylenderivats VII ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens erfolgen.

Die Ausbeute in Schritt b) liegt üblicherweise bei 80 bis 95%.

### Schritt c) - Verfahren A2-Hexarylen:

Die Cyclodehydrierung des in Schritt b) erhaltenen Bisterrylenderivats VII in Schritt c) zum Hexarylentetracarbonsäurediimid Ia entspricht Schritt g) des Verfahrens A1-Hexarylen. Auch hier ist die zu Schritt g1) des Verfahrens A1-Hexarylen analoge Vorgehensweise c1) bevorzugt.

Auch mit diesem Verfahren sind sowohl symmetrische als auch unsymmetrische Hexarylentetracarbonsäurediimide Ia (verschiedene Reste R an den beiden Imidstickstoffatomen und/oder verschiedene Reste R' in den beiden Terrylenbausteinen) zugänglich.

### Verfahren A1-Pentarylen:

In Analogie zum Verfahren A1-Hexarylen werden beim erfindungsgemäßen Verfahren A1 zur Herstellung von im Rylenkern (het)aryloxy- und (het)arylthiosubstituierten Pentarylentetracarbonsäurediimiden Ib ein 11-Halogenterrylen-3,4-dicarbonsäureimid Vlc und ein 9-(Dioxaborolan-2-yl)perylen-3,4-dicarbonsäureimid IVa einer Suzuki-Kupplungsreaktion unterzogen und das gebildete 11-(9-Perylen-3,4-dicarbonsäureimid)-terrylen-3,4-dicarbonsäureimid VIII cyclodehydriert.

### Schritt a) - Verfahren A1-Pentarylen:

Die Suzuki-Kupplungsreaktion zwischen dem 11-Halogenterrylen-3,4-dicarbonsäureimid VIc, das wie beim Verfahren A1-Hexarylen beschrieben herzustellen ist, und dem 9-(Dioxaborolan-2-yl)perylen-3,4-dicarbonsäureimid IVa in Schritt a) wird analog Schritt b) des Verfahrens A1-Hexarylen in Gegenwart eines organischen Lösungsmittels, gewünschtenfalls im Gemisch mit Wasser, sowie eines Übergangsmetallkatalysators und einer Base vorgenommen.

Verfahrenstechnisch geht man zweckmäßigerweise ebenfalls analog Schritt b) des Verfahrens A1-Hexarylen vor. Eine weitere Reinigung des durch Abtrennen der organischen Phase und Abdestillieren des Lösungsmittels isolierten 11-(9-Perylen-3,4-dicarbonsäureimid)terrylen-3,4-dicarbonsäureimids VIII ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens erfolgen.

Die Ausbeute in Schritt a) liegt üblicherweise bei 90 bis 95%.

### Schritt b) - Verfahren A1-Pentarylen:

Die Cyclodehydrierung des in Schritt a) erhaltenen 11-(9-Perylen-3,4-dicarbonsäureimid)terrylen-3,4-dicarbonsäureimids VIII zum Pentarylentetracarbonsäurediimid Ib kann b1) analog Schritt g1) des Verfahrens A1-Hexarylen in einem Hydroxy- und Aminofunktionen aufweisenden und eine im wesentlichen ungelöste Base enthaltenden organischen Reaktionsmedium oder b2) analog Schritt g2) des Verfahrens-A1-Hexarylen in Gegenwart eines basenstabilen, hochsiedenden, organischen Lösungsmittels sowie einer alkali- oder erdalkalimetallhaltigen Base vorgenommen werden. Auch hier ist die Verfahrensvariante b1) (und damit auch die Verfahrensvariante a1)) bevorzugt.

Verfahrenstechnisch geht man zweckmäßigerweise ebenfalls analog Schritt g1) bzw. g2) des Verfahrens A1-Hexarylen vor.

Zur Reinigung des erhaltenen Pentarylentetracarbonsäurediimids Ib kann ebenfalls eine schnelle Filtration über Kieselgel unter Waschen mit einer Mischung von polar- und unpolar-aprotischen Lösungsmitteln, z.B. Methylenchlorid/Hexan oder Pentan, sowie eine Säulenchromatographie an Kieselgel mit Methylenchlorid als Eluens oder wiederholtes Waschen mit Hexan oder Pentan vorgenommen werden.

Die Ausbeute in Schritt b1) liegt im allgemeinen bei 90 bis 100%, die Ausbeute in Schritt b2) beträgt üblicherweise 90 bis 100 %.

Mit Hilfe des erfindungsgemäßen Verfahrens A1-Pentarylen sind sowohl symmetrische als auch unsymmetrische Pentarylentetracarbonsäurediimide Ia (verschiedene Reste R an den beiden Imidstickstoffatomen und/oder verschiedene Reste R' im Terrylen- und im Perylenbaustein) zugänglich.

### Verfahren A2-Pentarylen:

In Analogie zum Verfahren A2-Hexarylen werden beim erfindungsgemäßen Verfahren A2 zur Herstellung von im Rylenkern (het)aryloxy- und (het)arylthiosubstituierten Pentarylentetracarbonsäurediimiden Ib ein 11-(Dioxaborolan-2-yl)terrylen-3,4-dicarbonsäureimid VId und ein 9-Bromperylen-3,4-dicarbonsäureimid lila einer Suzuki-Kupplungsreaktion unterzogen und das gebildete 11-(9-Perylen-3,4-dicarbonsäureimid)-terrylen-3,4-dicarbonsäureimid VIII cyclodehydriert.

### Schritt a) - Verfahren A2-Pentarylen:

Die Suzuki-Kupplungsreaktion zwischen dem 11-(Dioxaborolan-2-yl)terrylen-3,4-dicarbonsäureimid Vld, das wie in Schritt a) des Verfahrens A2-Hexarylen beschrieben herzustellen ist, und dem 9-Bromperylen-3,4-dicarbonsäureimid lila in Schritt a) wird analog Schritt b) des Verfahrens A1-Hexarylen in Gegenwart eines organischen Lösungsmittels, gewünschtenfalls im Gemisch mit Wasser, sowie eines Übergangsmetallkatalysators und einer Base vorgenommen.

Verfahrenstechnisch geht man zweckmäßigerweise ebenfalls analog Schritt b) des Verfahrens A1-Hexarylen vor. Eine weitere Reinigung des durch Abtrennen der organischen Phase und Abdestillieren des Lösungsmittels isolierten 11-(9-Perylen-3,4-dicarbonsäureimid)terrylen-3,4-dicarbonsäureimids VIII ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens erfolgen.

Die Ausbeute in Schritt a) liegt üblicherweise bei 90 bis 95%.

### Schritt b) - Verfahren A2-Pentarylen:

Die Cyclodehydrierung des in Schritt a) erhaltenen 11-(9-Perylen-3,4-dicarbonsäureimid)terrylen-3,4-dicarbonsäureimids VIII zum Pentarylentetracarbonsäurediimid Ib entspricht Schritt b) des Verfahrens A1-Pentarylen.

Mit Hilfe des erfindungsgemäßen Verfahrens A2-Pentarylen sind sowohl symmetrische als auch unsymmetrische Pentarylentetracarbonsäurediimide la (verschiedene Reste R an den beiden Imidstickstoffatomen und/oder verschiedene Reste R' im Terrylen- und im Perylenbaustein) zugänglich.

### Verfahren B-Hexarylen:

Mit dem erfindungsgemäßen Verfahren B-Hexarylen sind sowohl im Rylenkern (het)aryloxy- und (het)arylthiosubstituierte als auch im Rylenkern unsubstituierte Hexarylentetracarbonsäurediimide Ia zugänglich.

### Schritt a) - Verfahren B-Hexarylen:

In Schritt a) dieses Verfahrens wird ein Diboran II mit a1) einem 9-Bromperylen-3,4-dicarbonsäureimid lila oder a2) einem 3,9- oder 3,10-Dihalogenperylen IXa umgesetzt.

Die Dihalogenperylene IXa stellen üblicherweise Gemische des 3,9- und 3,10-Isomers dar. Dementsprechend fallen auch die aus ihnen in Schritt a) hergestellten Bis(dioxaborolan-2-yl)perylene IXb als Gemisch von 3,9- und 3,10-Isomer an. Dies gilt entsprechend für die in Schritt b) aus den disubstituierten Perylenen IXa oder IXb gebildeten Kupplungsprodukte Xa und Xb. Der Einfachheit halber werden die jeweiligen Isomere in der Beschreibung und den Patentansprüchen als getrennte Individuen aufgeführt. Erfindungsgemäß sollen damit jedoch auch die Isomerenmischungen umfasst werden.

Die Umsetzung wird analog Schritt a) des Verfahrens A1-Hexarylen in Gegenwart eines aprotischen organischen Lösungsmittels, eines Übergangsmetallkatalysators und einer Base vorgenommen.

Da in Schritt a2) beide Halogenatome des Dihalogenperylens IXa durch Dioxaborolan-2-ylreste ersetzt werden, wird hier die doppelte Menge an Diboran II benötigt, d.h., es werden üblicherweise 2 bis 4 mol, insbesondere 2 bis 3 mol, Diboran II je mol Dihalogenperylen IXa eingesetzt.

Ebenfalls zu verdoppeln sind die Menge an Übergangsmetallkatalysator und Base, die Lösungsmittelmenge und die weiteren Reaktionsbedingungen entsprechen Schritt a) des Verfahrens A1-Hexarylen.

Verfahrenstechnisch geht man zweckmäßigerweise ebenfalls wie für Schritt a) des Verfahrens A1-Hexarylen beschrieben vor. Eine weitere Reinigung des auch hier durch Abfiltrieren der festen Bestandteile und Abdestillieren des Lösungsmittels isolierten 9-(Dioxaborolan-2-yl)perylen-3,4-dicarbonsäureimids IVa bzw. 3,9- oder 3,10-Bis(dioxaborolan-2-yl)perylens IXb ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens erfolgen.

Die Ausbeute in Schritt a) liegt üblicherweise bei 75 bis 95%.

### Schritt b) - Verfahren B-Hexarylen:

Das in Schritt a) erhaltene Dioxaborolanylderivat wird in Schritt b) einer doppelten Suzuki-Kupplungsreaktion mit einem halogenierten Perylenedukt unterworfen. Dabei wird entweder das in Schritt a1) erhaltene 9-(Dioxaborolan-2-yl)perylen-3,4-dicarbonsäureimid IVa mit einem 3,9- oder 3,10-Dihalogenperylen IXa (Schritt b1)) oder das in Schritt a2) erhaltene 3,9- bzw. 3,10-Bis(dioxaborolan-2-yl)perylen IXb mit einem 9-Bromperylendicarbonsäureimid IIIa (Schritt b2)) umgesetzt.

Die zur Durchführung zweier Kupplungsreaktionen befähigten Perylenderivate IXa bzw. IXb bilden dabei die Mittelstücke und die Perylen-3,4-dicarbonsäureimide IVa bzw. lila die Endstücke des resultierenden Kupplungsprodukts.

Das Molverhältnis von 9-(Dioxaborolan-2-yl)perylen-3,4-dicarbonsäureimid IVa zu Dihalogenperylen IXa bzw. von 9-Bromperylendicarbonsäureimid lila zu Bis(dioxaborolan-2-yl)perylen IXb beträgt dementsprechend üblicherweise 2 : 1 bis 4 : 1, bevorzugt 2 : 1 bis 3 : 1.

Durch Einsatz zweier verschiedener 9-(Dioxaborolan-2-yl)perylen-3,4-dicarbonsäureimide IVa bzw. 9-Bromperylendicarbonsäureimide lila besteht hier auch die Möglichkeit, unsymmetrische Hexarylentetracarbonsäurediimide la herzustellen.

Die Kupplung wird analog Schritt b) des Verfahrens A1-Hexarylen in Gegenwart eines organischen Lösungsmittels, gewünschtenfalls im Gemisch mit Wasser, sowie eines Übergangsmetallkatalysators und einer Base durchgeführt.

Es werden jedoch wiederum verdoppelte Mengen Übergangsmetallkatalysator und Base eingesetzt. Die Lösungsmittelmenge und die weiteren Reaktionsbedingungen entsprechen Schritt b) des Verfahrens A1-Hexarylen.

Verfahrenstechnisch geht man zweckmäßigerweise ebenfalls wie für Schritt b) des Verfahrens A1-Hexarylen beschrieben vor. Eine weitere Reinigung des auch hier durch Abtrennen der organischen Phase und Abdestillieren des Lösungsmittels isolierten Perylen-3,9-bis(perylen-3,4-dicarbonsäureimids) Xa bzw. Perylen-3,10-bis(perylen-3,4-dicarbonsäureimids) Xb ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan oder mit Toluol als Eluens erfolgen.

Die Ausbeute in Schritt b) liegt üblicherweise bei 70 bis 90%.

### Schritt c) - Verfahren B-Hexarylen:

Die Cyclodehydrierung des in Schritt b) erhaltenen Perylen-3,9-bis(perylen-3,4-dicarbonsäureimids) Xa bzw. Perylen-3,10-bis(perylen-3,4-dicarbonsäureimids) Xb zum Hexarylentetracarbonsäurediimid Ia kann entweder einstufig (Variante c1) oder zweistufig (Variante c2) vorgenommen werden.

Bei der einstufigen Vorgehensweise c1) wird das Perylen-3,9-bis(perylen-3,4-dicarbonsäureimid) Xa bzw. Perylen-3,10-bis(perylen-3,4-dicarbonsäureimid) Xb in Gegenwart eines inerten organischen Lösungsmittels mit einer starken Lewis-Säure in Kontakt gebracht.

Als Lösungsmittel eignen sich dabei grundsätzlich alle unter den Reaktionsbedingungen inerten organischen Lösungsmittel, wobei polar-aprotische Lösungsmittel bevorzugt sind.

Beispiele für besonders geeignete Lösungsmittel sind halogenierte und nitrierte aromatische Kohlenwasserstoffe, wie Chlorbenzol, Di- und Trichlorbenzole und Nitrobenzol.

Die Lösungsmittelmenge beträgt in der Regel 10 bis 100 ml, vorzugsweise 20 bis 40 ml, je g Perylen-3,9-bis(perylen-3,4-dicarbonsäureimid) Xa bzw. Perylen-3,10-bis-(perylen-3,4-dicarbonsäureimid) Xb.

Als starke Lewis-Säure sind vor allem Aluminiumtrihalogenide, wie Aluminiumtrichlorid und Aluminiumtribromid, geeignet, wobei Aluminiumtrichlorid bevorzugt ist.

Üblicherweise werden 2 bis 10 mol, bevorzugt 6 bis 8 mol, Lewis-Säure je mol Perylen-3,9-bis(perylen-3,4-dicarbonsäureimid) Xa bzw. Perylen-3,10-bis(perylen-3,4-dicarbonsäureimid) Xb eingesetzt.

Die Reaktionstemperatur liegt im allgemeinen bei 20 bis 120°C, vorzugsweise bei 45 bis 80°C.

Die Reaktionszeit beträgt in der Regel 0,25 bis 48 h, insbesondere 0,25 bis 8 h.

Verfahrenstechnisch geht man zweckmäßigerweise so vor, daß man eine Mischung von Perylen-3,9-bis(perylen-3,4-dicarbonsäureimid) Xa bzw. Perylen-3,10-bis(perylen-3,4-dicarbonsäureimid) Xb, Lösungsmittel und Lewis-Säure 0,25 bis 48 h bei der gewünschten Reaktionstemperatur rührt, das Lösungsmittel nach Abkühlen auf Raumtemperatur abdestilliert und das gebildete Hexarylentetracarbonsäurediimid Ia zuerst mit verdünnter anorganischer Säure, z.B. einer verdünnten Salzsäure, und dann mit Wasser wäscht und abfiltriert.

Zur weiteren Reinigung können die (het)aryloxy- oder (het)arylthiosubstituierten Hexarylentetracarbonsäurediimide Ia wiederholt mit Hexan oder Pentan gewaschen werden, einer schnellen Filtration über Kieselgel unter Waschen mit einem halogenierten aliphatischen Kohlenwasserstoff, wie Methylenchlorid, oder einer Säulenchromatographie an Kieselgel mit Methylenchlorid als Eluens unterzogen werden. Unsubstituierte Hexarylentetracarbonsäurediimide la (R' = H) können durch Waschen mit Aceton und anschließend mit Methylenchlorid gereinigt werden.

Die Ausbeute bei der einstufigen Cyclodehydrierung c1) liegt im allgemeinen bei 20 bis 30%.

Bei zweistufiger Vorgehensweise c2) wird das Perylen-3,9-bis(perylen-3,4-dicarbonsäureimid) Xa bzw. Perylen-3,10-bis(perylen-3,4-dicarbonsäureimid) Xb in einem ersten Schritt c2a) bei Raumtemperatur in Gegenwart eines inerten organischen Lösungsmittels mit einer schwachen Lewis-Säure in Kontakt gebracht.

Das dabei gebildete 13-(9-Perylen-3,4-dicarbonsäureimid)quaterrylen-3,4-dicarbonsäureimid XI wird dann nach Zwischenisolierung in einem zweiten Schritt c2b) analog Schritt g) des Verfahrens A1-Hexarylen weiter zum Hexarylentetracarbonsäurediimid Ia cyclodehydriert. Die Cyclodehydrierung kann c2bα) in einem Hydroxy- und Aminofunktionen aufweisenden und eine im wesentlichen ungelöste Base enthaltenden organischen Reaktionsmedium (analog Schritt g1) des Verfahrens A1-Hexarylen) oder c2bβ) in Gegenwart eines basenstabilen, hochsiedenden, organischen Lösungsmittels sowie einer alkali- oder erdalkalimetallhaltigen Base und einer stickstoffhaltigen Hilfsbase (analog Schritt g2) des Verfahrens A1-Hexarylen) vorgenommen werden. Auch hier ist die Verfahrensvariante c2a) bevorzugt.

Die zweistufige Vorgehensweise c2) hat den Vorteil, daß aufgrund der milderen Reaktionsbedingungen im ersten Cyclodehydrierungsschritt c2a) die Gefahr der unerwünschten Abspaltung von Alkylsubstituenten an aromatischen Resten R oder R' verringert wird.

In Schritt c2a) können dieselben Lösungsmittel in denselben Mengen wie bei der einstufigen Vorgehensweise c1) eingesetzt werden, wobei dieselben Bevorzugungen gelten.

Als schwache Lewis-Säure sind vor allem Eisen(III)halogenide, wie Eisen(III)chlorid und Eisen(III)bromid, geeignet, wobei Eisen(III)chlorid bevorzugt ist. Die schwachen Lewis-Säuren können in den gleichen Mengen wie die starken Lewis-Säuren bei der einstufigen Vorgehensweise c1) eingesetzt werden.

Die Reaktion wird in Schritt c2a) jedoch bei Temperaturen um Raumtemperatur durchgeführt, d.h., ein Erhitzen des Reaktionsgemischs ist in der Regel nicht erforderlich.

Die Reaktionszeiten liegen üblicherweise bei etwa 4 bis 48 h, insbesondere bei 8 bis 24 h.

Verfahrenstechnisch geht man zweckmäßigerweise so vor, daß man Perylen-3,9-bis(perylen-3,4-dicarbonsäureimid) Xa bzw. Perylen-3,10-bis(perylen-3,4-dicarbonsäureimid) Xb, Lösungsmittel und Lewis-Säure unter Schutzgas 4 bis 48 h auf die gewünschte Reaktionstemperatur erhitzt und das gebildete 13-(9-Perylen-3,4-dicarbonsäureimid)quaterrylen-3,4-dicarbonsäureimid XI durch Abdestillieren des Lösungsmittels und nachfolgendes Waschen zunächst mit verdünnter anorganischer Säure, z.B. einer verdünnten Salzsäure, und dann mit Wasser isoliert.

Die Reinheit des so hergestellten 13-(9-Perylen-3,4-dicarbonsäureimid)quaterrylen-3,4-dicarbonsäureimids XI reicht in der Regel für den zweiten Cyclodehydrierungsschritt c2b) aus. Gegebenenfalls kann man das Rohprodukt durch Säulenchromatographie an Kieselgel mit Methylenchlorid als Eluens weiter aufreinigen.

Die Ausbeute in Schritt c2a) liegt im allgemeinen bei 30 bis 40%.

Der zweite Cyclodehydrierungsschritt c2b) zum Hexarylentetracarbonsäurediimid Ia wird analog Schritt g1) des Verfahrens A1-Hexarylen vorzugsweise in einem Hydroxy- und Aminofunktionen aufweisenden und eine im wesentlichen ungelöste Base enthaltenden organischen Reaktionsmedium vorgenommen (Schritt c2bα)), kann aber auch analog Schritt g2) des Verfahrens A1-Hexarylen in Gegenwart eines basenstabilen, hochsiedenden, organischen Lösungsmittels sowie einer alkali- oder erdalkalimetallhaltigen Base und einer stickstoffhaltigen Hilfsbase durchgeführt werden (Schritt c2bβ)).

Verfahrenstechnisch geht man zweckmäßigerweise ebenfalls wie für Schritt g) des Verfahrens A1-Hexarylen beschrieben vor.

Die weitere Reinigung des erhaltenen Hexarylentetracarbonsäurediimids Ia kann wie bei der einstufigen Vorgehensweise c1) vorgenommen werden.

Die Ausbeute in Schritt c2bα) liegt im allgemeinen bei 80 bis 100%, die Ausbeute in Schritt c2bβ) beträgt üblicherweise 80 bis 95%.

Mit Hilfe des erfindungsgemäßen Verfahrens B-Hexarylen sind sowohl symmetrische als auch unsymmetrische Hexarylentetracarbonsäurediimide Ia (verschiedene Reste R an den beiden Imidstickstoffatomen und/oder verschiedene Reste R' in den beiden äußeren Perylenbausteinen) zugänglich.

### Verfahren B-Pentarylen:

Mit dem zum Verfahren B-Hexarylen analogen erfindungsgemäßen Verfahren B-Pentarylen sind sowohl im Rylenkern (het)aryloxy- und (het)arylthiosubstituierte als auch im Rylenkern unsubstituierte Pentarylentetracarbonsäurediimide Ib zugänglich.

### Schritt a) - Verfahren B-Pentarylen:

In Schritt a) dieses Verfahrens wird ein Diboran II mit a1) einem 9-Bromperylen-3,4-dicarbonsäureimid lila oder a2) einem 1,5- oder 1,4-Dihalogennaphthalin IXc umgesetzt.

Diese Umsetzung wird analog Schritt a) des Verfahrens A1-Hexarylen in Gegenwart eines aprotischen organischen Lösungsmittels, eines Übergangsmetallkatalysators und einer Base vorgenommen.

Da in Schritt a2) beide Halogenatome des Dihalogennaphthalins IXc durch Dioxaborolan-2-ylreste ersetzt werden, wird hier die doppelte Menge an Diboran II benötigt, d.h., es werden üblicherweise 2 bis 4 mol, insbesondere 2 bis 3 mol, Diboran II je mol Dihalogennaphthalin IXc eingesetzt.

Analog Verfahren B-Hexarylen sind die Menge an Übergangsmetallkatalysator und Base ebenfalls zu verdoppeln, die Lösungsmittelmenge und die weiteren Reaktionsbedingungen entsprechen Schritt a) des Verfahrens A1-Hexarylen.

Verfahrenstechnisch geht man zweckmäßigerweise ebenfalls analog Schritt a) des Verfahrens A1-Hexarylen vor. Eine weitere Reinigung des auch hier durch Abfiltrieren der festen Bestandteile und Abdestillieren des Lösungsmittels isolierten 9-(Dioxaborolan-2-yl)perylen-3,4-dicarbonsäureimids IVa bzw. 1,5- oder 1,4-Bis(dioxaborolan-2-yl)-naphthalins IXd ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan als Eluens erfolgen.

Die Ausbeute in Schritt a) liegt üblicherweise bei 80 bis 95%.

### Schritt b) - Verfahren B-Pentarylen:

Das in Schritt a) erhaltene Dioxaborolanylderivat wird in Schritt b) einer doppelten Suzuki-Kupplungsreaktion mit einem halogenierten Naphthalin- oder Perylenedukt unterworfen. Dabei wird entweder das in Schritt a1) erhaltene 9-(Dioxaborolan-2-yl)perylen-3,4-dicarbonsäureimid IVa mit einem 1,5- oder 1,4-Dihalogennaphthalin IXc (Schritt b1)) oder das in Schritt a2) erhaltene 1,5- bzw. 1,4-Bis(dioxaborolan-2-yl)naphthalin IXd mit einem 9-Bromperylendicarbonsäureimid lila (Schritt b2)) umgesetzt.

Die zur Durchführung zweier Kupplungsreaktionen befähigten Naphthalinderivate IXc bzw. IXd bilden dabei die Mittelstücke und die Perylen-3,4-dicarbonsäureimide IVa bzw. lila die Endstücke des resultierenden Kupplungsprodukts.

Das Molverhältnis von 9-(Dioxaborolan-2-yl)perylen-3,4-dicarbonsäureimid IVa zu Dihalogennaphthalin IXc bzw. von 9-Bromperylendicarbonsäureimid lila zu Bis(dioxaborolan-2-yl)naphthalin IXd beträgt dementsprechend üblicherweise 2 : 1 bis 4 : 1, bevorzugt 2 : 1 bis 3 : 1.

Durch Einsatz zweier verschiedener 9-(Dioxaborolan-2-yl)perylen-3,4-dicarbonsäureimide IVa bzw. 9-Bromperylendicarbonsäureimide lila besteht auch hier die Möglichkeit, unsymmetrische Pentarylentetracarbonsäurediimide la herzustellen.

Die Kupplung wird analog Schritt b) des Verfahrens A1-Hexarylen in Gegenwart eines organischen Lösungsmittels, gewünschtenfalls im Gemisch mit Wasser, sowie eines Übergangsmetallkatalysators und einer Base durchgeführt.

Es werden jedoch wiederum verdoppelte Mengen Übergangsmetallkatalysator und Base eingesetzt. Die Lösungsmittelmenge und die weiteren Reaktionsbedingungen entsprechen Schritt b) des Verfahrens A1-Hexarylen.

Verfahrenstechnisch geht man zweckmäßigerweise ebenfalls analog Schritt b) des Verfahrens A1-Hexarylen vor. Eine weitere Reinigung des auch hier durch Abtrennen der organischen Phase und Abdestillieren des Lösungsmittels isolierten Naphthalin-1,5-bis(perylen-3,4-dicarbonsäureimids) Xlla bzw. Naphthalin-1,4-bis(perylen-3,4-dicarbonsäureimids) Xllb ist üblicherweise wiederum nicht erforderlich, kann aber ebenfalls durch Waschen mit einem die Verunreinigungen lösenden Lösungsmittel, wie Wasser, oder durch Säulenchromatographie an Kieselgel mit einem Gemisch von Methylenchlorid und Hexan oder Pentan als Eluens erfolgen.

Die Ausbeute in Schritt b) liegt üblicherweise bei 80 bis 90%.

### Schritt c) - Verfahren B-Pentarylen:

Die Cyclodehydrierung des in Schritt b) erhaltenen Naphthalin-1,5-bis(perylen-3,4-dicarbonsäureimids) Xlla bzw. Naphthalin-1,4-bis(perylen-3,4-dicarbonsäureimids) Xllb zum Pentarylentetracarbonsäurediimid Ib kann analog Schritt c) des Verfahrens B-Hexarylen ebenfalls einstufig (Variante c1)) oder zweistufig (Variante c2)) vorgenommen werden.

Bei der einstufigen Vorgehensweise c1) wird das Naphthalin-1,5-bis(perylen-3,4-dicarbonsäureimid) Xlla bzw. Naphthalin-1,4-bis(perylen-3,4-dicarbonsäureimid) Xllb analog der einstufigen Vorgehensweise in Schritt c) des Verfahrens B-Hexarylen in Gegenwart eines inerten organischen Lösungsmittels mit einer starken Lewis-Säure in Kontakt gebracht.

Verfahrenstechnisch geht man zweckmäßigerweise ebenfalls analog Schritt c1) des Verfahrens B-Hexarylen vor. Eine Reinigung des auch hier durch Abdestillieren des Lösungsmittels isolierten Pentarylentetracarbonsäurediimids Ib kann im Fall der (het)aryloxy- oder (het)arylthiosubstituierten Pentarylenteracarbonsäurediimiden Ib ebenfalls durch wiederholtes Waschen mit Hexan oder Pentan, durch eine schnelle Filtration über Kieselgel unter Waschen mit einem halogenierten aliphatischen Kohlenwasserstoff, wie Methylenchlorid, oder eine Säulenchromatographie an Kieselgel mit Methylenchlorid als Eluens erfolgen. Unsubstituierte Pentarylentetracarbonsäurediimide Ib (R' = H) können durch Waschen mit Aceton und anschließend mit Methylenchlorid gereinigt werden.

Die Ausbeute bei einstufiger Cyclodehydrierung c) liegt üblicherweise bei 20 bis 35%.

Bei zweistufiger Vorgehensweise c2) wird das Naphthalin-1,5-bis(perylen-3,4-dicarbon-säureimid) Xlla bzw. Naphthalin-1,4-bis(perylen-3,4-dicarbonsäureimid) Xllb in einem ersten Schritt c2a) bei Raumtemperatur in Gegenwart eines inerten organischen Lösungsmittels mit einer schwachen Lewis-Säure in Kontakt gebracht.

Das dabei gebildete 11-(9-Perylen-3,4-dicarbonsäureimid)terrylen-3,4-dicarbonsäure-imid VIII wird dann nach Zwischenisolierung in einem zweiten Schritt c2b) weiter zum Pentarylentetracarbonsäurediimid Ib cyclodehydriert. Die Cyclodehydrierung kann c2bα) in einem Hydroxy- und Aminofunktionen aufweisenden und eine im wesentlichen ungelöste Base enthaltenden organischen Reaktionsmedium (analog Schritt g1) des Verfahrens A1-Hexarylen) oder c2bβ) in Gegenwart eines basenstabilen, hochsiedenden, organischen Lösungsmittels sowie einer alkali- oder erdalkalimetallhaltigen Base und einer stickstoffhaltigen Hilfsbase (analog Schritt g2) des Verfahrens A1-Hexarylen) vorgenommen werden. Auch hier ist die Verfahrensvariante c2bα) bevorzugt.

Die zweistufige Vorgehensweise c2) hat wie beim Verfahren B-Hexarylen den Vorteil, daß aufgrund der milderen Reaktionsbedingungen im ersten Cyclodehydrierungsschritt die Gefahr der unerwünschten Abspaltung von Alkylsubstituenten an aromatischen Resten R oder R' verringert wird.

Die erste Cyclodehydrierung (Schritt c2a)) wird analog Schritt c2a) des Verfahrens B-Hexarylen vorgenommen.

Verfahrenstechnisch geht man zweckmäßigerweise ebenfalls analog Schritt c2a) des Verfahrens B-Hexarylen vor.

Die Ausbeute in Schritt c2a) liegt im allgemeinen bei 30 bis 40%.

Der zweite Cyclodehydrierungsschritt c2b) zum Pentarylentetracarbonsäurediimid Ib wird ebenfalls vorzugsweise analog Schritt c2b) des Verfahrens B-Hexarylen, der wiederum analog Schritt g1) des Verfahrens A1-Hexarylen vorgenommen wird, durchgeführt (Schritt c2bα)). Selbstverständlich kann aber auch hier die zu Schritt g1) des Verfahrens A1-Hexarylen analoge Verfahrensvariante c2bβ) gewählt werden.

Verfahrenstechnisch geht man zweckmäßigerweise ebenfalls analog vor.

Die Reinigung des erhaltenen Pentarylentetracarbonsäurediimids Ib kann wie bei der einstufigen Verfahrensvariante c1) beschrieben erfolgen.

Die Ausbeute in Schritt c2bα) liegt im allgemeinen bei 80 bis 100%, die Ausbeute in Schritt c2bβ) beträgt üblicherweise 80 bis 95%.

Mit Hilfe des erfindungsgemäßen Verfahrens B-Pentarylen sind sowohl symmetrische als auch unsymmetrische Pentarylentetracarbonsäurediimide Ib (verschiedene Reste R an den beiden Imidstickstoffatomen und/oder verschiedene Reste R' in den beiden äußeren Perylenbausteinen) zugänglich.

Die erfindungsgemäßen Rylentetracarbonsäurediimide I zeigen starke Absorption im nahen Infrarotbereich bei Wellenlängen von 830 bis 975 nm und ergänzen damit den mit Hilfe der bisher bekannten Rylenverbindungen zugänglichen Spektralbereich auf vorteilhafte Weise.

Sie eignen sich für eine Vielzahl von Anwendungen, wie die Einfärbung von hochmolekularen organischen und anorganischen Materialien, z.B. von Lacken, Druckfarben und Kunststoffen, zur Herstellung im nahinfraroten Bereich des elektromagnetischen Spektrums absorbierender wäßriger Polymerisatdispersionen, zur Erzeugung für das menschliche Auge nicht sichtbarer, Infrarotlicht absorbierender Markierungen und Beschriftungen, als Infrarotabsorber für das Wärmemanagement, als IR-Laserstrahlenabsorbierende Materialien beim Schweißbehandeln von Kunststoffteilensowie als Aktivkomponenten in der Photovoltaik.

### Beispiele

### Beispiel 1: N,N'-Bis(2,6-diisopropylphenyl)-1,6,15,20-tetra[4-(1,1,3,3-tetramethylbutyl)-phenoxy]hexarylen-3,4:17,18-tetracarbonsäurediimid Ia'

### a) Herstellung von 1-(4,4,5,5-Tetramethyl-1,3,2-dioxaboran-2-yl)-5-nitronaphthalin IVb'

Eine Lösung von 6,0 g (23,8 mmol) 1-Brom-5-nitronaphthalin in 100 ml Dioxan wurde mit Argon gespült. Dann wurden 14,0 g (61,6 mmol) Kaliumacetat, 12,0 g (47,3 mmol) Bis(pinacolato)diboran und 0,8 g (0,97 mmol) [1,1'-Bis(diphenylphosphino)ferrocen]-palladium(II)chlorid zugegeben. Nach nochmaligem Spülen mit Argon wurde die Mischung auf 70°C erhitzt und 16 h bei dieser Temperatur gerührt. Nach Abkühlen auf Raumtemperatur wurde das Reaktionsgemisch mit Methylenchlorid extrahiert. Die organische Phase wurde zweimal mit Wasser gewaschen und über Magnesiumsulfat getrocknet. Dann wurde das Lösungsmittel unter Vakuum abdestilliert. Anschließend wurde das Rohprodukt einer Säulenchromatographie an Kieselgel mit Methylenchlorid als Eluens unterzogen.

Es wurden 6,9 g IVb' in Form eines blaßgelben Feststoffs erhalten, was einer Ausbeute von 97% entspricht.

Analytische Daten:
Schmelzpunkt: 111°C;
Elementaranalyse (C₁₆H₁₈BNO₄) (Gew.-% ber./gef.):
   C: 64,24/64,43; H : 6,07/6,14; N: 4,68/4,61;
¹H-NMR (250 MHz, CD₂Cl₂, 25°C): δ = 9,14 (dd, 1H, J = 8,5 u. 1,0 Hz); 8,58 (dd, 1H, J = 8,9 u. 1,0 Hz); 8,20 (dd, 1H, J = 7,0 u. 1,3 Hz); 8,15 (dd, 1H, J = 7,0 u. 1,3 Hz); 7,70 (t, 1H); 7,60 (t, 1H); 1,33 ppm (s, 12H);
¹³C-NMR (125 MHz, CD₂Cl₂, 25°C): δ = 147,6; 138,0; 137,2; 135,3; 128,6; 126,2; 125,2; 124,8; 123,6; 84,7; 83,6; 25,2 ppm;
IR (KBr): v (cm⁻¹) = 2990, 2932, 2365, 2341, 1526, 1347, 1300, 1149, 1126, 788; UV-Vis (CHCl₃): λₘₐₓ (ε) = 333 nm (4290 M⁻¹cm⁻¹);
MS (FD): m/z (rel. Int.) = 299,6 (100%) [M⁺].

### b) Herstellung von N-(2,6-Diisopropylphenyl)-1,6-bis[4-(1,1,3,3-tetramethylbutyl)-phenoxy]-9-(5-nitronaphthyl)perylen-3,4-dicarbonsäureimid V'

Zu einer unter Argon gerührten Mischung von 1,05 g (1,09 mmol) N-(2,6-Diisopropylphenyl)-1,6-bis[4-(1,1,3,3-tetramethylbutyl)phenoxy]-9-bromperylen-3,4-dicarbonsäure-imid IIIa', 1,03 g (3,44 mmol) 1-(4,4,5,5-Tetramethyl-1,3,2-dioxaboran-2-yl)-5-nitro-naphthalin IVb' und 40 ml Toluol wurden zuerst eine Lösung von 0,69 g (5,0 mmol) Kaliumcarbonat in 20 ml Wasser und 2 ml Ethanol und dann 0,06 g (0,051 mmol) Tetrakis(triphenylphosphin)palladium(0) zugegeben. Anschließend wurde die Mischung unter Argon auf 80°C erhitzt und 16 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit einer 1:1-Mischung von Toluol und Hexan als Eluens unterzogen.

Es wurden 1,15 g V' in Form eines roten Feststoffs erhalten, was einer Ausbeute von 100% entspricht.

Analytische Daten:
Schmelzpunkt: 202°C;
Elementaranalyse (C₇₂H₇₂N₂O₆) (Gew.-% ber./gef.):
   C: 81,48/81,53; H : 6,84/7,02; N: 2,64/2,70;
¹H-NMR (500 MHz, CD₂Cl₂, 25°C): δ = 9,50 (d, 1H, J = 8,2 Hz); 9,38 (dd, 1H, J = 6,6 u. 2,2 Hz); 8,62 (d, 1H, J = 8,9 Hz); 8,28 (d, 2H, J = 3,2 Hz); 8,18 (dd, 1H, J = 7,6 u. 1,0 Hz); 7,77-7,65 (m, 3H); 7,49 (d, 1H, J = 7,9 Hz); 7,39-7,11 (m, 10H); 7,00 (dd, 4H, J = 11,7 u. 8,4 Hz); 2,75 (sep, 2H, J = 6 Hz); 1,76 (d, 4H, J = 2,5 Hz); 1,40 (d, 12H, J = 2,2 Hz); 1,13 (dd, 12 H, J = 6,6 u. 1,9 Hz); 0,75 ppm (d, 18H, J = 2,2 Hz); ¹³C-NMR (Spinecho, 125 MHz, CD₂Cl₂, 25°C): δ = 163,7; 163,5; 154,6; 154,4; 153,7; 147,7; 147,1; 147,0; 146,5; 140,0; 139,2; 134,1; 133,3; 132,2; 133,1; 131,6; 130,1; 129,8; 129,7; 129,4; 129,2; 120,0; 128,6; 128,5; 128,4; 127,3; 127,0; 125,7; 125,0; 124,4; 124,3; 124,2; 124,1; 123,5; 123,4; 122,2; 119,0; 118,8; 57,5; 32,7; 31,7; 30,1; 29,5; 29,3; 24,2; 23,2; 23,1 ppm;
IR (KBr): v (cm⁻¹) = 2963, 2869, 2365, 2341, 1708, 1669, 1600, 1530, 1499, 1322, 1273, 1207, 1168, 877, 792;
UV-Vis (CHCl₃): λₘₐₓ (ε) = 517 (42030), 486 (27590), 420 (790), 345 (7790), 276 (34430) nm (M⁻¹cm⁻¹);
Fluoreszenz (CHCl₃): λₘₐₓ = 574 nm;
MS (FD): m/z (rel. Int.) = 1060,3 (100%) [M⁺].

### c) Herstellung von N-(2,6-Diisopropylphenyl)-1,6-bis[4-(1,1,3,3-tetramethylbutyl)-phenoxy]-11-nitroterrylen-3,4-dicarbonsäureimid VIa'

Eine Lösung von 1,0 g (0,94 mmol) V', 1,17 g (9,42 mmol) 1,5-Diazabicyclo[4.3.0]non-5-en und 0,45 g (4,7 mmol) Natrium-tert.-butylat in 7 ml Diethylenglykoldimethylether wurde in einem 25 ml-Schlenckrohr mit Argon gespült, auf 70°C erhitzt und 2 h bei dieser Temperatur gerührt. Nach Abkühlen auf 40°C wurde das Reaktionsprodukt mit 100 ml Wasser gefällt, abfiltriert, mit Wasser gewaschen und getrocknet. Auch wenn das Rohprodukt direkt ohne weitere Reinigung für den Folgeschritt eingesetzt werden kann, wurde es für analytische Zwecke einer Säulenchromatographie an Kieselgel mit einer 4:1-Mischung von Methylenchlorid und Hexan als Eluens unterzogen.

Es wurden 0,35 g VIa' in Form eines blauen Feststoffs erhalten, was einer Ausbeute von 35% entspricht.

Analytische Daten:
Schmelzpunkt: 363°C;
Elementaranalyse (C₇₂H₇₀N₂O₆) (Gew.-% ber./gef.):
   C: 81,63/80,78; H: 6,66/6,64; N: 2,64/2,58;
¹H-NMR (500 MHz, CD₂Cl₂, 25°C): δ = 9,13 (dd, 2H, J = 10,0 u. 8,5 Hz); 8,20 (d, 2H, J = 7,0 Hz); 8,25 (d, 1H, J = 8,5 Hz); 7,98 (m, 2H); 7,92 (m, 2H); 7,85 (d, J = 8,5 Hz); 7,48 (m, 6H); 7,34 (d, 2H, J = 7,9 Hz); 7,10 (dd, 4H, J = 8,5 u. 6,4 Hz); 2,78 (sep, 2H, J = 6,7 Hz); 1,76 (s, 4H); 1,40 (s, 12H); 1,14 ppm (d, 12H, J = 7,0 Hz);
¹³C-NMR (Spinecho, 125 MHz, CD₂Cl₂, 25°C): δ = 163,5; 154,7; 154,5; 153,7; 153,6; 147,3; 147,2; 146,5; 145,2; 136,4; 131,6; 131,4; 131,0; 130,9; 129,9; 129,8; 129,6;
129,4; 129,1; 128,6; 128,3; 128,0; 127,3; 126,7; 126,6; 126,3; 125,2; 124,4; 124,1; 124,0; 123,9; 123,5; 122,9; 122,6; 122,5; 122,2; 119,7; 118,6; 57,4; 38,7; 32,7; 32,3; 31,9; 31,7; 30,1; 29,8; 29,5; 24,2; 23,1; 14,3;
IR (KBr): v (cm⁻¹) = 2959, 2873, 2365, 2341, 1708, 1677, 1603, 1584, 1502, 1324, 1281, 1211, 1173, 811;
UV-Vis (CHCl₃): λₘₐₓ (ε) = 643 (82900), 598 (49160), 445 (6680), 279 (39450) nm (M⁻¹cm⁻¹);
Fluoreszenz (CHCl₃): λₘₐₓ = 702 nm;
MS (FD): m/z (rel. Int.) = 1059,6 (100%) [M⁺].

### d) Herstellung von N-(2,6-Diisopropylphenyl)-1,6-bis[4-(1,1,3,3-tetramethylbutyl)-phenoxy]-11-aminoterrylen-3,4-dicarbonsäureimid VIb'

Zu einer Lösung von 2,0 g (0,95 mmol) VIa' in 300 ml Ethanol wurden 700 mg (12,5 mmol) Eisenpulver gegeben. Nach Zutropfen von 20 ml konzentrierter Salzsäure wurde die Mischung auf Rückflußtemperatur erhitzt und 1 h bei dieser Temperatur gerührt. Nach Abkühlen auf Raumtemperatur wurde die Reaktionslösung zur Abtrennung des ungelösten Eisenpulvers über eine Filternutsche gegeben. Das Eisenpulver wurde mehrmals mit Methylenchlorid gewaschen. Die organische Phase wurde aus dem Filtrat abgetrennt, und das Lösungsmittel wurde unter Vakuum abgezogen.

Es wurden 0,80 g VIb' in Form eines dunkelblauen Feststoffs erhalten, was einer Ausbeute von 92% entspricht.

Analytische Daten:
MS (FD): m/z (rel. Int.) = 1029,1 (100%) [M⁺].

### e) Herstellung von N-(2,6-Diisopropylphenyl)-1,6-bis[4-(1,1,3,3-tetramethylbutyl)-phenoxy]-11-iodterrylen-3,4-dicarbonsäureimid VIc'

Zu einer Lösung von 0,65 g (0,63 mmol) Vlb' in einer Mischung von 8 ml Diethylether, 6 ml Tetrahydrofuran und 1 ml Acetonitril wurden 29 ml 4,5 m Salzsäure langsam zugetropft. Nach Kühlen auf -5 bis -10°C wurde eine Lösung von 4,0 g (58,0 mmol) Natriumnitrit in 8 ml Acetonitril und 12 ml Wasser über einen Zeitraum von 15 min zugetropft, so daß die Temperatur nicht über -5°C stieg. Nach weiterem 30minütigen Rühren bei dieser Temperatur wurde die Reaktionslösung in eine gekühlte Mischung von 10,0 g (66,7 mmol) Natriumiodid, 66 ml Acetonitril und 33 ml Wasser gegeben, weitere 2 h bei 0°C gerührt und dann langsam auf Raumtemperatur erwärmt. Die durch Extraktion mit Methylenchlorid gewonnene organische Phase wurde zuerst mit Natriumsulfitlösung und dann mit Wasser gewaschen, dann wurde das Methylenchlorid im Vakuum abgezogen. Das Rohprodukt wurde kurz über Kieselgel filtriert, mit Methylenchlorid gewaschen, im Vakuum getrocknet und ohne weitere Reinigung in ⁻Schritt f) eingesetzt. Es wurden 0,12 g VIc' in Form eines blauen Feststoffs erhalten, was einer Ausbeute von 17% entspricht.

Analytische Daten:
MS (FD): m/z (rel. Int.) = 1140 (100%) [M⁺].

### f) Herstellung von Bis[N-(2,6-diisopropylphenyl)-1,6-bis[4-(1,1,3,3-tetramethylbutyl)phenoxy]terrylen-3,4-dicarbonsäureimid] VII'

Zunächst wurde N-(2,6-Diisopropylphenyl)-1,6-bis[4-(1,1,3,3-tetramethylbutyl)phenoxy]-11-(4,4,5,5-tetramethyl-1,3,2-dioxaboran-2-yl)terrylen-3,4-dicarbonsäureimid VId' wie folgt hergestellt:
Zu einer Lösung von 100 mg (0,09 mmol) VIc' in 10 ml Toluol in einem 50 ml-Schlenckrohr wurden nacheinander 34 mg (0,15 mmol) Bis(pinacolato)diboran, 34 mg (0,30 mmol) Kaliumacetat und 10 mg (0,03 mmol) [1,1'-Bis(diphenylphosphino)ferrocen]palladium(II)chlorid gegeben. Die erhaltene Mischung wurde dann unter Argon auf 60°C erhitzt und über Nacht bei dieser Temperatur gehalten. Nach Abkühlen auf Raumtemperatur wurde das Lösungsmittel abdestilliert. Der feste Rückstand wurde einer Säulenfiltration an Kieselgel mit Methylenchlorid als Eluens unterzogen.

Es wurden 0,08 g VId' in Form eines dunkelblauen Feststoffs erhalten, was einer Ausbeute von 78% entspricht.

Das erhaltene VId' wurde ohne weitere Reinigung zur Herstellung von Bis[N-(2,6-diisopropylphenyl)-1,6-bis[4-(1,1,3,3-tetramethylbutyl)phenoxy]terrylen-3,4-dicarbonsäure-imid] VII' eingesetzt.

Zu einer unter Argon gerührten Lösung von 0,04 g (0,035 mmol) VIc' und 0,04 g (0,035 mmol) VId' in 2 ml Dioxan wurde zunächst eine Lösung von 0,027 g (0,20 mmol) Kaliumcarbonat in einer Mischung von 0,9 ml Wasser und 0,1 ml Ethanol und anschließend 0,010 g (0,009 mmol) Tetrakis(triphenylphosphin)palladium(0) zugegeben. Nach nochmaligem Spülen mit Argon wurde die Mischung 24 h bei 70°C gerührt. Das durch Abdestillieren des Lösungsmittels im Vakuum erhaltene Rohprodukt wurde einer Säulenfiltration an Kieselgel mit einer 1:1-Mischung von Methylenchlorid und Hexan als Eluens unterzogen und ohne weitere Reinigung in Schritt g) eingesetzt.

Es wurden 0,06 g VII' in Form eines blauen Feststoffs erhalten, was einer Ausbeute von 85% entspricht.

### g) Cyclodehydrierung zum N,N'-Bis(2,6-diisopropylphenyl)-1,6,15-20-tetra[4-(1,1,3,3-tetramethylbutyl)phenoxy]hexarylen-3,4:17,18-tetracarbonsäurediimid Ia'

Eine Mischung von 0,050 g (0,025 mmol) VII', 0,105 g (0,76 mmol) Kaliumcarbonat und 3 ml Ethanolamin wurde mit Argon gespült, auf 135°C erhitzt und 20 h bei dieser Temperatur gerührt. Das nach Abkühlen auf 40°C durch Fällen der Reaktionslösung auf Wasser gefällte Reaktionsprodukt wurde abfiltriert, mit Hexan gewaschen und im Vakuum getrocknet.

Es wurden 0,42 g Ia' in Form eines grünen Feststoffs erhalten, was einer Ausbeute von 84% entspricht.
Analytische Daten:
Schmelzpunkt: > 400°C
IR (KBr): v (cm⁻¹) = 2957, 2360, 2341, 1702, 1666, 1589, 1567, 1537, 1503, 1472, 1409, 1364, 1320, 1279, 1211, 1179, 1105, 1056, 875, 837;
UV-Vis (CHCl₃): λₘₐₓ (ε) = 953 nm (293000), 849 (110300), 763 (35500) (M⁻¹cm⁻¹);
MS (MALDI-TOF): m/z (rel. Int.) = 2024,0 (100%) [M⁺].

### Beispiel 2: N,N'-Bis(2,6-diisopropylphenyl)-1,6,13,18-tetra[4-(1,1,3,3-tetramethylbutyl)-phenoxy]pentarylen-3,4:15,16-tetracarbonsäurediimid Ib'

### a) Herstellung von N-(2,6-Diisopropylphenyl)-1,6-bis[4-(1,1,3,3-tetramethylbutyl)-phenoxy]-9-(4,4,5,5-tetramethyl-1,3,2-dioxaboran-2-yl)perylen-3,4-dicarbon-säureimid IVa'

Eine Lösung von 0,97 g (1,0 mmol) lila in 100 ml Toluol wurde mit Argon gespült. Anschließend wurden 0,33 g (3,0 mmol) Kaliumacetat, 0,34 g (1,5 mmol) Bis(pinacolato)-diboran und 0,04 g (0,1 mmol) [1,1'-Bis(diphenylphosphino)ferrocen]palladium(II)-chlorid zugegeben. Nach nochmaligem Spülen mit Argon wurde die Mischung auf 80°C erhitzt und 16 h bei dieser Temperatur gerührt. Nach Abkühlen auf Raumtemperatur wurde das Lösungsmittel abgezogen. Das Rohprodukt wurde einer Säulenchromatographie an Kieselgel mit Methylenchlorid als Eluens unterzogen.

Es wurden 0,67 g IVa' in Form eines roten Feststoffs erhalten, was einer Ausbeute von 65% entspricht.

Analytische Daten:
Schmelzpunkt: 182°C;
Elementaranalyse (C₆₈H₇₈BNO₆) (Gew.-% ber./gef.):
   C: 80,37/80,16; H : 7,74/7,72; N: 1,38/1,41;
¹H-NMR (250 MHz, CD₂Cl₂, 25°C): δ = 9,35 (dd, 1H, J = 8,0 Hz); 9,28 (d, 1H, J = 8,0 Hz); 8,89 (dd, 1H, J = 8,0 Hz); 8,22 (s, 1H); 8,21 (s, 1H); 8,13 (d, 1H, J = 8,0 Hz); 7,64 (t, 1H, J = 7,5 Hz); 7,40 (m, 5H); 7,30 (d, 2H, J = 8,0 Hz); 7,06 (m, 4H); 2,69 (m, 2H); 1,72 (s, 4H); 1,42 (s, 12H); 1,37 (s, 12H); 1,09 (d, 12H, J = 7,5 Hz); 0,71 (s, 18H); ¹³C-NMR (Spinecho, 62,5 MHz, CD₂Cl₂, 25°C): δ = 163,6; 154,2; 154,0; 153,7; 146,7; 146,6; 146,4; 137,3; 136,2; 132,2; 131,5; 131,4; 130,3; 129,7; 129,4; 128,9; 128,3; 127,9; 127,8; 127,5; 127,1; 124,6; 124,3; 123,4; 122,3; 121,7; 118,5; 118,3; 84,5; 57,3; 38,5; 32,5; 31,8; 31,6; 29,3; 25,1; 24,0;
IR (KBr): v (cm⁻¹) = 2958, 2361, 1707, 1671, 1598, 1503, 1467, 1412, 1329, 1272, 1138, 1013, 872, 770, 676, 579;
UV-Vis (CHCl₃): λₘₐₓ (ε) = 520 (42060), 490 (27600), 421 (7800) nm (M⁻¹cm⁻¹);
MS (FD): m/z (rel. Int.) = 1015,8 (100%) [M⁺].

### b) Herstellung von N-(2,6-Diisopropylphenyl-1,6-bis[4-(1,1,3,3-tetramethylbutyl)-phenoxy]-11-(9-[N-(2,6-diisopropylphenyl)]-1,6-bis[4-(1,1,3,3-tetramethylbutyl)-phenoxy]perylen-3,4-dicarbonsäureimid)terrylen-3,4-dicarbonsäureimid VIII'

Zu einer unter Argon gerührten Mischung von 0,09 g (0,92 mmol) IVa' und 0,06 g (0,05 mmol) N-(2,6-Diisopropylphenyl)-1,6-bis[4-(1,1,3,3-tetramethylbutyl)phenoxy]-11-iod-terrylen-3,4-dicarbonsäureimid VIc' in 3 ml Toluol wurden zuerst eine Lösung von 0,03 g (0,2 mmol) Kaliumcarbonat in 0,9 ml Wasser und 0,1 ml Ethanol und dann 0,01 g (0,01 mmol) Tetrakis(triphenylphosphin)palladium(0) zugegeben. Die Mischung wurde unter Argon auf 70°C erhitzt und 72 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit einer 1:1-Mischung von Methylenchlorid und Hexan als Eluens unterzogen.

Es wurden 16 mg VIII' in Form eines roten Feststoffs erhalten, was einer Ausbeute von 17% entspricht.

Analytische Daten:
MS (FD): m/z (rel. Int.) = 1902,6 (100%) [M⁺].

### c) Cyclodehydrierung zum N,N'-Bis(2,6-diisopropylphenyl)-1,6,13,18-tetra[4-(1,1,3,3-tetramethylbutyl)phenoxy]pentarylen-3,4:15,16-tetracarbonsäurediimid Ib'

Eine Mischung von 0,016 g (0,008 mmol) VIII', 0,053 g (0,38 mmol) Kaliumcarbonat und 2 ml Ethanolamin wurde mit Argon gespült, auf 135°C erhitzt und 16 h bei dieser Temperatur gerührt. Das nach Abkühlen auf 40°C durch Fällen der Reaktionslösung auf 100 ml Wasser gefällte Reaktionsprodukt wurde abfiltriert, mit Wasser gewaschen und getrocknet. Das Rohprodukt wurde einer Säulenchromatographie an Kieselgel mit einer 3:2-Mischung von Methylenchlorid und Hexan als Eluens unterzogen.

Es wurden 15 mg Ib' in Form eines schwarzgrünen Feststoffs erhalten, was einer Ausbeute von 94% entspricht.

Analytische Daten:
¹H-NMR (500 MHz, CD₂Cl₂, 25°C): δ = 9,22 (d, 4H, J = 8,5 Hz); 8,21 (s, 4H); 7,99 (m, 8H); 7,47 (t, 2H, J = 8,2 Hz); 7,38 (d, 8H, J = 8,8 Hz); 7,32 (d, 4H, J = 7,6 Hz); 7,03 (d, 8H, J = 8,8 Hz); 2,78 (sep, 4H, J = 6,7 Hz); 1,70 (s, 8H); 1,33 (s, 24H); 1,10 (d, 24H, J = 7,1 Hz); 0,72 (s, 36H);
UV-Vis (CHCl₃): λₘₐₓ (ε) = 877 (235200), 783 (110300), 763 (91000), 706 (25400) nm (M⁻¹cm⁻¹);
MS (FD): m/z (rel. Int.) = 1900,7 (100%) [M⁺].

### Beispiel 3: N,N'-Bis(1-Heptyloctyl)pentarylen-3,4:15,16-tetracarbonsäurediimid Ib"

### a) Herstellung von N-(1-Heptyloctyl)-9-(4,4,5,5-tetramethyl-1,3,2-dioxaboran-2-yl)-perylen-3,4-dicarbonsäureimid IVa'

Zu einer Lösung von 1,2 g (2 mmol) N-(1-Heptyloctyl)-9-bromperylen-3,4-dicarbon-säureimid lila" in 20 ml Dioxan in einem 50 ml-Schlenckrohr wurden nacheinander 558 mg (2,5 mmol) Bis(pinacolato)diboran, 558 mg (5,3 mmol) Kaliumacetat und 44 mg (0,1 mmol) [1,1'-Bis(diphenylphosphino)ferrocen]palladium(II)chlorid zugegeben. Die erhaltene Mischung wurde dann unter Argon auf 70°C erhitzt und über Nacht bei dieser Temperatur gehalten. Nach Abkühlen auf Raumtemperatur wurde das Produkt mit Methylenchlorid extrahiert und mit Wasser gewaschen. Dann wurde das Lösungsmittel abdestilliert. Der feste Rückstand wurde einer Säulenfiltration an Kieselgel mit Methylenchlorid als Eluens unterzogen.

Es wurden 1,0 g IVa' in Form eines roten Feststoffs erhalten, was einer Ausbeute von 78% entspricht.

Analytische Daten:
Schmelzpunkt: 213°C;
¹H-NMR (300 MHz, THF-d₈, 25°C): δ = 8,87 (d, 1H, J = 7,7 Hz); 8,55-8,47 (m, 6H); 8,15 (d, 1H, J = 7,7 Hz); 7,59 (t, 1H, J = 7,7 Hz); 5,27-5,17 (m, 1H); 2,40-2,28 (m, 2H); 1,84-1,77 (m, 2H); 1,44 (s, 12H); 1,34-1,24 (m, 20 H); 0,85-0,81 (t, 6H, J = 6,8 Hz) ppm; ¹³C-NMR (75 MHz, THF-d₈, 25°C): δ = 165,2; 164,3; 139,0; 137,8; 137,3; 132,8; 132,2; 131,5; 130,6; 130,0; 128,6; 127,8; 127,4; 124,4; 123,3; 123,2; 122,0; 121,9; 121,3; 84,9; 54,5; 33,2; 32,8; 30,5; 30,2; 27,8; 23,5; 14,4 ppm;
IR (KBr): v (cm⁻¹) = 2925, 2854, 2362, 2337, 1691, 1653, 1592, 1507, 1461, 1416, 1376, 1332, 1272, 1246, 1209, 1142, 1113, 1068, 966, 858, 811, 754, 674;
UV-Vis (CHCl₃): λₘₐₓ (ε) = 514 (47400), 489 (45200) nm (M⁻¹cm⁻¹);
Fluoreszenz (CHCl₃): λₘₐₓ = 577, 546 nm;
MS (FD): m/z (rel. Int.) = 657,2 (100%) [M⁺].

### b) Herstellung von Naphthalin-1,4-bis[N-(1-heptyloctyl)perylen-3,4-dicarbonsäure-imid] XIIa'

Zu einer unter Argon gerührten Mischung von 1,00 g (1,52 mmol) IVa' und 0,22 g (0,76 mmol) 1,4-Dibromnaphthalin IXc' in 40 ml Toluol wurden zuerst eine Lösung von 0,63 g (4,6 mmol) Kaliumcarbonat in 20 ml Wasser und 2 ml Ethanol und dann 0,09 g (0,076 mmol) Tetrakis(triphenylphosphin)palladium(0) zugegeben. Anschließend wurde die Mischung unter Argon auf 80°C erhitzt und 16 h bei dieser Temperatur gerührt. Nach Abkühlen auf Raumtemperatur wurde die organische Phase abgetrennt und das Lösungsmittel im Vakuum abgezogen. Das Rohprodukt wurde einer Säulenchromatographie an Kieselgel mit Toluol als Eluens unterzogen.

Es wurden 0,45 g XIIa' in Form eines roten Feststoffs erhalten, was einer Ausbeute von 72% entspricht.

Analytische Daten:
Schmelzpunkt: 330,5°C;
¹H-NMR (500 MHz, THF-d₈, 25°C): δ = 8,76 (d, 1H, J = 7,7 Hz); 8,75 (d, 1H, J = 7,7
Hz); 8,67 (d, 1H, J = 8,5 Hz); 8,66 (d, 1H, J = 8,5 Hz); 8,60-8,55 (m, 8H); 7,81 (d, 1H, J = 6,8 Hz); 7,76 (d, 1H, J = 7,7 Hz); 7,73 (s, 2H); 7,68 (d, 1H, J = 7,7 Hz); 7,61-7,59 (m, 3H); 7,58 (t, 1H, J = 7,7 Hz); 7,50 (t, 1H, J = 8,5 Hz); 7,34 (dd, 2H, J = 4,3 Hz); 5,24 (m, 2H); 2,33 (m, 4H); 1,85 (m, 4H); 1,38-1,26 (m, 4H); 0,85 (m, 12H) ppm;
¹³C-NMR (75 MHz, THF-d₈, 25°C): δ = 164,37; 142,15; 139,18; 137,70; 137,58; 134,89; 133,86; 132,53; 131,78; 130,88; 130,69; 130,35; 130,22; 129,25; 128,36; 128,03; 127,54; 127,30; 124,86; 124,41; 123,13; 122,36; 121,54; 121,44; 54,55; 33,25; 32,81; 30,53; 30,21; 27,83; 23,51; 14,40 ppm;
IR (KBr): v (cm⁻¹) = 2922, 2852, 2362, 1693, 1652, 1591, 1572, 1504, 1458, 1405, 1351, 1291, 1244, 1170, 1107, 843, 810, 758;
UV-Vis (CHCl₃): λₘₐₓ (ε) = 521 (91200), 496 (75500), 266 (65400) nm (M⁻¹cm⁻¹); Fluoreszenz (CHCl₃): λₘₐₓ = 561 nm;
MS (FD): m/z (rel. Int.) = 1188,5 (100%) [M⁺].

### c) Cyclodehydrierung zum N,N'-Bis(1-Heptyloctyl)pentarylen-3,4:15,16-tetracarbon-säurediimid Ib"

Eine Mischung von 0,10 g (0,084 mmol) XIIa', 0,09 g (0,67 mmol) wasserfreiem Aluminiumchlorid und 2 ml Chlorbenzol wurde 20 min bei 75°C gerührt. Nach Einengung des Losungsmittelvolumens unter vermindertem Druck wurde die verbleibende Flüssigkeit mit Diethylether verdünnt, und der Niederschlag wurde abfiltriert. Der verbliebende Feststoff wurde nachfolgend mit verdünnter Salzsäure hydrolysiert, zuerst mit Wasser, dann mit Aceton und abschließend mit Methylenchlorid gewaschen.

Es wurden 24 mg Ib" in Form eines schwarzgrünen Feststoffs erhalten, was einer Ausbeute von 24% entspricht.

Analytische Daten:
Schmelzpunkt: > 400°C;
IR (KBr): v (cm⁻¹) = 2921, 2851, 2362, 1689, 1648, 1591, 1564, 1456, 1386, 1340, 1270, 1210, 1104, 1057, 837, 806;
UV-Vis (THF): λₘₐₓ (ε) = 831 (216000), 758 (124600), 673 (59600) nm (M⁻¹cm⁻¹); MS (MALDI-TOF) : m/z (rel. Int.) = 1182,0 (100%) [M⁺].

### Beispiel 4: N,N'-Bis(2,6-diisopropylphenyl)-1,6,15,20-tetra[4-(1,1,3,3-tetramethylbutyl)-phenoxy]hexarylen-3,4:17,18-tetracarbonsäurediimid la'

### a) Herstellung von Perylen-3,9- und -3,10-bis[N-(2,6-diisopropylphenyl)-1,6-di[4-(1,1,3,3-tetramethylbutyl)phenoxy]perylen-3,4-dicarbonsäureimid] X'

Zu einer Lösung von 1,0 g (0,98 mmol) N-(2,6-Diisopropylphenyl)-1,6-bis[4-(1,1,3,3-tetramethylbutyl)phenoxy]-9-(4,4,5,5-tetramethyl-1,3,2-dioxaboran-2-yl)perylen-3,4-di-carbonsäureimid IVa' und 0,2 g (0,49 mmol) eines Gemischs von 3,9- und 3,10-Dibromperylen IXa' in 40 ml Toluol wurden nacheinander eine Lösung von 0,415 g (3,0 mmol) Kaliumcarbonat in 20 ml Wasser und 2 ml Ethanol und 0,057 g (0,049 mmol) Tetrakis(triphenylphosphin)palladium(0) zugegeben. Die Mischung wurde unter Argon auf 80°C erhitzt und 16 h bei dieser Temperatur gerührt. Nach Abkühlen auf Raumtemperatur wurde die organische Phase abgetrennt und das Lösungsmittel unter vermindertem Druck abdestilliert. Das Rohprodukt wird einer Säulenchromatographie an Kieselgel mit einer 1:1-Mischung von Toluol und Hexan als Eluens unterzogen.

Es wurden 0,99 g X' in Form eines roten Feststoffs erhalten, was einer Ausbeute von 79% entspricht.

Analytische Daten:
Schmelzpunkt: 300°C;
¹H-NMR (700 MHz, CD₂Cl₂, 25°C): δ = 9,52 (d, 2H, J = 6,73 Hz); 9,40 (dd, 2H, J = 4,3 u. 3,3 Hz); 8,43 (d, 1H, J = 7,8 Hz); 8,38 (d, 1H, J = 7,8 Hz); 8,31-8,26 (m, 5H); 7,69-7,67 (m, 4H); 7,60-7,57 (m, 2H); 7,49-7,42 (m, 13H); 7,38-7,36 (m, 4H); 7,33 (d, 4H, J = 7,8 Hz); 7,15 (d, 4H, J = 6,8 Hz); 7,10 (d, 4H, J = 7,3 Hz); 2,74 (sep, 4H, J = 6,6 Hz); 1,77 (s, 4H); 1,76 (s, 4H); 1,40 (d, 24H, J = 7,1 Hz); 1,14-1,13 (m, 24H); 0,76 (s, 18H); 0.75 (s, 18H);
¹³C-NMR (Spinecho, 175 MHz, CD₂Cl₂, 25°C): δ = 163,7; 154,3; 154,2; 153,8; 147,0; 146,9; 146,5; 141,1; 138,3; 138,2; 134,4; 133,2; 132,2; 131,8; 131,7; 131,6; 131,5; 130,1; 129,7; 129,3; 129,2; 129,1; 128,9; 128,5; 128,4; 128,3; 128,0; 127,6; 127,4; 127,3; 127,1; 127,0; 126,9; 124,5; 124,4; 123,5; 122,1; 122,0; 121,2; 121,0; 120,6; 129,5; 118,8; 118,6; 57,5; 38,7; 38,6; 32,7; 31,9; 31,7; 30,1; 29,5; 24,1;
IR (KBr): v (cm⁻¹) = 2957, 2870, 2362, 2336, 1707, 1671, 1598, 1502, 1466, 1412, 1335, 1272, 1210, 1173, 1014, 915, 874, 834, 810;
UV-Vis (CHCl₃): λₘₐₓ (ε) = 531 (133920), 500 (841009), 454 (38830), 421 (34400) nm (M⁻¹cm⁻¹);
Fluoreszenz (CHCl₃): λₘₐₓ = 660 nm;
MS (FD): m/z (rel. Int.) = 2029,6 (100%) [M⁺].

### b) Herstellung von N-(2,6-Diisopropylphenyl-1,6-bis[4-(1,1,3,3-tetramethylbutyl)-phenoxy]-13-(9-[N-(2,6-diisopropylphenyl)]-1,6-bis[4-(1,1,3,3-tetramethylbutyl)-phenoxy]perylen-3,4-dicarbonsäureimid)quaterrylen-3,4-dicarbonsäureimid XI'

Zu einer Lösung von 1,3 g (0,64 mmol) X' in 20 ml Methylenchlorid wurde eine Lösung von 0,83 g (5,14 mmol) Eisen(III)chlorid in 4 ml Nitromethan unter Argon zugetropft. Nach einer Reaktionszeit von 24 h bei Raumtemperatur wurde das Lösungsmittel abgezogen, die Reaktionsmischung mit wäßriger Salzsäure versetzt, filtriert und mit Wasser neutral gewaschen. Das getrocknete Rohprodukt wurde direkt ohne weitere Reinigung für den Folgeschritt eingesetzt.

Es wurden 0,28 g XI' in Form eines braunen Feststoffs erhalten, was einer Ausbeute von 22% entspricht.

### c) Cyclodehydrierung zum N,N'-Bis(2,6-diisopropylphenyl)-1,6,15,20-tetra[4-(1,1,3,3-tetramethylbutyl)phenoxy]hexarylen-3,4:17,18-tetracarbonsäuredimid Ia'

Eine Mischung von 0,2 g (0,1 mmol) XI', 0,11 g (0,8 mmol) Kaliumcarbonat und 4 ml Ethanolamin wurde unter Argon auf 130°C erhitzt und 16 h bei dieser Temperatur gerührt. Nach Abkühlen auf Raumtemperatur wurde die Reaktionsmischung mit 20 ml Wasser versetzt. Der gebildete Niederschlag wurde abfiltriert, mit Wasser gewaschen und getrocknet. Das Rohprodukt wurde einer Säulenchromatographie an Kieselgel mit Methylenchlorid als Eluens unterzogen.

Es wurden 0,17 g Ia' in Form eines grünen Feststoffs erhalten, was einer Ausbeute von 87% entspricht.

Analytische Daten:
Schmelzpunkt: > 400°C;
IR (KBr): v (cm⁻¹) = 2957, 2360, 2341, 1702, 1666, 1589, 1567, 1537, 1503, 1472, 1409, 1364, 1320, 1279, 1211, 1179, 1105, 1056, 875, 837;
UV-Vis (CHCl₃): λₘₐₓ (ε) = 953 (293000), 849 (110300), 763 (35500) nm (M⁻¹cm⁻¹);
MS (MALDI-TOF): m/z (rel. Int.) = 2024,0 (100%) [M⁺].

## Patentansprüche

1. Rylentetracarbonsäurediimide der allgemeinen Formel I 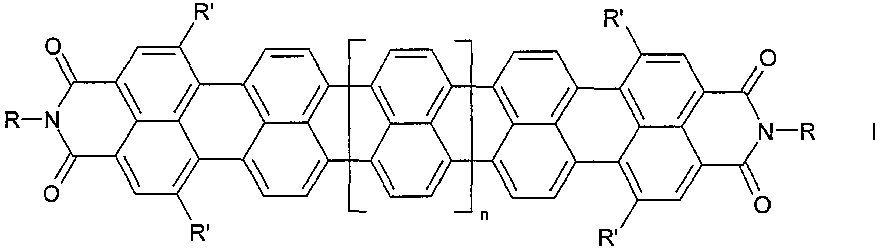 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff; C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆Alkyl substituiert sein kann;
n 1 oder 2.

2. Rylentetracarbonsäurediimide der allgemeinen Formel I nach Anspruch 1, in der die Variablen folgende Bedeutung haben:
R gleiche Reste:
Wasserstoff;
C₁-C₃₀-Alkyl, dessen Kohlenstoffkette durch eine oder mehrere Gruppierungen -O- und/oder -CO- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann; C₅-C₈-Cycloalkyl, das durch C₁-C₆-Alkyl ein- oder mehrfach substituiert sein kann;
Phenyl, Naphthyl, Pyridyl oder Pyrimidyl, das jeweils durch C₁-C₁₈-Alkyl, C₁-C₆-Alkoxy, Cyano, Nitro, Halogen, -CONR²R³, -SO₂R² und/oder Phenyl- oder Naphthylazo, das jeweils durch C₁-C₁₀-Alkyl, C₁-C₆-Alkoxy oder Cyano substituiert sein kann, ein- oder mehrfach substituiert sein kann;
R' gleiche Reste:
Wasserstoff; Brom;
Phenoxy, Phenylthio, Pyridyloxy, Pyrimidyloxy, Pyridylthio oder Pyrimidylthio, das jeweils ein- oder mehrfach durch C₁-C₁₂-Alkyl oder C₁-C₁₂-Alkoxy substituiert sein kann;
R¹ Wasserstoff oder C₁-C₆-Alkyl;
R², R³ unabhängig voneinander Wasserstoff; C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann;
n 1 oder 2.

3. Verfahren zur Herstellung von Hexarylentetracarbonsäurediimiden der allgemeinen Formel Ia 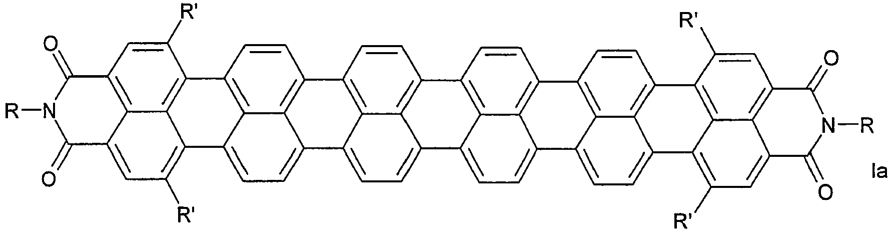 in der die Variablen die in Anspruch 1 genannte Bedeutung haben, R' jedoch nicht Wasserstoff bedeutet, **dadurch gekennzeichnet, daß** man
a) ein Diboran der allgemeinen Formel II 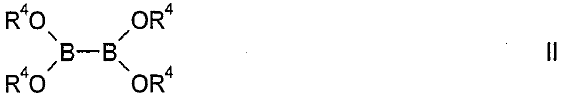 in der die Reste R⁴ gleich oder verschieden sind und unabhängig voneinander Wasserstoff, C₁-C₃₀-Alkyl, C₅-C₈-Cycloalkyl, Aryl oder Hetaryl bedeuten, wobei die an jeweils einem Boratom befindlichen Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
in Gegenwart eines aprotischen organischen Lösungsmittels, eines Übergangsmetallkatalysators und einer Base mit
a1) einem 9-Bromperylen-3,4-dicarbonsäureimid der allgemeinen Formel lila 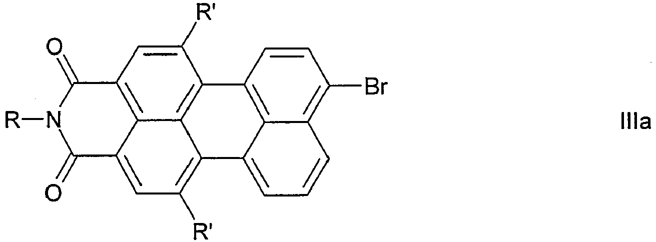 oder
a2) einem Naphthalinderivat der allgemeinen Formel IIIb 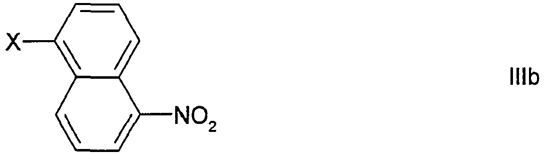 in der X Halogen, C₁-C₁₂-Alkylsulfonyl, dessen Alkylrest ein- oder mehrfach durch Halogen substituiert sein kann, oder C₆-C₁₈-Arylsulfonyl bedeutet, umsetzt,
b1) das in Schritt a1) gebildete 9-(Dioxaborolan-2-yl)perylen-3,4-dicarbonsäureimid der allgemeinen Formel IVa 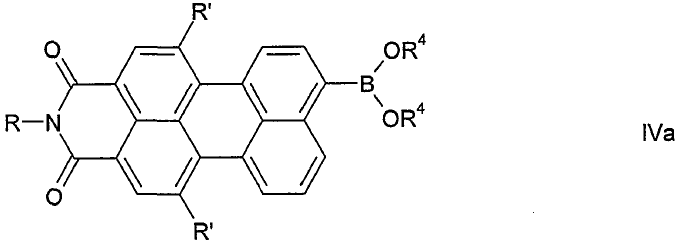 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Naphthalinderivat IIIb unterwirft
oder
b2) das in Schritt a2) gebildete 1-(Dioxaborolan-2-yl)-5-nitronaphthalin der allgemeinen Formel IVb 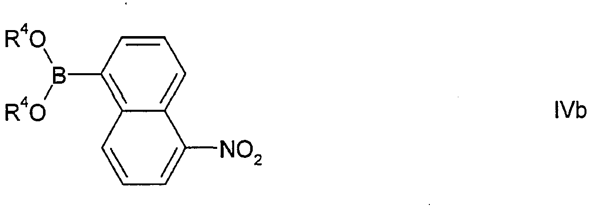 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid lila unterwirft,
c) das in Schritt b1) bzw. b2) gebildete 9-(5-Nitronaphthyl)perylen-3,4-dicarbonsäureimid der allgemeinen Formel V 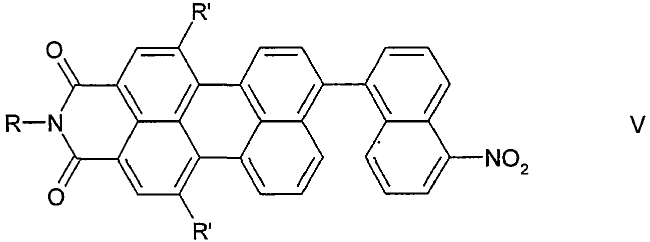 einer Cyclodehydrierung in Gegenwart eines basenstabilen, hochsiedenden, organischen Lösungsmittels sowie einer alkali- oder erdalkalimetallhaltigen Base und einer stickstoffhaltigen Hilfsbase unterzieht,
d) das in Schritt c) gebildete 11-Nitroterrylen-3,4-dicarbonsäureimid der allgemeinen Formel VIa 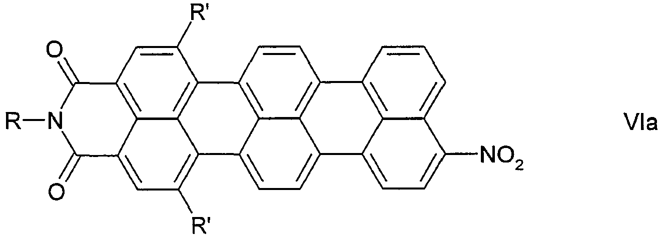 mit nascierendem Wasserstoff reduziert,
e) das in Schritt d) gebildete 11-Aminoterrylen-3,4-dicarbonsäureimid der allgemeinen Formel VIb 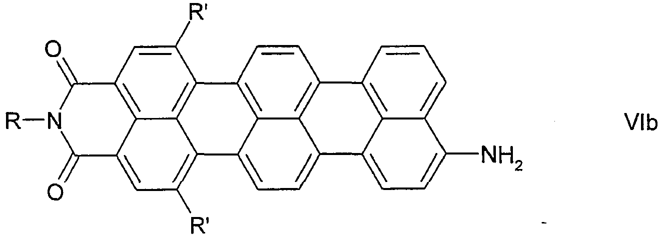 diazotiert und das gebildete Diazoniumsalz mit einem Metallbromid oder -iodid umsetzt,
f) das in Schritt e) gebildete 11-Halogenterrylen-3,4-dicarbonsäureimid der allgemeinen Formel VIc 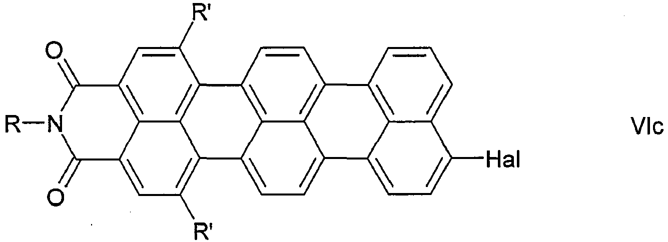 in der Hal Brom oder Iod bedeutet,
f1) in Gegenwart eines organischen Übergangsmetallkomplexes als Katalysator, freier Ligandmoleküle und eines aprotischen Lösungsmittels zu einem Bisterrylenderivat der allgemeinen Formel VII 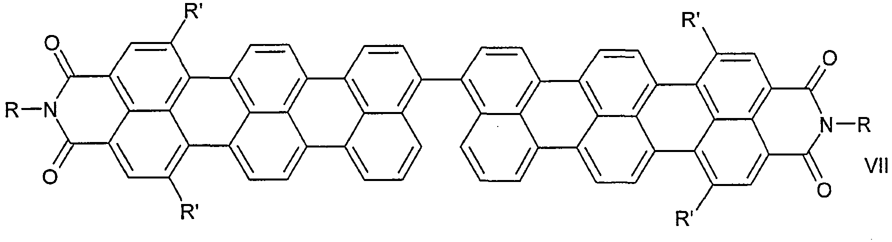 kuppelt
oder
f2) in Gegenwart von 30 bis 70 mol-%, bezogen auf das 11-Halogenterrylen-3,4-dicarbonsäureimid Vlc, eines Diborans II, eines Übergangsmetallkatalysators, einer Base und eines aprotischen organischen Lösungsmittels ohne Zwischenisolierung des in situ gebildeten 11-(Dioxaborolan-2-yl)terrylen-3,4-dicarbonsäureimids der allgemeinen Formel VId 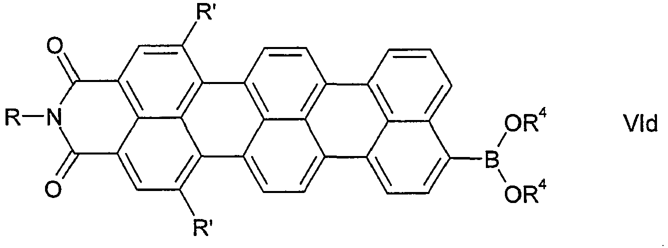 gemäß einer Suzuki-Kupplungsreaktion zum Bisterrylenderivat VII umsetzt und
g) das Bisterrylenderivat VII durch Cyclodehydrierung
g1) in einem Hydroxy- und Aminofunktionen aufweisenden und eine im wesentlichen ungelöste Base enthaltenden organischen Reaktionsmedium oder
g2) in Gegenwart eines basenstabilen, hochsiedenden, organischen Lösungsmittels sowie einer alkali- oder erdalkalimetallhaltigen Base und einer stickstoffhaltigen Hilfsbase
in das Hexarylentetracarbonsäurediimid la überführt.

4. Verfahren zur Herstellung von Hexarylentetracarbonsäurediimiden der allgemeinen Formel Ia 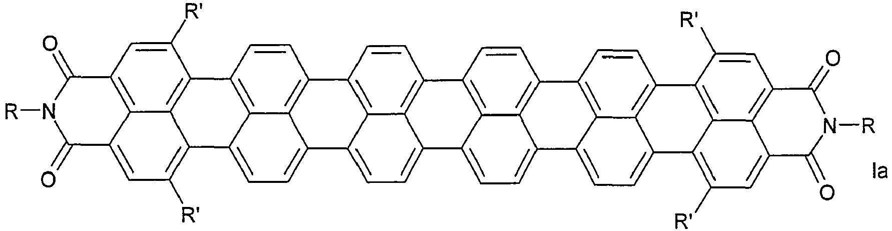 in der die Variablen die in Anspruch 1 genannte Bedeutung haben, R' jedoch nicht Wasserstoff bedeutet, **dadurch gekennzeichnet, daß** man
a) ein Diboran der allgemeinen Formel II 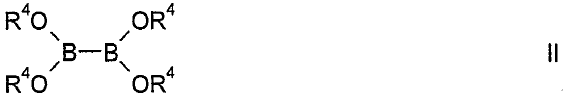 in der die Reste R⁴ gleich oder verschieden sind und unabhängig voneinander Wasserstoff, C₁-C₃₀-Alkyl, C₅-C₈-Cycloalkyl, Aryl oder Hetaryl bedeuten, wobei die an jeweils einem Boratom befindlichen Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
in Gegenwart eines aprotischen organischen Lösungsmittels, eines Übergangsmetallkatalysators und einer Base mit einem 11-Halogenterrylen-3,4-dicarbonsäureimid der allgemeinen Formel VIc 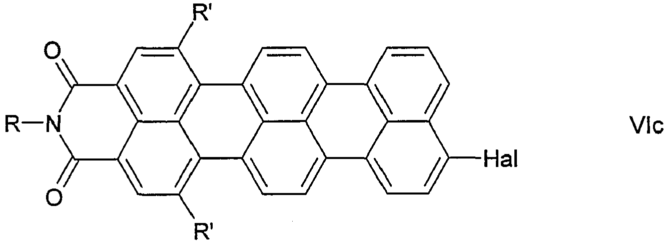 in der Hal Brom oder Iod bedeutet, umsetzt,
b) das in Schritt a) gebildete 11-(Dioxaborolan-2-yl)terrylen-3,4-dicarbonsäureimid der allgemeinen Formel VId 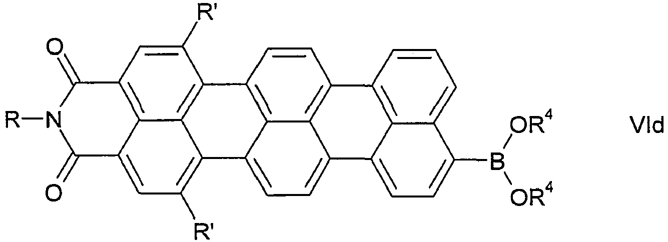 in Gegenwart eines organischen Lösungsmittels, gewünschtenfalls im Gemisch mit Wasser, sowie eines Übergangsmetallkatalysators und einer Base einer Suzuki-Kupplungsreaktion mit einem 11-Halogenterrylen-3,4-dicarbonsäureimid VIc unterwirft und
c) das in Schritt b) gebildete Bisterrylenderivat der allgemeinen Formel VII 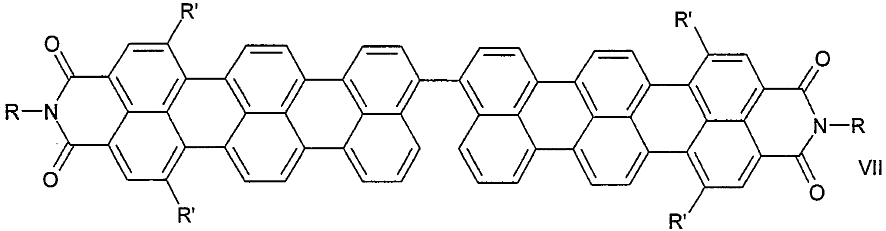 durch Cyclodehydrierung
c1) in einem Hydroxy- und Aminofunktionen aufweisenden und eine im wesentlichen ungelöste Base enthaltenden organischen Reaktionsmedium
oder
c2) in Gegenwart eines basenstabilen, hochsiedenden, organischen Lösungsmittels sowie einer alkali- oder erdalkalimetallhaltigen Base und einer stickstoffhaltigen Hilfsbase
in das Hexarylentetracarbonsäurediimid Ia überführt.

5. Verfahren zur Herstellung von Pentarylentetracarbonsäurediimiden der allgemeinen Formel Ib 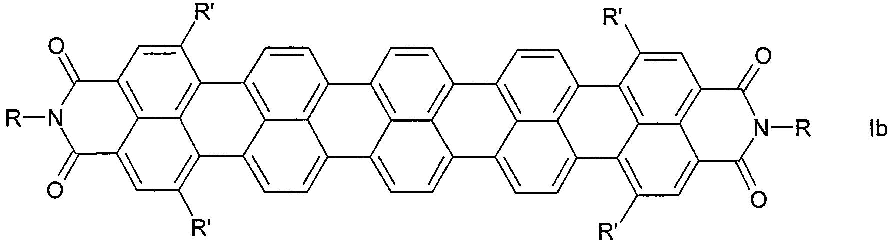 in der die Variablen die in Anspruch 1 genannte Bedeutung haben, R' jedoch nicht Wasserstoff bedeutet, **dadurch gekennzeichnet, daß** man
a) ein 11-Halogenterrylen-3,4-dicarbonsäureimid der allgemeinen Formel VIc 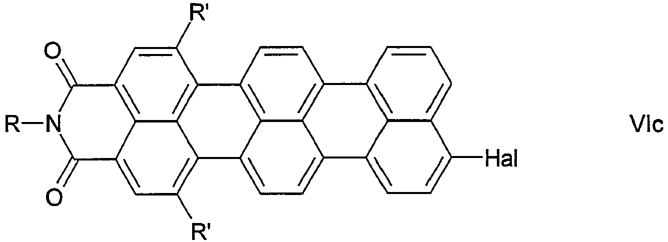 in der Hal Brom oder Iod bedeutet, in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-(Dioxaborolan-2-yl)perylen-3,4-dicarbonsäureimid der allgemeinen Formel IVa 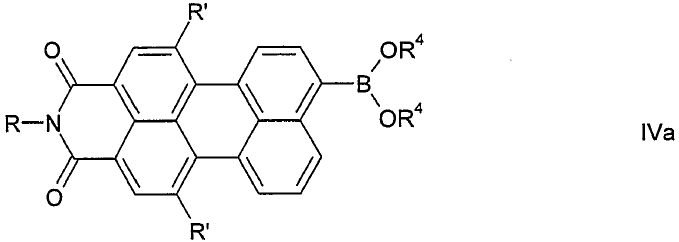 in der die Reste R⁴ gleich oder verschieden sind und unabhängig voneinander Wasserstoff, C₁-C₃₀-Alkyl, C₅-C₈-Cycloalkyl, Aryl oder Hetaryl bedeuten, wobei die beiden Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
unterwirft und
b) das in Schritt a) gebildete 11-(9-Perylen-3,4-dicarbonsäureimid)terrylen-3,4-dicarbonsäureimid der allgemeinen Formel VIII 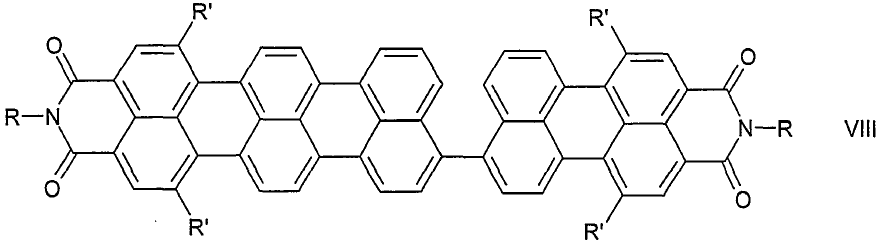 durch Cyclodehydrierung
b1) in einem Hydroxy- und Aminofunktionen aufweisenden und eine im wesentlichen ungelöste Base enthaltenden organischen Reaktionsmedium
oder
b2) in Gegenwart eines basenstabilen, hochsiedenden, organischen Lösungsmittels sowie einer alkali- oder erdalkalimetallhaltigen Base und einer stickstoffhaltigen Hilfsbase
in das Pentarylentetracarbonsäurediimid Ib überführt.

6. Verfahren zur Herstellung von Pentarylentetracarbonsäurediimiden der allgemeinen Formel Ib 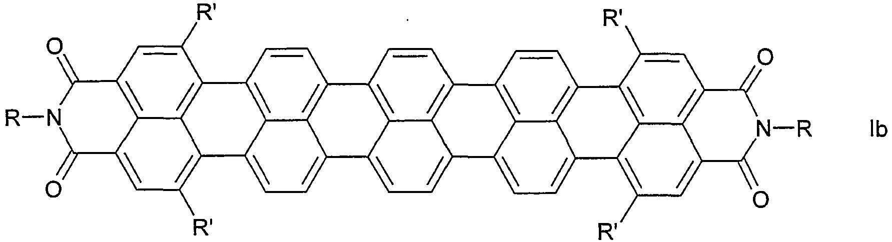 in der die Variablen die in Anspruch 1 genannte Bedeutung haben, R' jedoch nicht Wasserstoff bedeutet, **dadurch gekennzeichnet, daß** man
a) 11-(Dioxaborolan-2-yl)terrylen-3,4-dicarbonsäureimid der allgemeinen Formel VId 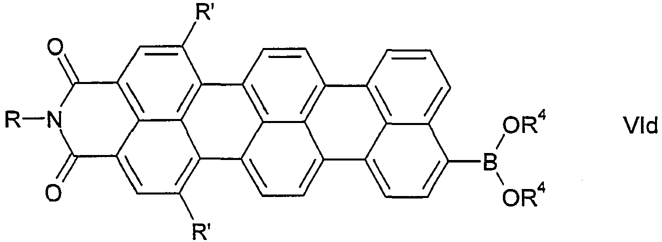 in der die Reste R⁴ gleich oder verschieden sind und unabhängig voneinander Wasserstoff, C₁-C₃₀-Alkyl, C₅-C₈-Cycloalkyl, Aryl oder Hetaryl bedeuten, wobei die beiden Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
in Gegenwart eines organischen Lösungsmittels, gewünschtenfalls im Gemisch mit Wasers, sowie eines Übergangsmetallkatalysators und einer Base einer Suzuki-Kupplungsreaktion mit einem 9-Bromperylen-3,4-dicarbonsäureimid der allgemeinen Formel lila 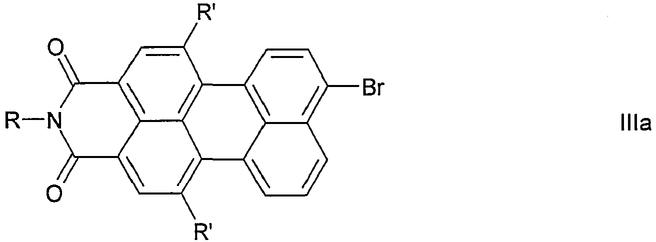 unterwirft und
b) das in Schritt a) gebildete 11-(9-Perylen-3,4-dicarbonsäureimid)terrylen-3,4-dicarbonsäureimid der allgemeinen Formel VIII 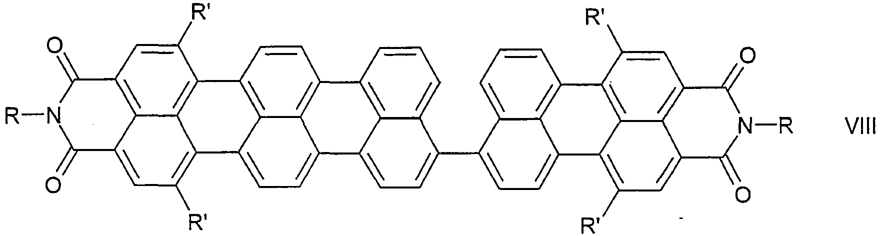 durch Cyclodehydrierung
b1) in einem Hydroxy- und Aminofunktionen aufweisenden und eine im wesentlichen ungelöste Base enthaltenden organischen Reaktionsmedium
oder
b2) in Gegenwart eines basenstabilen, hochsiedenden, organischen Lösungsmittels sowie einer alkali- oder erdalkalimetallhaltigen Base und einer stickstoffhaltigen Hilfsbase
in das Pentarylentetracarbonsäurediimid Ib überführt.

7. Bisterrylenderivate der allgemeinen Formel VII 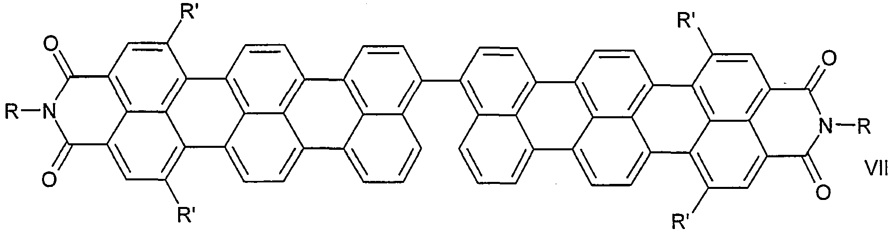 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

8. 11-(9-Perylen-3,4-dicarbonsäureimid)terrylen-3,4-dicarbonsäureimide der allgemeinen Formel VIII 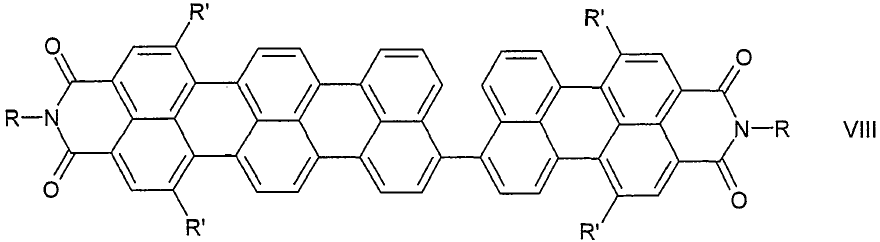 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

9. Terrylen-3,4-dicarbonsäureimide der allgemeinen Formel VI 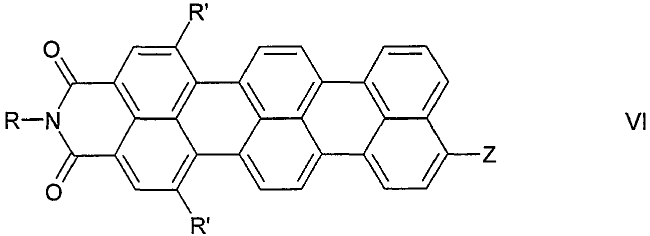 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
Z Brom, Iod, Amino, Nitro oder ein Rest 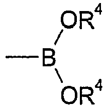
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann;
R⁴ gleiche oder verschiedene Reste:
Wasserstoff, C₁-C₃₀-Alkyl, C₅-C₈-Cycloalkyl, Aryl oder Hetaryl, wobei die beiden Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können.

10. 9-(5-Nitronaphthyl)perylen-3,4-dicarbonsäureimide der allgemeinen Formel V 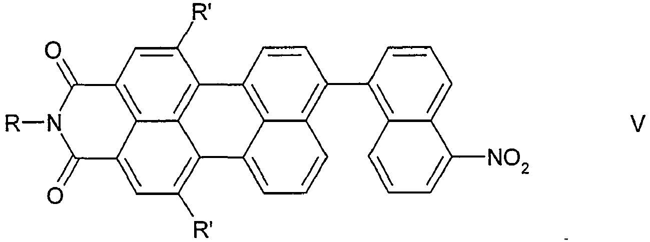 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

11. Verfahren zur Herstellung von Hexarylentetracarbonsäurediimiden der allgemeinen Formel Ia 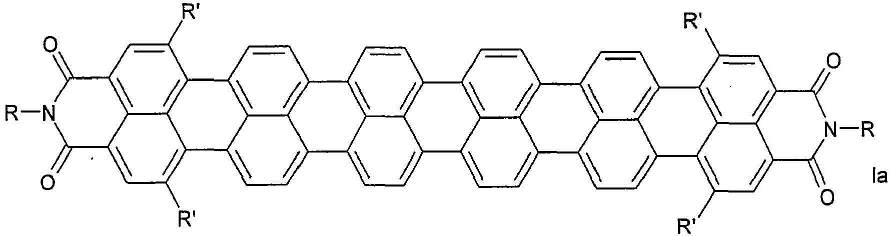 in der die Variablen die in Anspruch 1 genannte Bedeutung haben, **dadurch gekennzeichnet, daß** man
a) ein Diboran der allgemeinen Formel II 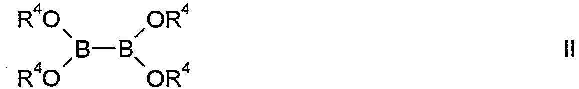 in der die Reste R⁴ gleich oder verschieden sind und unabhängig voneinander Wasserstoff, C₁-C₃₀-Alkyl, C₅-C₈-Cycloalkyl, Aryl oder Hetaryl bedeuten, wobei die an jeweils einem Boratom befindlichen Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
in Gegenwart eines aprotischen organischen Lösungsmittels, eines Übergangsmetallkatalysators und einer Base mit
a1) einem 9-Bromperylen-3,4-dicarbonsäureimid der allgemeinen Formel IIIa 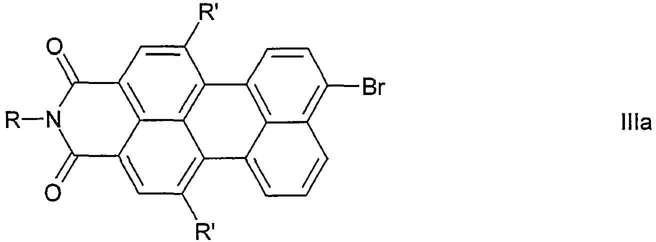 oder
a2) einem Dihalogenperylen der allgemeinen Formel IXa 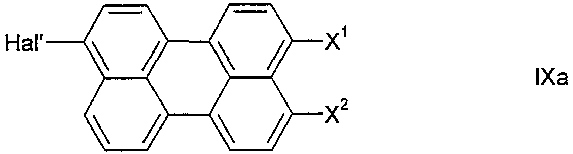 in der Hal' für Chlor oder Brom steht und einer der beiden Reste X¹ oder X² ebenfalls Hal' und der andere Rest Wasserstoff bedeutet, umsetzt,
b1) das in Schritt a1) gebildete 9-(Dioxaborolan-2-yl)perylen-3,4-dicarbonsäureimid der allgemeinen Formel IVa 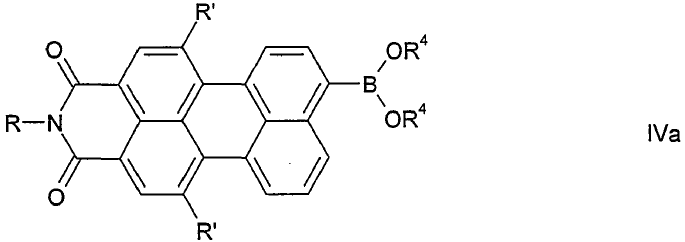 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Dihalogenperylen IXa im Molverhältnis 2 : 1 bis 6 : 1 unterwirft
oder
b2) das in Schritt a2) gebildete Bis(dioxaborolan-2-yl)perylen der allgemeinen Formel IXb 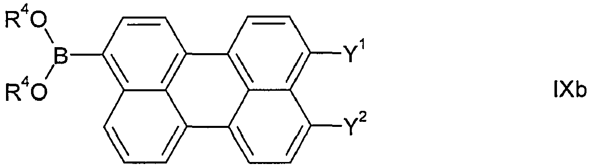 in der einer der beiden Reste Y¹ oder Y² ebenfalls einen Rest 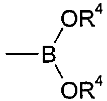 und der andere Rest Wasserstoff bedeutet,
in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbonsäureimid lila im Molverhältnis 1 : 2 bis 1 : 6 unterwirft und
c) das in Schritt b1) bzw. b2) gebildete Perylen-3,9-bis(perylen-3,4-dicarbonsäureimid) der allgemeinen Formel Xa 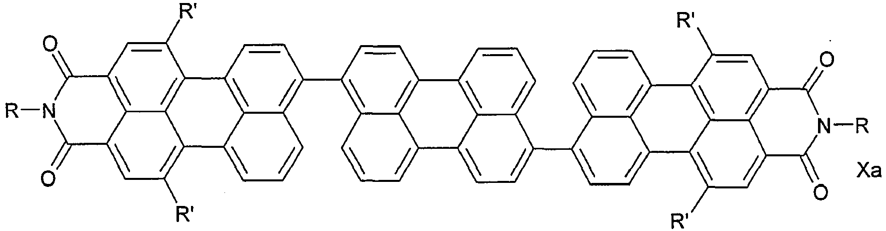 oder Perylen-3,10-bis(perylen-3,4-dicarbonsäureimid) der allgemeinen Formel Xb 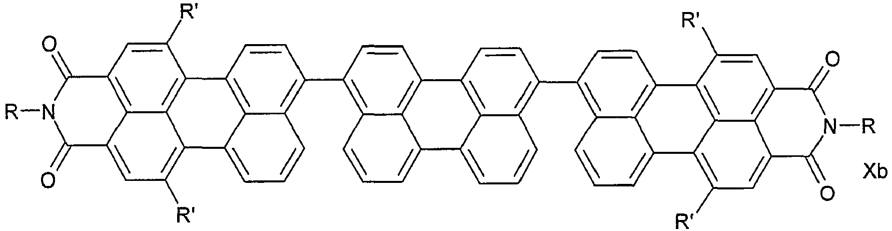
c1) einer einstufigen Cyclodehydrierung in Gegenwart einer starken Lewis-Säure und eines inerten organischen Lösungsmittels direkt zum Hexarylentetracarbonsäurediimid Ia unterzieht
oder
c2a) in einem ersten Schritt bei Raumtemperatur in Gegenwart eines inerten organischen Lösungsmittels mit einer schwachen Lewis-Säure in Kontakt bringt
und
c2b) das dabei gebildete 13-(9-Perylen-3,4-dicarbonsäureimid)quaterrylen-3,4-dicarbonsäureimid der allgemeinen Formel XI 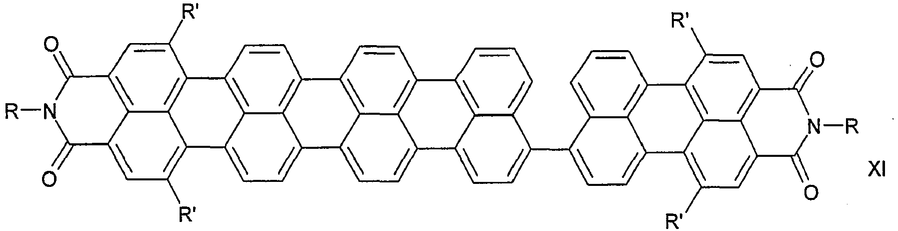 dann nach Zwischenisolierung in einem zweiten Schritt
c2bα)in einem Hydroxy- und Aminofunktionen aufweisenden und eine im wesentlichen ungelöste Base enthaltenden organischen Reaktionsmedium
oder
c2bβ)in Gegenwart eines basenstabilen, hochsiedenden, organischen Lösungsmittels sowie einer alkali- oder erdalkalimetallhaltigen Base und einer stickstoffhaltigen Hilfsbase
weiter zum Hexarylentetracarbonsäurediimid Ia cyclodehydriert.

12. Verfahren zur Herstellung von Pentarylentetracarbonsäurediimiden der allgemeinen Formel Ib 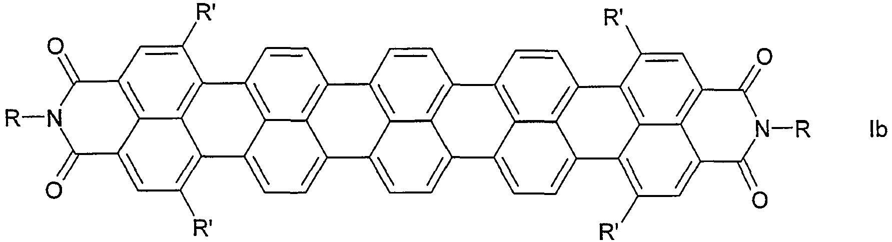 in der die Variablen die in Anspruch 1 genannte Bedeutung haben, **dadurch gekennzeichnet, daß** man
a) ein Diboran der allgemeinen Formel II 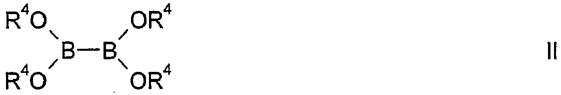 in der die Reste R⁴ gleich oder verschieden sind und unabhängig voneinander Wasserstoff, C₁-C₃₀-Alkyl, C₆-C₈-Cycloalkyl, Aryl oder Hetaryl bedeuten, wobei die an jeweils einem Boratom befindlichen Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können,
in Gegenwart eines aprotischen organischen Lösungsmittels, eines Übergangsmetallkatalysators und einer Base mit
a1) einem 9-Bromperylen-3,4-dicarbonsäureimid der allgemeinen Formel IIIa 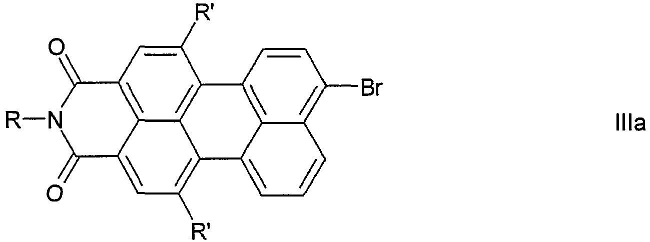 oder
a2) einem Dihalogennaphthalin der allgemeinen Formel IXc 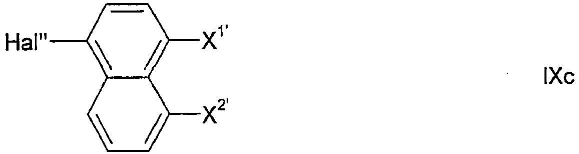 in der Hal" für Chlor, Brom oder Iod steht und einer der beiden Reste X¹' oder X²' ebenfalls Hal" und der andere Rest Wasserstoff bedeutet, umsetzt,
b1) das in Schritt a1) gebildete 9-(Dioxaborolan-2-yl)perylen-3,4-dicarbon-säureimid der allgemeinen Formel IVa 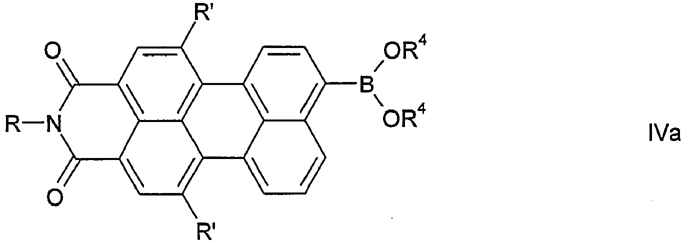 in Gegenwart eines organischen Lösungsmittels, gewünschtenfalls im Gemisch mit Wasser, sowie eines Übergangsmetallkatalysators und einer Base einer Suzuki-Kupplungsreaktion mit einem Dihalogennaphthalin IXc im Molverhältnis 2 : 1 bis 6 : 1 unterwirft
oder
b2) das in Schritt a2) gebildete Bis(dioxaborolan-2-yl)naphthalin der allgemeinen Formel Ixd 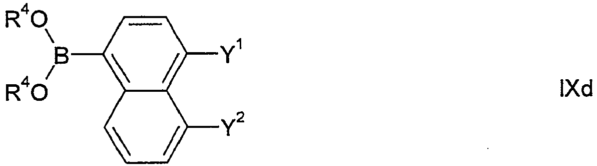 in der einer der beiden Reste Y¹ oder Y² ebenfalls einen Rest 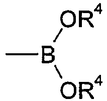 und der andere Rest Wasserstoff bedeutet,
in Gegenwart eines organischen Lösungsmittels, gewünschtenfalls im Gemisch mit Wasser, sowie eines Übergangsmetallkatalysators und einer Base einer Suzuki-Kupplungsreaktion mit einem 9-Bromperylen-3,4-dicarbon-säureimid lila im Molverhältnis 1 : 2 bis 1 : 6 unterwirft und
c) das in Schritt b1) bzw. b2) gebildete Naphthalin-1,5-bis(perylen-3,4-dicar-bonsäureimid) der allgemeinen Formel Xlla 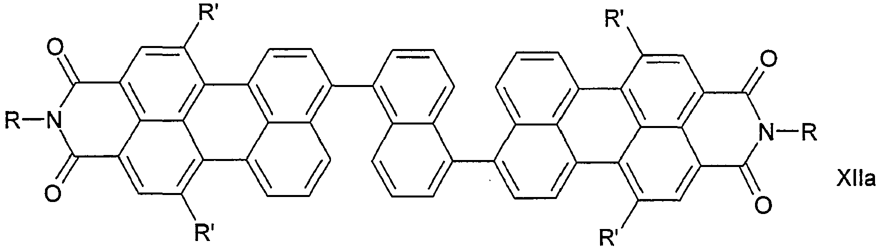 oder Naphthalin-1,4-bis(perylen-3,4-dicarbonsäureimid) der allgemeinen Formel XIIb 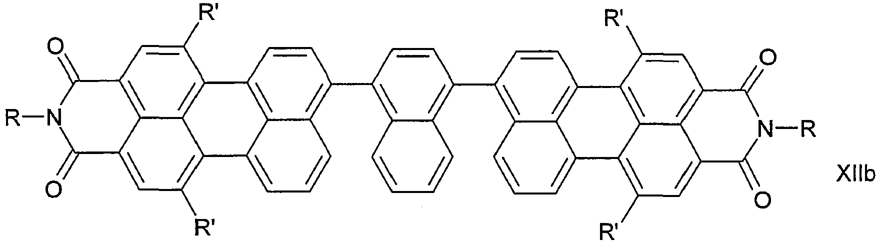
c1) einer einstufigen Cyclodehydrierung in Gegenwart einer Lewis-Säure und eines inerten organischen Lösungsmittels direkt zum Pentarylentetracarbonsäurediimid Ib unterzieht
oder
c2a) in einem ersten Schritt bei Raumtemperatur in Gegenwart eines inerten organischen Lösungsmittels mit einer schwachen Lewis-Säure in Kontakt bringt
und
c2b) das dabei gebildete 11-(9-Perylen-3,4-dicarbonsäureimid)terrylen-3,4-dicarbonsäureimid der allgemeinen Formel VIII 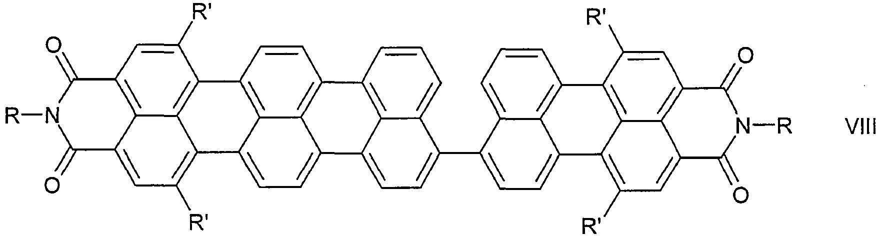 dann nach Zwischenisolierung in einem zweiten Schritt
c2bα)in einem Hydroxy- und Aminofunktionen aufweisenden und eine im wesentlichen ungelöste Base enthaltenden organischen Reaktionsmedium
oder
c2bβ)in Gegenwart eines basenstabilen, hochsiedenden, organischen Lösungsmittels sowie einer alkali- oder erdalkalimetallhaltigen Base und einer stickstoffhaltigen Hilfsbase
weiter zum Pentarylentetracarbonsäurediimid Ib cyclodehydriert.

13. 13-(9-Perylen-3,4-dicarbonsäureimid)quaterrylen-3,4-dicarbonsäureimide der allgemeinen Formel XI 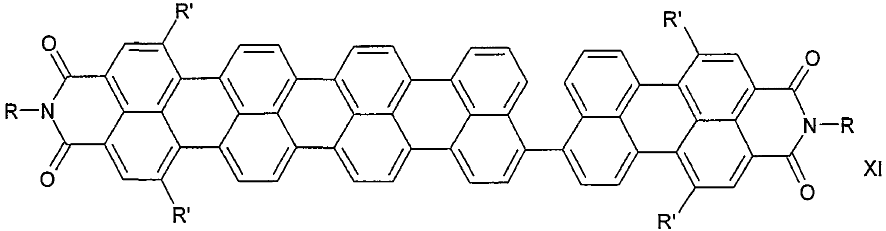 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

14. Perylen-3,9-bis(perylen-3,4-dicarbonsäureimide) der allgemeinen Formel Xa 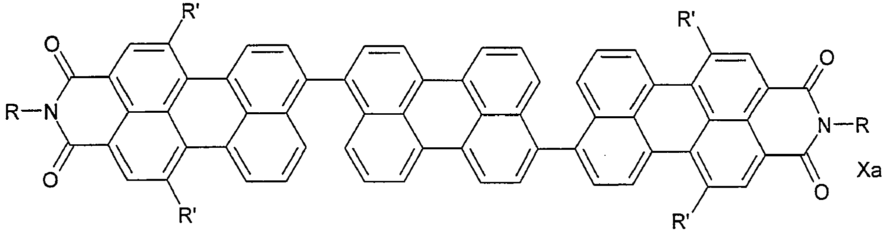 oder Perylen-3,10-bis(perylen-3,4-dicarbonsäureimide) der allgemeinen Formel Xb 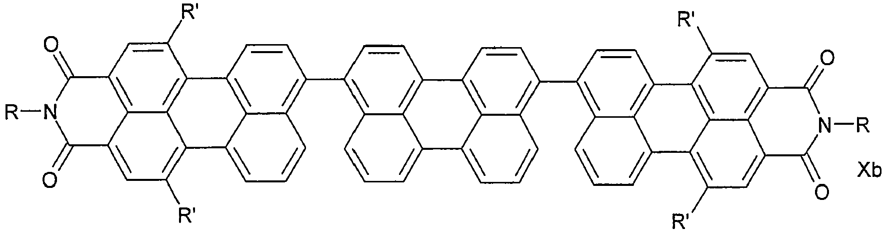 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann und an das jeweils weitere 5- bis 7-gliedrige gesättigte oder ungesättigte Ringe anneliert sein können, die -O-, -S-, -NR¹-, -CO- und/oder -SO₂- als Ringglieder enthalten können und/oder durch einen oder mehrere gleiche oder verschiedene Reste R² substituiert sein können;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

15. Bis(dioxaborolan-2-yl)perylene der allgemeinen Formel IXb 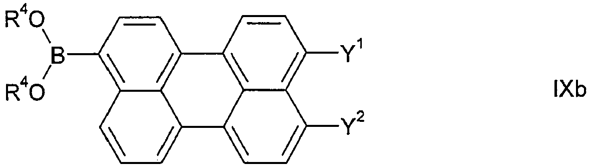 in der die Variablen folgende Bedeutung haben:
R⁴ gleiche oder verschiedene Reste:
Wasserstoff, C₁-C₃₀-Alkyl, C₅-C₈-Cycloalkyl, Aryl oder Hetaryl, wobei die beiden Reste R⁴ auch unter Ausbildung eines die beiden Sauerstoffatome sowie das Boratom enthaltenden Fünfrings, der an den Kohlenstoffatomen durch bis zu 4 C₁-C₃₀-Alkyl-, C₅-C₈-Cycloalkyl-, Aryl- oder Hetarylgruppen substituiert sein kann, miteinander verbunden sein können;
einer der beiden Reste Y¹ oder Y² ebenfalls einen Rest 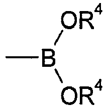 und der andere Rest Wasserstoff.

16. Naphthalin-1,5-bis(perylen-3,4-dicarbonsäureimide) der allgemeinen Formel Xlla 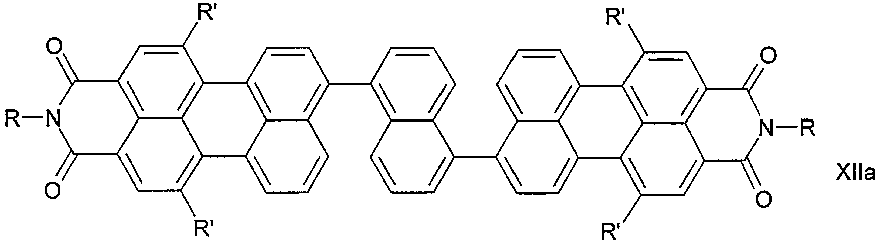 oder Naphthalin-1,4-bis(perylen-3,4-dicarbonsäureimide) der allgemeinen Formel Xllb 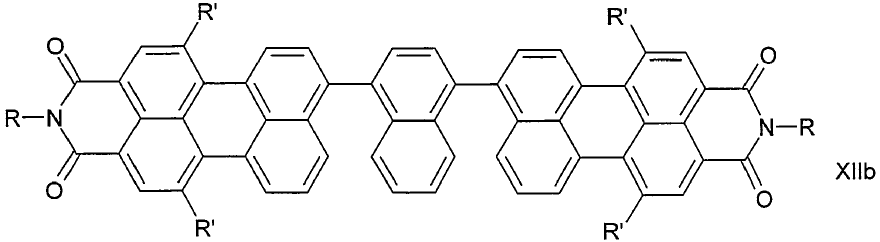 in der die Variablen folgende Bedeutung haben:
R gleiche oder verschiedene Reste:
Wasserstoff;
C₁-C₃₀-Alkyl, dessen Kohlenstoffkette durch eine oder mehrere Gruppierungen -O-, -S-, -NR¹-, -CO- und/oder -SO₂- unterbrochen sein kann und das durch Cyano, C₁-C₆-Alkoxy, Aryl, das durch C₁-C₁₈-Alkyl oder C₁-C₆-Alkoxy substituiert sein kann, und/oder einen über ein Stickstoffatom gebundenen 5- bis 7-gliedrigen heterocyclischen Rest, der weitere Heteroatome enthalten und aromatisch sein kann, ein- oder mehrfach substituiert sein kann;
C₅-C₈-Cycloalkyl, dessen Kohlenstoffgerüst durch eine oder mehrere Gruppierungen -O-, -S- und/oder -NR¹- unterbrochen und/oder das durch C₁-C₁₈-Alkyl ein- oder mehrfach substituiert sein kann;
Aryl oder Hetaryl, das jeweils durch C₁-C₁₈-Alkyl, C₁-C₁₈-Alkoxy, Cyano, Nitro, Halogen, -NR²R³, -CONR²R³, -SO₂R² und/oder Aryl- oder Hetarylazo, das jeweils durch C₁-C₁₈-Alkyl, C₁-C₁₈-Alkoxy oder Cyano substituiert sein kann, ein- oder mehrfach substituiert sein kann;
R' gleiche oder verschiedene Reste:
Wasserstoff;
Aryloxy, Arylthio, Hetaryloxy oder Hetarylthio, das jeweils ein- oder mehrfach durch Alkylreste R, Arylreste R, C₁-C₁₂-Alkoxy, Cyano, Halogen, Hydroxy, -COOR¹, -CONR²R³ und/oder -NHCOR² substituiert sein kann;
R¹ Wasserstoff oder C₁-C₁₈-Alkyl;
R², R³ unabhängig voneinander Wasserstoff;
C₁-C₁₈-Alkyl, das durch C₁-C₆-Alkoxy, Halogen, Hydroxy, Carboxy und/oder Cyano substituiert sein kann; Aryl oder Hetaryl, das jeweils durch die vorstehenden, für Alkyl genannten Reste sowie durch C₁-C₆-Alkyl substituiert sein kann.

17. Verwendung von Rylentetracarbonsäurediimiden der Formel I gemäß Anspruch 1 oder 2 zur Einfärbung von hochmolekularen organischen und anorganischen Materialien.

18. Verwendung nach Anspruch 17, **dadurch gekennzeichnet, daß** die hochmolekularen Materialien Lacke, Druckfarben oder Kunststoffe sind.

19. Verwendung von Rylentetracarbonsäurediimiden der Formel I gemäß Anspruch 1 oder 2 zur Herstellung im nahinfraroten Bereich des elektromagnetischen Spektrums absorbierender wäßriger Polymerisatdispersionen.

20. Verwendung von Rylentetracarbonsäurediimiden der Formel I gemäß Anspruch 1 oder 2 zur Erzeugung für das menschliche Auge nicht sichtbarer, Infrarotlicht absorbierender Markierungen und Beschriftungen.

21. Verwendung von Rylentetracarbonsäurediimiden der Formel I gemäß Anspruch 1 oder 2 als Infrarotabsorber für das Wärmemanagement.

22. Verwendung von Rylentetracarbonsäurediimiden der Formel I gemäß Anspruch 1 oder 2 als IR-Laserstrahlenabsorbierende Materialien beim Schweißbehandeln von Kunststoffteilen.

23. Verwendung von Rylentetracarbonsäurediimiden der Formel I gemäß Anspruch 1 oder 2 als Aktivkomponenten in der Photovoltaik.

## Claims

1. A rylenetetracarboximide of the general formula I 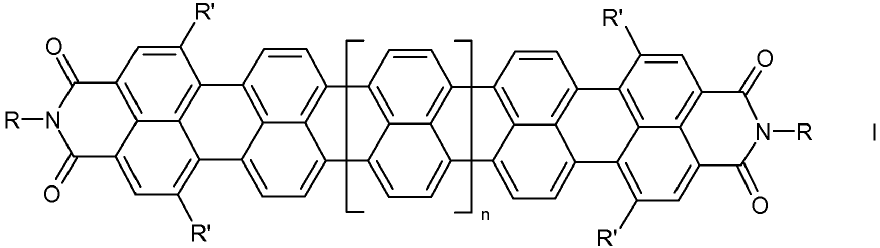 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or - NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹, - CONR²R³ and/or -NHCOR²;
R¹ is hydrogen or C₁-C₁₈-alkyl;
R², R³ are each independently hydrogen;
C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;
n is 1 or 2.

2. A rylenetetracarboximide of the general formula I according to claim 1, in which the variables are defined as follows:
R are identical radicals:
hydrogen;
C₁-C₃₀-alkyl, whose carbon chain may be interrupted by one or more -O- and/or -CO-moieties and which may be mono- or polysubstituted by cyano, C₁-C₆-alkoxy, aryl, which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl which may be mono- or polysubstituted by C₁-C₆-alkyl;
phenyl, naphthyl, pyridyl or pyrimidyl, each of which may be mono- or polysubstituted by C₁-C₁₈-alkyl, C₁-C₆-alkoxy, cyano, nitro, halogen, -CONR²R³, -SO₂R² and/or phenyl- or naphthylazo, each of which may be substituted by C₁-C₁₀-alkyl, C₁-C₆-alkoxy or cyano;
R' are identical radicals:
hydrogen; bromine;
phenoxy, phenylthio, pyridyloxy, pyrimidyloxy, pyridylthio or pyrimidylthio, each of which may be mono- or polysubstituted by C₁-C₁₂-alkyl or C₁-C₁₂-alkoxy;
R¹ is hydrogen or C₁-C₆-alkyl;
R², R³ are each independently hydrogen; C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;
n is 1 or 2.

3. A process for preparing hexarylenetetracarboximides of the general formula Ia 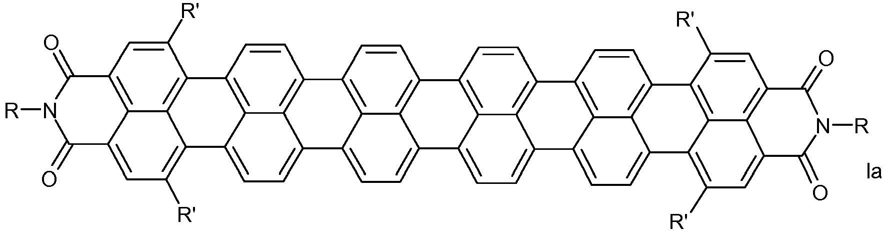 in which the variables are each as defined in claim 1 except that R' is not hydrogen, which comprises
a) reacting a diborane of the general formula II 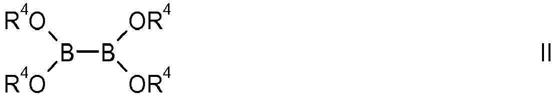 in which the R⁴ radicals are the same or different and are each independently hydrogen, C₁-C₃₀-alkyl, C₅-C₈-cycloalkyl, aryl or hetaryl, where the R⁴ radicals disposed in each case on one boron atom may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups,
in the presence of an aprotic organic solvent, of a transition metal catalyst and of a base with
a1) a 9-bromoperylene-3,4-dicarboximide of the general formula IIIa 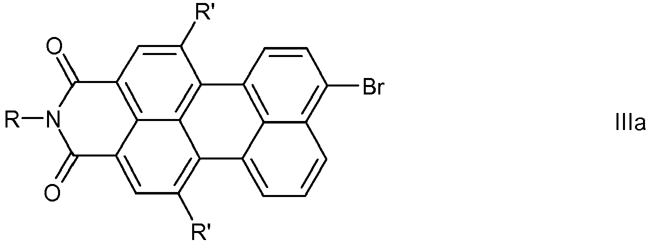 or
a2) a naphthalene derivative of the general formula IIIb 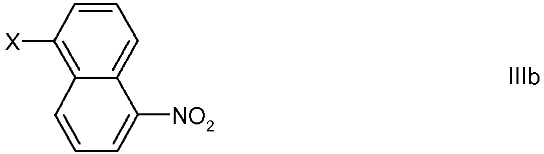 in which X is halogen, C₁-C₁₂-alkylsulfonyl, whose alkyl radical may be mono- or polysubstituted by halogen, or C₆-C₁₈-arylsulfonyl,
b1) subjecting the 9-(dioxaborolan-2-yl)perylene-3,4-dicarboximide of the general formula IVa 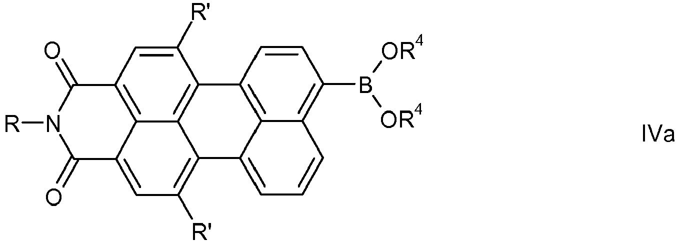 formed in step a1), in the presence of an organic solvent, if desired in a mixture with water, and of a transition metal catalyst and of a base, to a Suzuki coupling reaction with a naphthalene derivative IIIb
or
b2) subjecting the 1-(dioxaborolan-2-yl)-5-nitronaphthalene of the general formula IVb 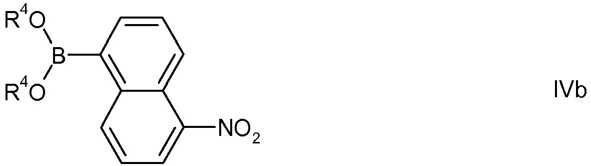 formed in step a2), in the presence of an organic solvent, if desired in a mixture with water, and of a transition metal catalyst and of a base, to a Suzuki coupling reaction with a 9-bromoperylene-3,4-dicarboximide IIIa,
c) subjecting the 9-(5-nitronaphthyl)perylene-3,4-dicarboximide of the general formula V 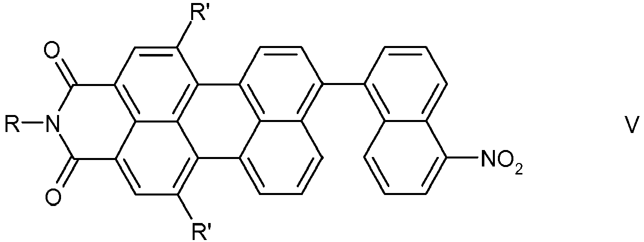 formed in step b1) or b2) to a cyclodehydrogenation in the presence of a base-stable, high-boiling organic solvent and of an alkali metal- or alkaline earth metal-containing base and of a nitrogen-containing auxiliary base,
d) reducing the 11-nitroterrylene-3,4-dicarboximide of the general formula VIa 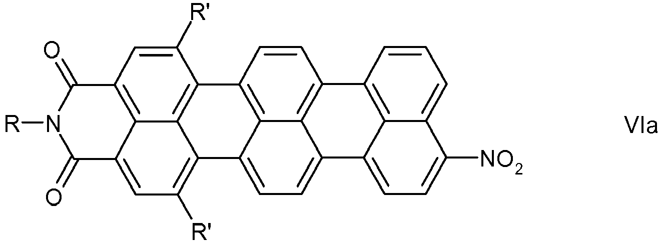 formed in step c) with nascent hydrogen,
e) diazotizing the 11-aminoterrylene-3,4-dicarboximide of the general formula VIb 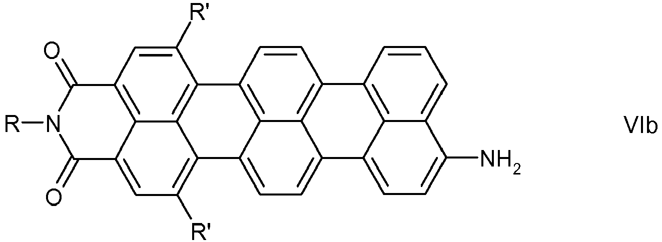 formed in step d) and reacting the diazonium salt formed with a metal bromide or iodide,
f) coupling the 11-haloterrylene-3,4-dicarboximide of the general formula VIc 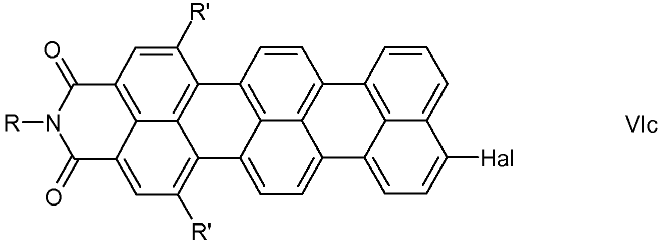 in which Hal is bromine or iodine, formed in step e),
f1) in the presence of an organic transition metal complex as a catalyst, of free ligand molecules and of an aprotic solvent to give a bisterrylene derivative of the general formula VII 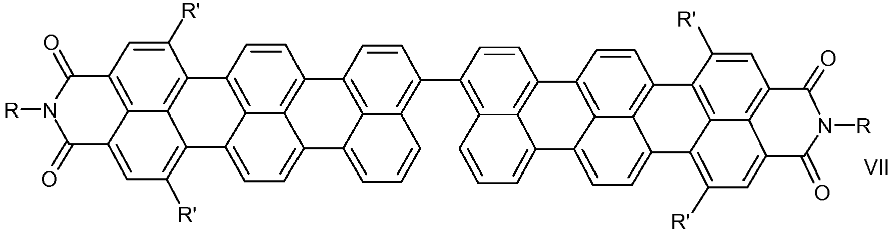 or
f2) in the presence of from 30 to 70 mol%, based on the 11-haloterrylene-3,4-dicarboximide VIc, of a diborane II, of a transition metal catalyst, of a base and of an aprotic organic solvent, without intermediate isolation of the 11-(dioxaborolan-2-yl)terrylene-3,4-dicarboximide of the general formula VId 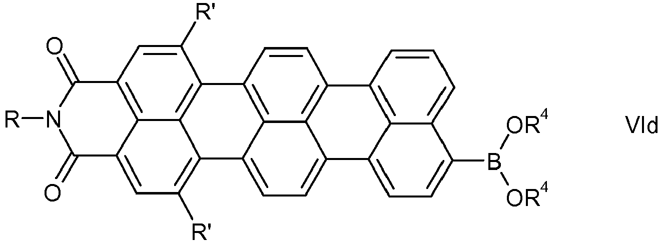 formed in situ, converting it by a Suzuki coupling reaction to the bisterrylene derivative VII and
g) converting the bisterrylene derivative VII by cyclodehydrogenation,
g1) in an organic reaction medium having hydroxyl and amino functions and comprising a substantially undissolved base
or
g2) in the presence of a base-stable, high-boiling organic solvent and of an alkali metal- or alkaline earth metal-containing base and of a nitrogen-containing auxiliary base,
to the hexarylenetetracarboximide Ia.

4. A process for preparing hexarylenetetracarboximides of the general formula Ia 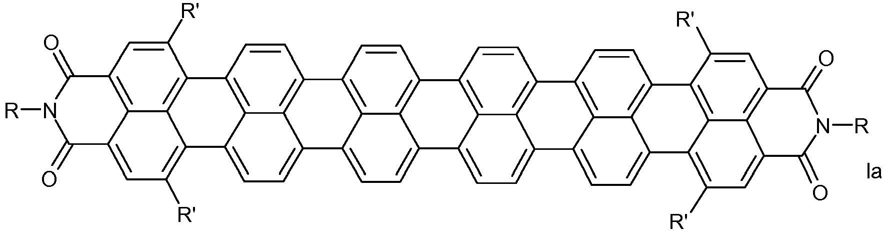 in which the variables are each as defined in claim 1 except that R' is not hydrogen, which comprises
a) reacting a diborane of the general formula II 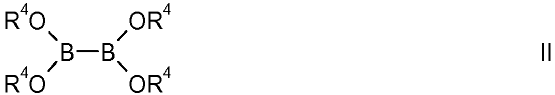 in which the R⁴ radicals are the same or different and are each independently hydrogen, C₁-C₃₀-alkyl, C₅-C₈-cycloalkyl, aryl or hetaryl, where the R⁴ radicals disposed in each case on one boron atom may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups,
in the presence of an aprotic organic solvent, of a transition metal catalyst and of a base with an 11-haloterrylene-3,4-dicarboximide of the general formula VIc 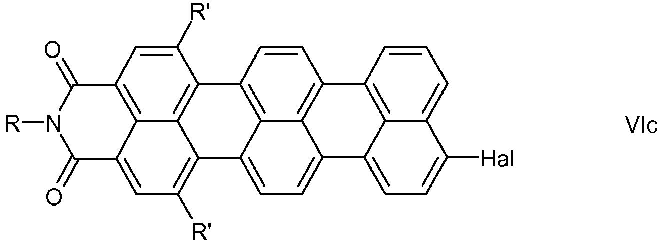 in which Hal is bromine or iodine,
b) subjecting the 11-(dioxaborolan-2-yl)terrylene-3,4-dicarboximide of the general formula VId 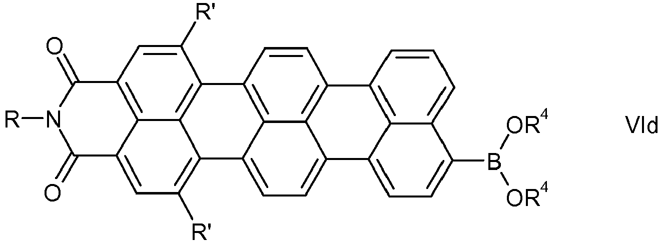 formed in step a), in the presence of an organic solvent, if desired in a mixture with water, and of a transition metal catalyst and of a base, to a Suzuki coupling reaction with an 11-haloterrylene-3,4-dicarboximide VIc and
c) converting the bisterrylene derivative of the general formula VII 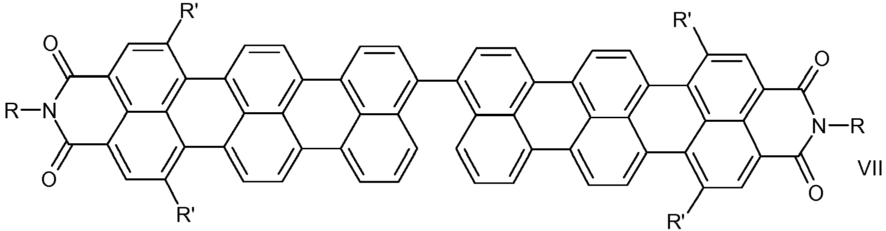 formed in step b) by cyclodehydrogenation,
c1) in an organic reaction medium having hydroxyl and amino functions and comprising a substantially undissolved base
or
c2) in the presence of a base-stable, high-boiling organic solvent and of an alkali metal- or alkaline earth metal-containing base and of a nitrogen-containing auxiliary base,
to the hexarylenetetracarboximide Ia.

5. A process for preparing pentarylenetetracarboximides of the general formula Ib 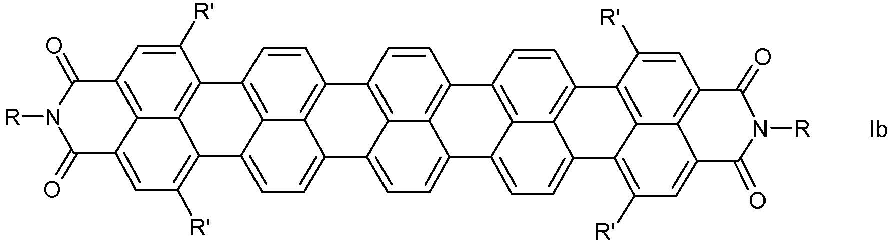 in which the variables are as defined in claim 1 except that R' is not hydrogen, which comprises
a) subjecting an 11-haloterrylene-3,4-dicarboximide of the general formula VIc 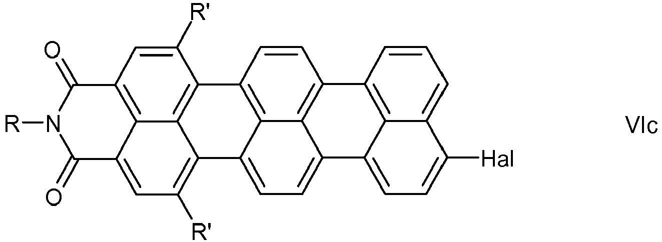 in which Hal is bromine or iodine, in the presence of an organic solvent, if desired in a mixture with water, and also of a transition metal catalyst and of a base, to a Suzuki coupling reaction with a 9-(dioxaborolan-2-yl)perylene-3,4-dicarboximide of the general formula IVa 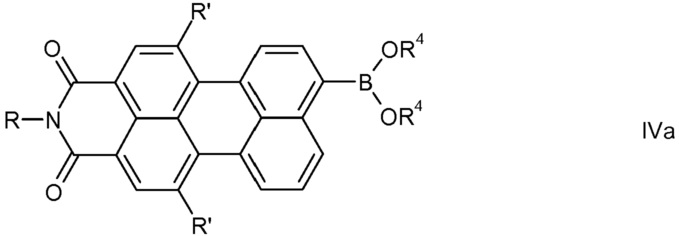 in which the R⁴ radicals are the same or different and are each independently hydrogen, C₁-C₃₀-alkyl, C₅-C₈-cycloalkyl, aryl or hetaryl, where the two R⁴ radicals may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups,
and
b) converting the 11-(9-perylene-3,4-dicarboximide)terrylene-3,4-dicarboximide of the general formula VIII 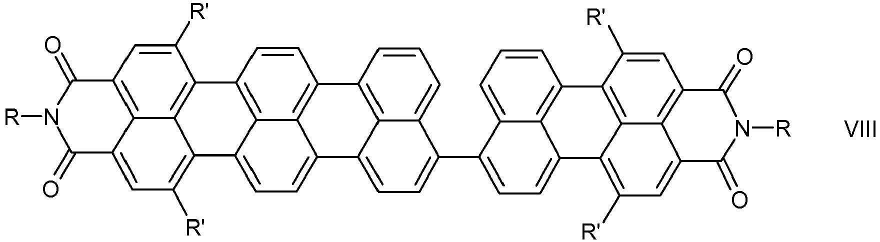 formed in step a) by cyclodehydrogenation,
b1) in an organic reaction medium having hydroxyl and amino functions and comprising a substantially undissolved base
or
b2) in the presence of a base-stable, high-boiling organic solvent and of an alkali metal- or alkaline earth metal-containing base and of a nitrogen-containing auxiliary base, to the pentarylenetetracarboximide Ib.

6. A process for preparing pentarylenetetracarboximides of the general formula Ib 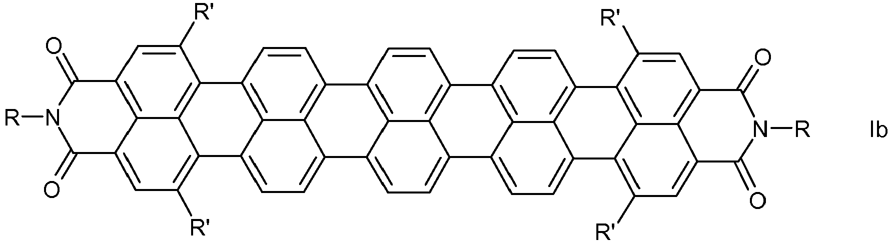 in which the variables are as defined in claim 1 except that R' is not hydrogen, which comprises
a) subjecting 11-(dioxaborolan-2-yl)terrylene-3,4-dicarboximide of the general formula VId 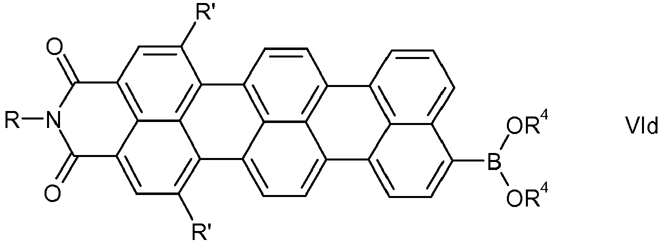 in which the R⁴ radicals are the same or different and are each independently hydrogen, C₁-C₃₀-alkyl, C₅-C₈-cycloalkyl, aryl or hetaryl, where the two R⁴ radicals may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups, in the presence of an organic solvent, if desired in a mixture with water, and also of a transition metal catalyst and of a base, to a Suzuki coupling reaction with a 9-bromoperylene-3,4-dicarboximide of the general formula IIIa 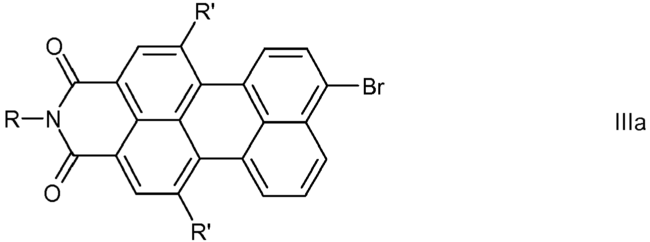 and
b) converting the 11-(9-perylene-3,4-dicarboximide)terrylene-3,4-dicarboximide of the general formula VIII 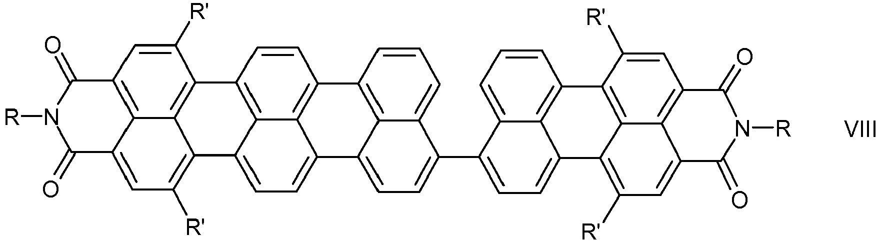 formed in step a) by cyclodehydrogenation,
b1) in an organic reaction medium having hydroxyl and amino functions and comprising a substantially undissolved base
or
b2) in the presence of a base-stable, high-boiling organic solvent and of an alkali metal- or alkaline earth metal-containing base and of a nitrogen-containing auxiliary base,
to the pentarylenetetracarboximide Ib.

7. A bisterrylene derivative of the general formula VII 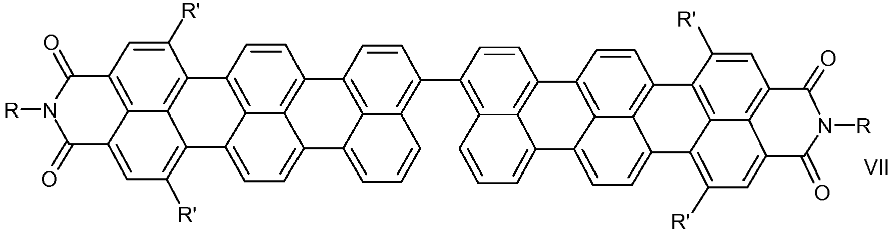 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen; C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;

8. An 11-(9-perylene-3,4-dicarboximide)terrylene-3,4-dicarboximide of the general formula VIII 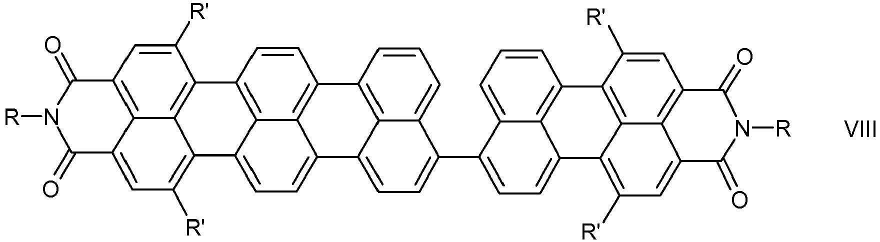 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen;
C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;

9. A terrylene-3,4-dicarboximide of the general formula VI 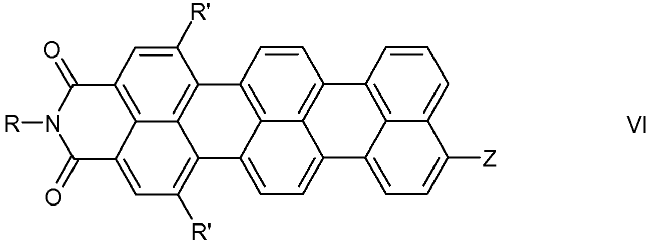 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
Z is bromine, iodine, amino, nitro or a radical 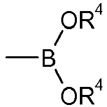
R¹ is hydrogen or C₁-C₁₈-alkyl;
R², R³ are each independently hydrogen;
C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;
R⁴ are identical or different radicals:
hydrogen, C₁-C₃₀-alkyl, C₅-C₈-cycloalkyl, aryl or hetaryl, where the two R⁴ radicals may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups.

10. A 9-(5-nitronaphthyl)perylene-3,4-dicarboximide of the general formula V 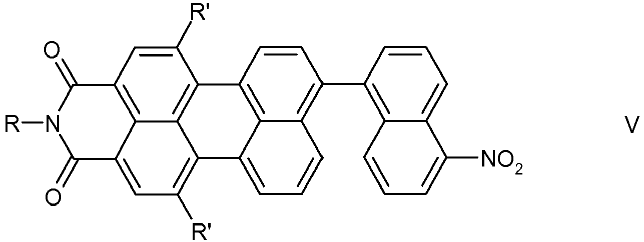 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen;
C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;

11. A process for preparing hexarylenetetracarboximides of the general formula Ia 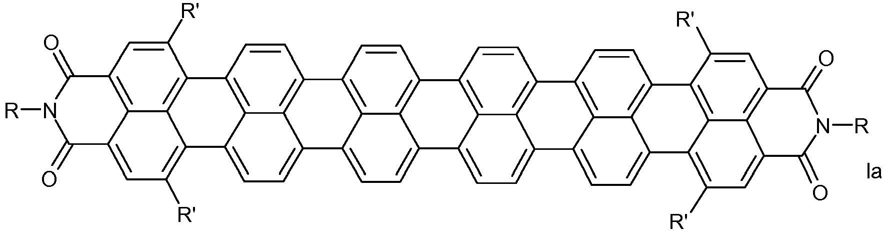 in which the variables are each as defined in claim 1, which comprises
a) reacting a diborane of the general formula II 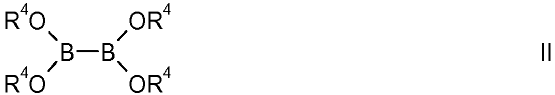 in which the R⁴ radicals are the same or different and are each independently hydrogen, C₁-C₃₀-alkyl, C₅-C₈-cycloalkyl, aryl or hetaryl, where the R⁴ radicals disposed in each case on one boron atom may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups,
in the presence of an aprotic organic solvent, of a transition metal catalyst and of a base with
a1) a 9-bromoperylene-3,4-dicarboximide of the general formula IIIa 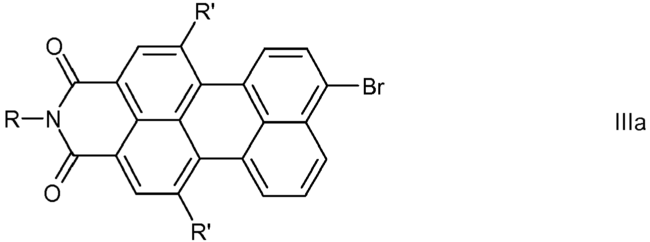 or
a2) a dihaloperylene of the general formula IXa 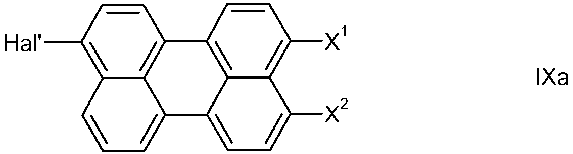 in which Hal' is chlorine or bromine and one of the two X¹ and X² radicals is likewise Hal' and the other radical is hydrogen,
b1) subjecting the 9-(dioxaborolan-2-yl)perylene-3,4-dicarboximide of the general formula IVa 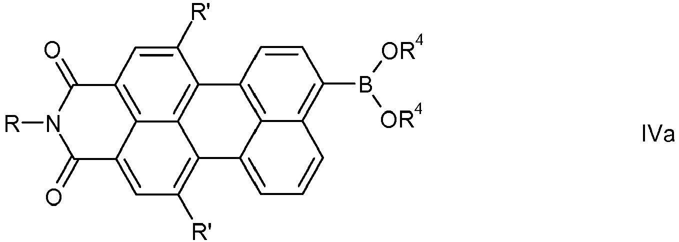 formed in step a1), in the presence of an organic solvent, if desired in a mixture with water, and of a transition metal catalyst and of a base, to a Suzuki coupling reaction with a dihaloperylene IXa in a molar ratio of from 2:1 to 6:1
or
b2) subjecting the bis(dioxaborolan-2-yl)perylene of the general formula IXb 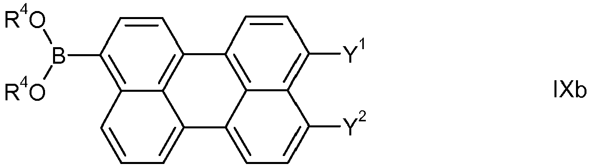 formed in step a2), in which one of the two Y¹ and Y² radicals is likewise a radical 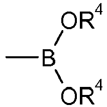 and the other radical is hydrogen,
in the presence of an organic solvent, if desired in a mixture with water, and also of a transition metal catalyst and of a base, to a Suzuki coupling reaction with a 9-bromoperylene-3,4-dicarboximide IIIa in a molar ratio of from 1:2 to 1:6 and
c) subjecting the perylene-3,9-bis(perylene-3,4-dicarboximide) of the general formula Xa 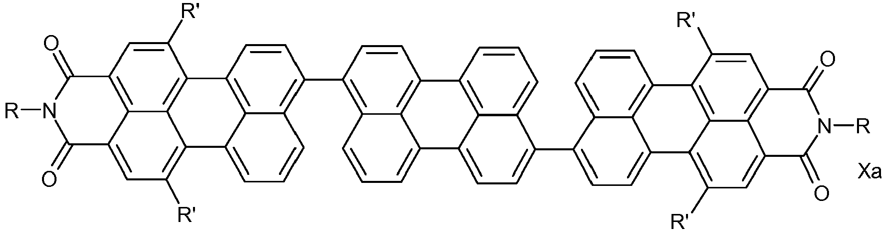 or perylene-3,10-bis(perylene-3,4-dicarboximide) of the general formula Xb 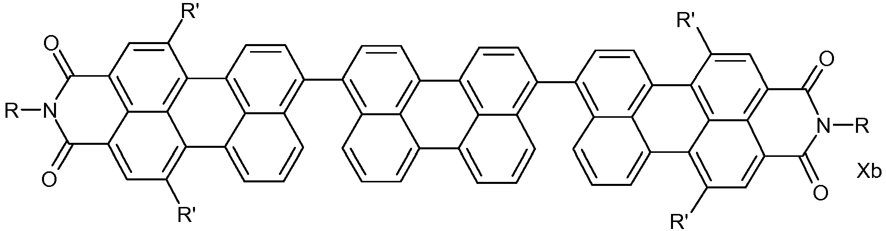 formed in step b1) or b2)
c1) to a single-stage cyclodehydrogenation in the presence of a strong Lewis acid and of an inert organic solvent directly to give the hexarylenetetracarboximide Ia
or
c2a) contacting it with a weak Lewis acid in a first step at room temperature in the presence of an inert organic solvent
and
c2b) then, after intermediate isolation, in a second step, further cyclodehydrogenating the thus formed 13-(9-perylene-3,4-dicarboximide)quaterrylene-3,4-dicarboximide of the general formula XI 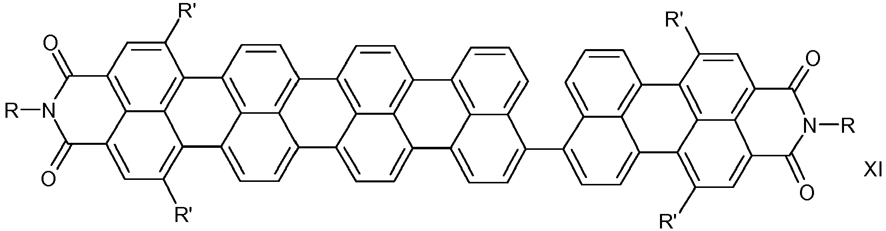
c2bα) in an organic reaction medium having hydroxyl and amino functions and comprising a substantially undissolved base
or
c2bβ) in the presence of a base-stable, high-boiling organic solvent and of an alkali metal- or alkaline earth metal-containing base and of a nitrogen-containing auxiliary base
to the hexarylenetetracarboximide Ia.

12. A process for preparing pentarylenetetracarboximides of the general formula Ib 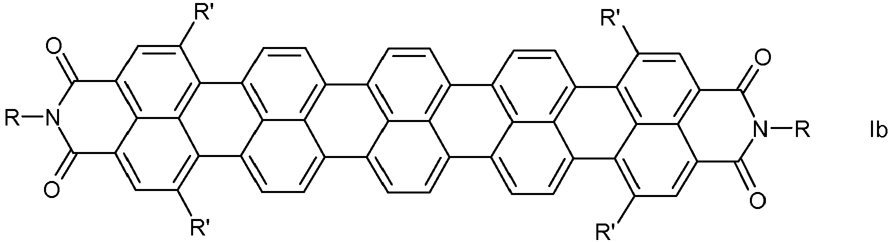 in which the variables are as defined in claim 1, which comprises
a) reacting a diborane of the general formula II 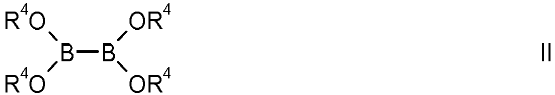 in which the R⁴ radicals are the same or different and are each independently hydrogen, C₁-C₃₀-alkyl, C₅-C₈-cycloalkyl, aryl or hetaryl, where the R⁴ radicals disposed in each case on one boron atom may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups,
in the presence of an aprotic organic solvent, of a transition metal catalyst and of a base with
a1) a 9-bromoperylene-3,4-dicarboximide of the general formula IIIa 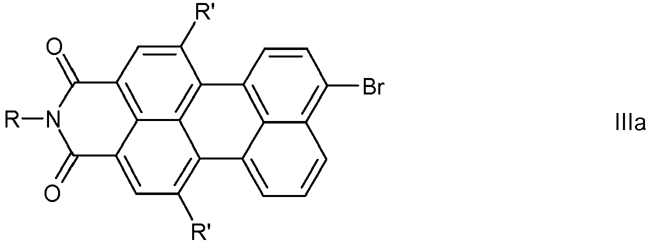 or
a2) a dihalonaphthalene of the general formula IXc 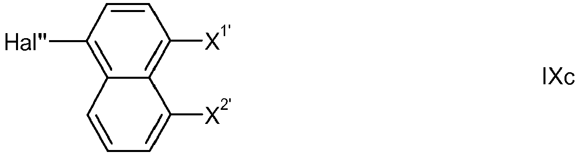 in which Hal" is chlorine, bromine or iodine and one of the two X¹' and X²' radicals is likewise Hal" and the other radical is hydrogen,
b1) subjecting the 9-(dioxaborolan-2-yl)perylene-3,4-dicarboximide of the general formula IVa 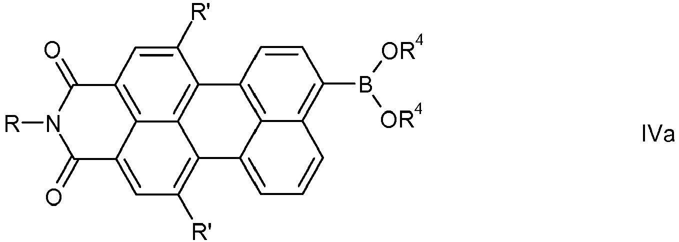 formed in step a1), in the presence of an organic solvent, if desired in a mixture with water, and of a transition metal catalyst and of a base, to a Suzuki coupling reaction with a dihalonaphthalene IXc in a molar ratio of from 2:1 to 6:1
or
b2) subjecting the bis(dioxaborolan-2-yl)naphthalene of the general formula IXd 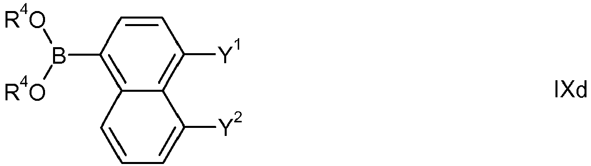 formed in step a2), in which one of the two Y¹ and Y² radicals is likewise a radical 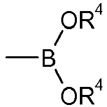 and the other radical is hydrogen,
in the presence of an organic solvent, if desired in a mixture with water, and also of a transition metal catalyst and of a base, to a Suzuki coupling reaction with a 9-bromoperylene-3,4-dicarboximide IIIa in a molar ratio of from 1:2 to 1:6 and
c) subjecting the naphthalene-1,5-bis(perylene-3,4-dicarboximide) of the general formula XIIa 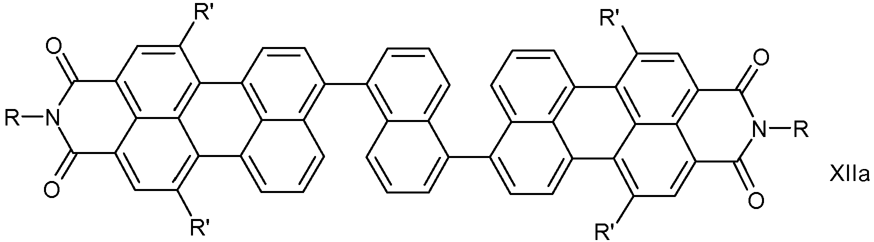 or naphthalene-1,4-bis(perylene-3,4-dicarboximide) of the general formula XIIb 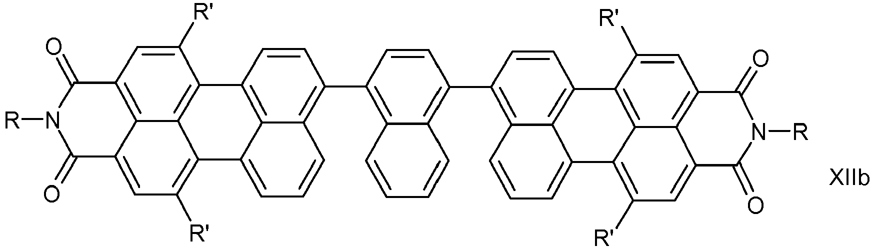 formed in step b1) or b2)
c1) to a single-stage cyclodehydrogenation in the presence of a Lewis acid and of an inert organic solvent directly to give the pentarylenetetracarboximide Ib
or
c2a) contacting it with a weak Lewis acid in a first step at room temperature in the presence of an inert organic solvent
and
c2b) then, after intermediate isolation, in a second step, further cyclodehydrogenating the thus formed 11-(9-perylene-3,4-dicarboximide)terrylene-3,4-dicarboximide of the general formula VIII 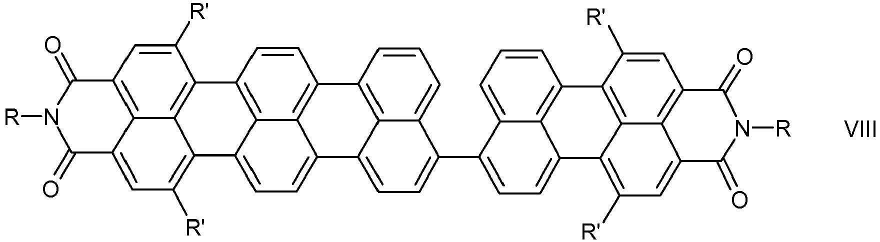
c2bα) in an organic reaction medium having hydroxyl and amino functions and comprising a substantially undissolved base
or
c2bβ) in the presence of a base-stable, high-boiling organic solvent and of an alkali metal- or alkaline earth metal-containing base and of a nitrogen-containing auxiliary base
to the pentarylenetetracarboximide Ib.

13. A 13-(9-perylene-3,4-dicarboximide)quaterrylene-3,4-dicarboximide of the general formula XI 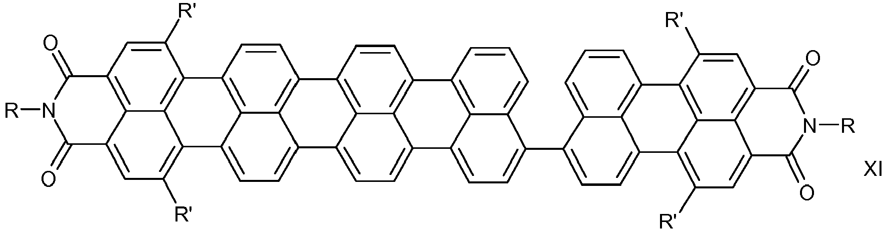 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen;
C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl.

14. A perylene-3,9-bis(perylene-3,4-dicarboximide) of the general formula Xa 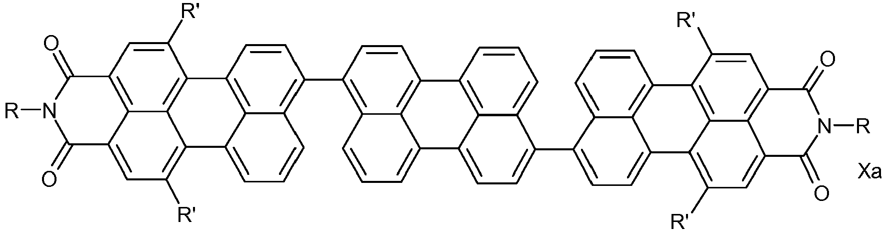 or perylene-3,10-bis(perylene-3,4-dicarboximide) of the general formula Xb 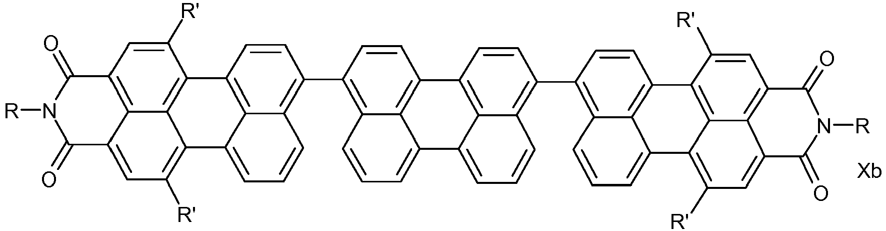 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano, and to which further 5- to 7-membered saturated or unsaturated rings may be fused, which may comprise -O-, -S-, -NR¹-, -CO- and/or -SO₂- as ring members and/or be substituted by one or more identical or different R² radicals;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen; C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;

15. A bis(dioxaborolan-2-yl)perylene of the general formula IXb 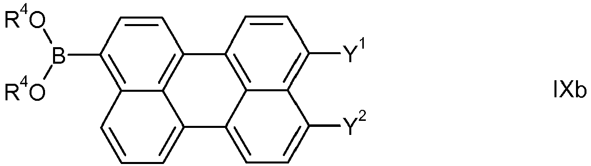 in which the variables are defined as follows:
R⁴ are identical or different radicals:
hydrogen, C₁-C₃₀-alkyl, C₅-C₈-cycloalkyl, aryl or hetaryl, where the two R⁴ radicals may also be joined together to form a five-membered ring which comprises the two oxygen atoms and the boron atom and may be substituted on the carbon atoms by up to 4 C₁-C₃₀-alkyl, C₅-C₈-cycloalkyl, aryl or hetaryl groups;
one of the two Y¹ and Y² radicals is likewise a radical 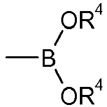
and the other radical is hydrogen.

16. A naphthalene-1,5-bis(perylene-3,4-dicarboximide) of the general formula XIIa 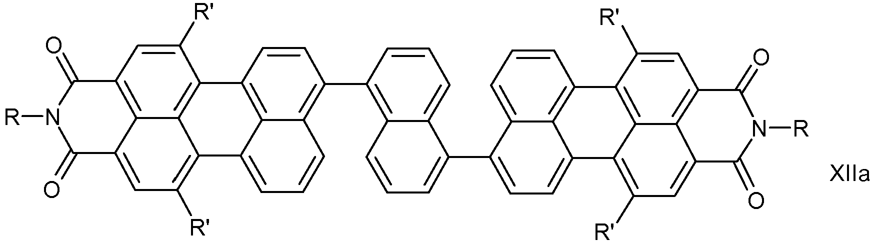 or naphthalene-1,4-bis(perylene-3,4-dicarboximide) of the general formula XIIb 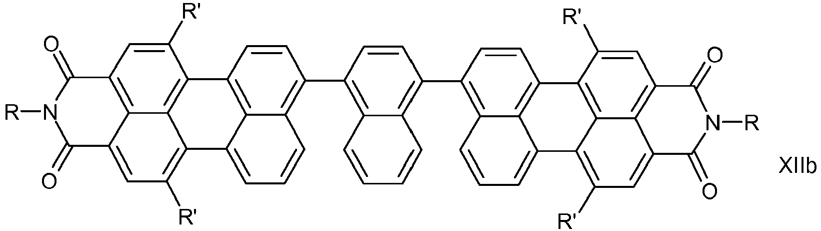 in which the variables are defined as follows:
R are identical or different radicals:
hydrogen;
C₁-C₃₀-alkyl whose carbon chain may be interrupted by one or more -O-, -S-, -NR¹-, -CO- and/or -SO₂- moieties and which may be mono- or polysubstituted by cyano, C₁-C₆-alkoxy, aryl which may be substituted by C₁-C₁₈-alkyl or C₁-C₆-alkoxy, and/or a 5- to 7-membered heterocyclic radical which is bonded via a nitrogen atom and may comprise further heteroatoms and be aromatic;
C₅-C₈-cycloalkyl whose carbon skeleton may be interrupted by one or more -O-, -S- and/or-NR¹- moieties and/or which may be mono- or polysubstituted by C₁-C₁₈-alkyl;
aryl or hetaryl, each of which may be mono- or polysubstituted by C₁-C₁₈-alkyl, C₁-C₁₈-alkoxy, cyano, nitro, halogen, -NR²R³, -CONR²R³, -SO₂R² and/or aryl- or hetarylazo, each of which may be substituted by C₁-C₁₈-alkyl, C₁-C₁₈-alkoxy or cyano;
R' are identical or different radicals:
hydrogen;
aryloxy, arylthio, hetaryloxy or hetarylthio, each of which may be mono- or polysubstituted by alkyl radicals R, aryl radicals R, C₁-C₁₂-alkoxy, cyano, halogen, hydroxyl, -COOR¹,-CONR²R³ and/or -NHCOR²;
R¹ is hydrogen or C₁-C₁₈-alkyl;
R², R³ are each independently hydrogen; C₁-C₁₈-alkyl, which may be substituted by C₁-C₆-alkoxy, halogen, hydroxyl, carboxyl and/or cyano; aryl or hetaryl, each of which may be substituted by the aforementioned radicals specified for alkyl and by C₁-C₆-alkyl;

17. The use of rylenetetracarboximides of the formula I according to claim 1 or 2 for coloring high molecular weight organic and inorganic materials.

18. The use according to claim 17, wherein the high molecular weight materials are coatings, printing inks or plastics.

19. The use of rylenetetracarboximides of the formula I according to claim 1 or 2 for producing aqueous polymer dispersions which absorb in the near infrared region of the electromagnetic spectrum.

20. The use of rylenetetracarboximides of the formula I according to claim 1 or 2 for producing markings and inscriptions which absorb infrared light and are invisible to the human eye.

21. The use of rylenetetracarboximides of the formula I according to claim 1 or 2 as infrared absorbers for heat management.

22. The use of rylenetetracarboximides of the formula I according to claim 1 or 2 as IR laser beam-absorbing materials in the fusion treatment of plastics parts.

23. The use of rylenetetracarboximides of the formula I according to claim 1 or 2 as active components in photovoltaics.

## Revendications

1. Diimides d'acide rylènetétracarboxylique de formule générale 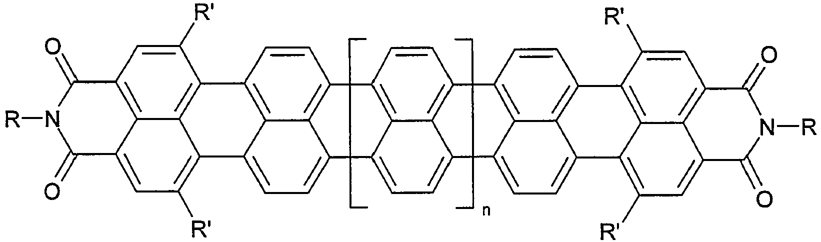 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle ;
n 1 ou 2.

2. Diimides d'acide rylènetétracarboxylique de formule générale I selon la revendication 1, dans laquelle les variables ont la signification suivante :
R des radicaux identiques :
hydrogène ;
C₁-C₃₀-alkyle, dont la chaîne carbonée peut être interrompue par un ou plusieurs groupements -O-et/ou -CO- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ; C₅-C₈-cycloalkyle, qui peut être monosubstitué ou polysubstitué par C₁-C₆-alkyle ; phényle, naphtyle, pyridyle ou pyrimidyle, qui peut à chaque fois être monosubstitué ou polysubstitué par C₁-C₁₈-alkyle ; C₁-C₆-alcoxy ; cyano ; nitro ; halogéno ; -CONR²R³ ; -SO₂R² ; et/ou phénylazo et/ou naphtylazo, qui peut à chaque fois être substitué par C₁-C₁₀-alkyle, C₁-C₆-alcoxy ou cyano ;
R' des radicaux identiques :
hydrogène ; brome ;
phénoxy, phénylthio, pyridyloxy, pyrimidyloxy, pyridylthio ou pyrimidylthio, qui peut à chaque fois être monosubstitué ou polysubstitué par C₁-C₁₂-alkyle ou C₁-C₁₂-alcoxy ;
R¹ hydrogène ou C₁-C₆-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle ;
n 1 ou 2.

3. Procédé pour la préparation de diimides d'acide hexarylènetétracarboxylique de formule générale Ia 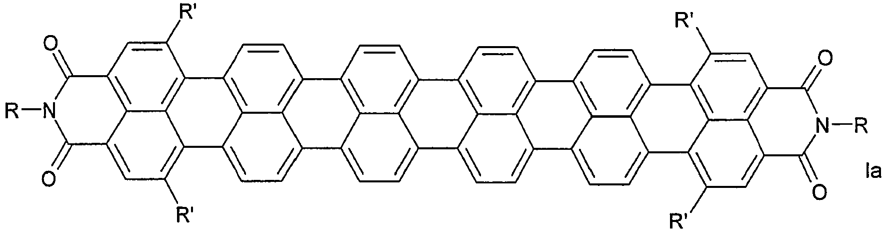 dans laquelle les variables présentent la signification mentionnée dans la revendication 1, R' ne signifiant cependant pas hydrogène, **caractérisé en ce que**
a) un diborane de formule générale II 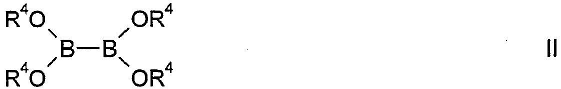 dans laquelle les radicaux R⁴ sont identiques ou différents et signifient, indépendamment les uns des autres, hydrogène, C₁-C₃₀-alkyle, C₅-C₈-cycloalkyle, aryle ou hétéroaryle, les radicaux R⁴ se trouvant sur à chaque fois un atome de bore pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle ;
est transformé, en présence d'un solvant organique aprotique, d'un catalyseur à base d'un métal de transition et d'une base, avec
a1) un imide d'acide 9-bromopérylène-3,4-dicarboxylique de formule générale IIIa 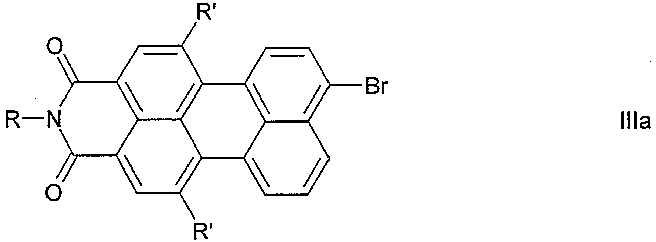 ou
a2) un dérivé de naphtalène de formule générale IIIB 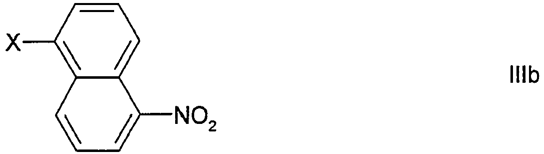 dans laquelle X signifie halogène ; C₁-C₁₂-alkylsulfonyle dont le radical alkyle peut être monosubstitué ou polysubstitué par halogéno ; ou C₆-C₁₈-arylsulfonyle,
b1) l'imide d'acide 9-(dioxaborolan-2-yl)pérylène-3,4-dicarboxylique formé dans l'étape a1) de formule générale IVa 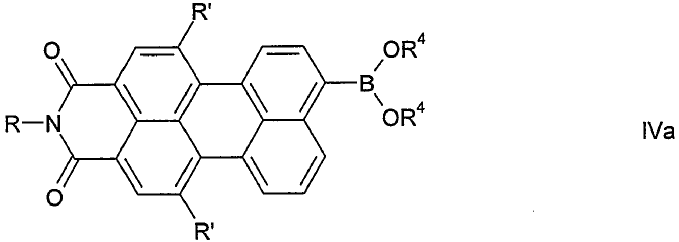 est soumis, en présence d'un solvant organique, si souhaité en mélange avec de l'eau, ainsi que d'un catalyseur à base d'un métal de transition et d'une base, à une réaction de couplage de Suzuki avec un dérivé de naphtalène IIIb ou
b2) le 1-(dioxaborolan-2-yl)-5-nitronaphtalène formé dans l'étape a2) de formule générale IVb 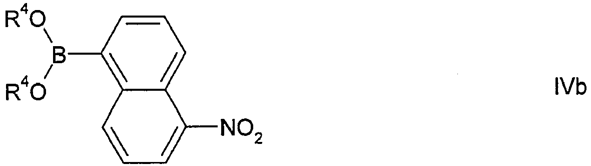 est soumis, présence d'un solvant organique, si souhaité en mélange avec de l'eau, ainsi que d'un catalyseur à base d'un métal de transition et d'une base, à une réaction de couplage de Suzuki avec un imide d'acide 9-bromopérylène-3,4-dicarboxylique IIIa,
c) l'imide d'acide 9-(5-nitronaphtyl)pérylène-3,4-dicarboxylique formé dans l'étape b1) ou b2) de formule générale V 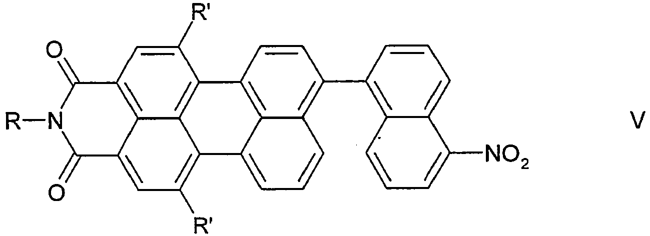 est soumis à une cyclodéshydrogénation en présence d'un solvant organique stable aux bases, à point d'ébullition élevé, ainsi que d'une base contenant un métal alcalin ou un métal alcalino-terreux et d'une base auxiliaire azotée,
d) l'imide d'acide 11-nitroterrylène-3,4-dicarboxylique formé dans l'étape c) de formule générale VIa 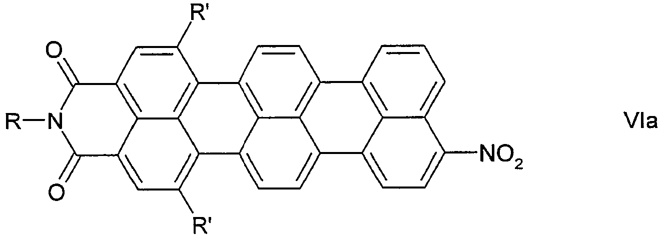 est réduit avec de l'hydrogène naissant,
e) l'imide d'acide 11-aminoterrylène-3,4-dicarboxylique formé dans l'étape d) de formule générale VIb 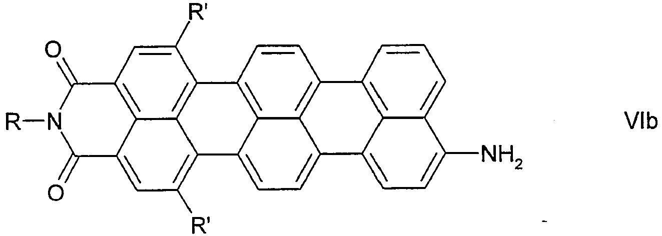 est diazoté et le sel de diazonium formé est transformé avec un iodure ou un bromure de métal,
f) l'imide d'acide 11-halogénoterrylène-3,4-dicarboxylique formé dans l'étape e) de formule générale VIc 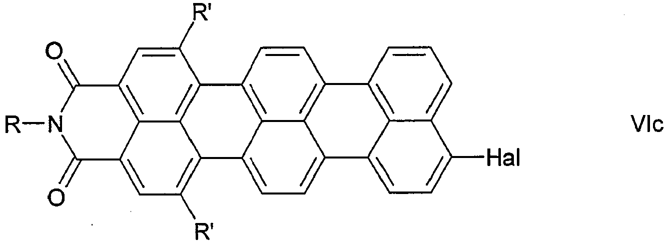 dans laquelle Hal signifie brome ou iode,
f1) est couplé, en présence d'un complexe de métal de transition organique comme catalyseur, de molécules de ligand libres et d'un solvant aprotique, en un dérivé de bisterrylène de formule générale VII 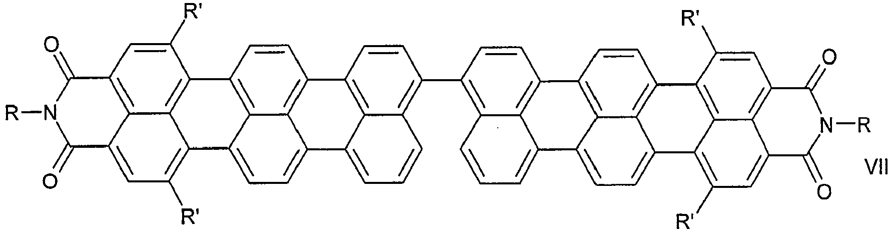 ou
f2) est transformé, en présence de 30 à 70% en mole, par rapport à l'imide d'acide 11-halogénoterrylène-3,4-dicarboxylique VIc, d'un diborane II, d'un catalyseur à base d'un métal de transition, d'une base et d'un solvant organique aprotique, sans isolement intermédiaire de l'imide d'acide 11-(dioxaborolan-2-yl)terrylène-3,4-dicarboxylique formé in situ de formule générale VId 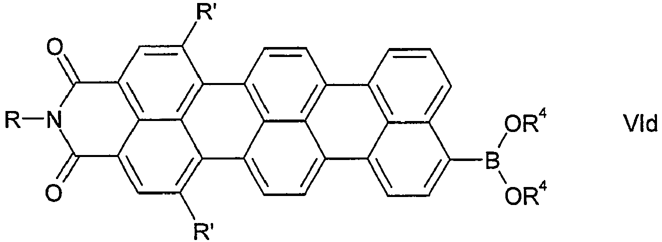 selon une réaction de couplage de Suzuki en dérivé de bis-terrylène VII et
g) le dérivé de bis-terrylène VII est transformé par cyclodéshydrogénation
g1) dans un milieu réactionnel organique présentant des fonctions hydroxy et amino et contenant une base essentiellement non dissoute ou
g2) en présence d'un solvant organique stable aux bases, à point d'ébullition élevé, ainsi que d'une base contenant un métal alcalin ou un métal alcalino-terreux et d'une base auxiliaire azotée,
en diimide d'acide hexarylènetétracarboxylique Ia.

4. Procédé pour la préparation de diimides d'acide hexarylènetétracarboxylique de formule générale Ia 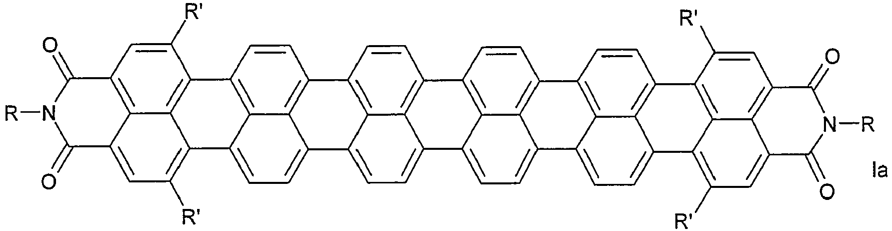 dans laquelle les variables présentent la signification mentionnée dans la revendication 1, R' ne signifiant cependant pas hydrogène, **caractérisé en ce que**
a) un diborane de formule générale II 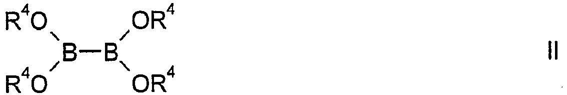 dans laquelle les radicaux R⁴ sont identiques ou différents et signifient, indépendamment les uns des autres, hydrogène, C₁-C₃₀-alkyle, C₅-C₈-cycloalkyle, aryle ou hétéroaryle, les radicaux R⁴ se trouvant sur à chaque fois un atome de bore pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle ;
est transformé, en présence d'un solvant organique aprotique, d'un catalyseur à base d'un métal de transition et d'une base, avec un imide d'acide 11-halogénoterrylène-3,4-dicarboxylique de formule générale VIc 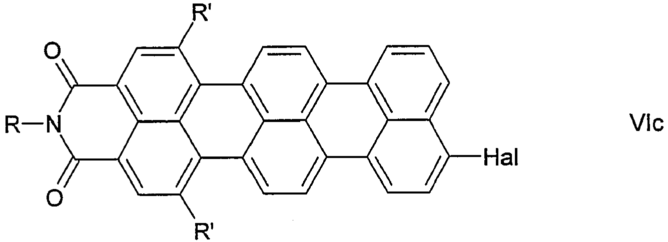 dans laquelle Hal signifie brome ou iode,
b) l'imide d'acide 11-(dioxaborolan-2-yl)terrylène-3,4-dicarboxylique formé dans l'étape a) de formule générale VId 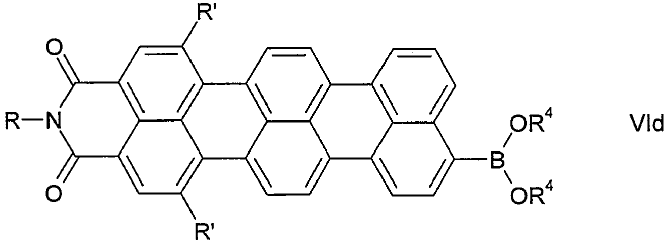 est soumis, en présence d'un solvant organique, si souhaité en mélange avec de l'eau, ainsi que d'un catalyseur à base d'un métal de transition et d'une base, à une réaction de couplage de Suzuki avec un imide d'acide 11-halogénoterrylène-3,4-dicarboxylique VIc et
c) le dérivé de bisterrylène formé dans l'étape b) de formule générale VII 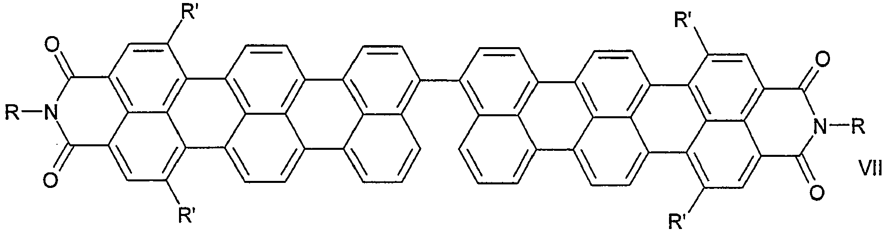 est transformé par cyclodéshydrogénation
c1) dans un milieu réactionnel organique présentant des fonctions hydroxy et amino et contenant une base essentiellement non dissoute ou
c2) en présence d'un solvant organique stable aux bases, à point d'ébullition élevé, ainsi que d'une base contenant un métal alcalin ou un métal alcalino-terreux et d'une base auxiliaire azotée,
en diimide d'acide hexarylènetétracarboxylique Ia.

5. Procédé pour la préparation de diimides d'acide pentarylènetétracarboxylique de formule générale Ib 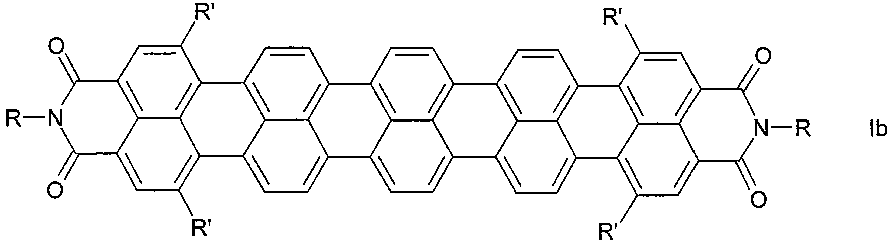 dans laquelle les variables présentent la signification mentionnée dans la revendication 1, R' ne signifiant cependant pas hydrogène, **caractérisé en ce que**
a) un imide d'acide 11-halogénoterrylène-3,4-dicarboxylique de formule générale VIc 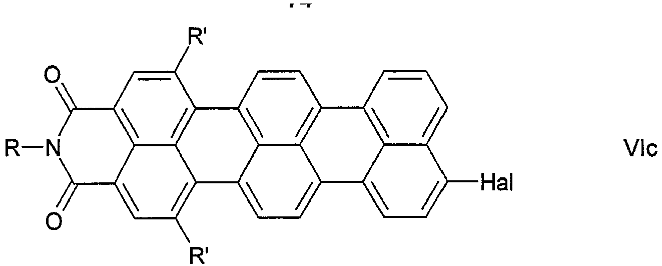 dans laquelle Hal signifie brome ou iode, est soumis, en présence d'un solvant organique, si souhaité en mélange avec de l'eau, ainsi que d'un catalyseur à base d'un métal de transition et d'une base, à une réaction de couplage de Suzuki avec un imide d'acide 9-(dioxaborolan-2-yl)pérylène-3,4-dicarboxylique de formule IVa 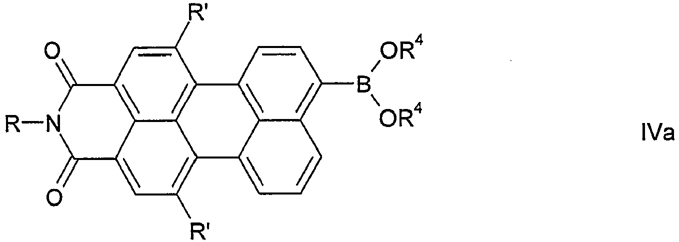 dans laquelle les radicaux R⁴ sont identiques ou différents et signifient, indépendamment l'un de l'autre, hydrogène, C₁-C₃₀-alkyle, C₅-C₈-cycloalkyle, aryle ou hétéroaryle, les deux radicaux R⁴ pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle, et
b) l'imide d'acide 11-(imide d'acide 9-pérylène-3,4-dicarboxylique)terrylène-3,4-dicarboxylique formé dans l'étape a) de formule générale VIII 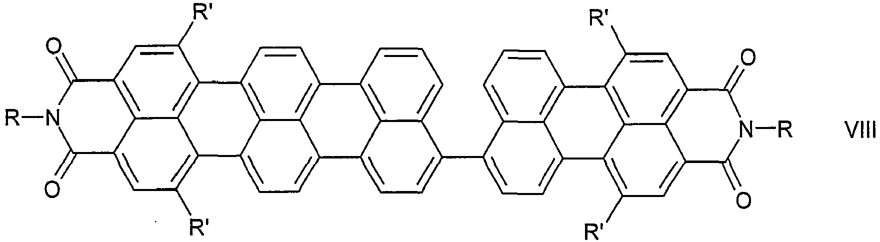 est transformé par cyclodéshydrogénation
b1) dans un milieu réactionnel organique présentant des fonctions hydroxy et amino et contenant une base essentiellement non dissoute ou
b2) en présence d'un solvant organique stable aux bases, à point d'ébullition élevé, ainsi que d'une base contenant un métal alcalin ou un métal alcalino-terreux et d'une base auxiliaire azotée,
en diimide d'acide pentarylènetétracarboxylique Ib.

6. Procédé pour la préparation de diimides d'acide pentarylènetétracarboxylique de formule générale Ib 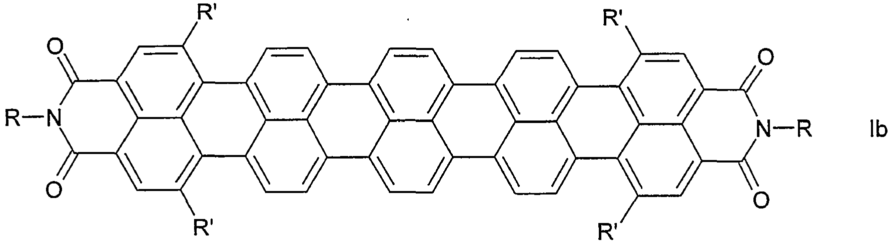 dans laquelle les variables présentent la signification mentionnée dans la revendication 1, R' ne signifiant cependant pas hydrogène, **caractérisé en ce que**
a) un imide d'acide 11-(dioxaborolan-2-yl)terrylène-3,4-dicarboxylique de formule générale VId 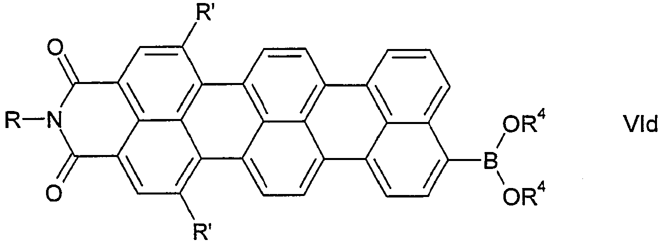 dans laquelle les radicaux R⁴ sont identiques ou différents et signifient, indépendamment l'un de l'autre, hydrogène, C₁-C₃₀-alkyle, C₅-C₈-cycloalkyle, aryle ou hétéroaryle, les deux radicaux R⁴ pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle, est soumis, en présence d'un solvant organique, si souhaité en mélange avec de l'eau, ainsi que d'un catalyseur à base d'un métal de transition et d'une base, à une réaction de couplage de Suzuki avec un imide d'acide 9-bromopérylène-3,4-dicarboxlique de formule générale IIIa 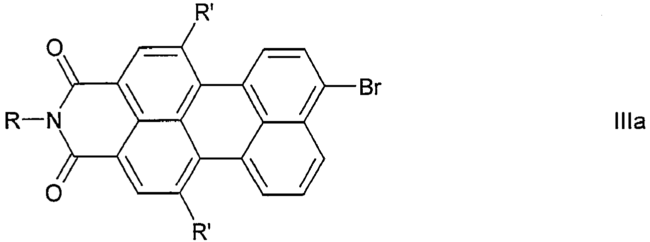 et
b) l'imide d'acide 11-(imide d'acide 9-pérylène-3,4-dicarboxylique)terrylène-3,4-dicarboxylique de formule générale VIII formé dans l'étape a) 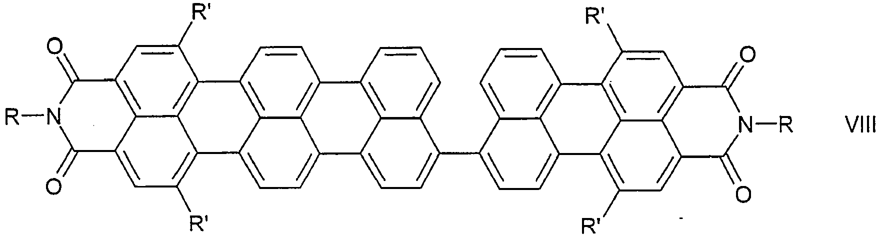 est transformé par cyclodéshydrogénation
b1) dans un milieu réactionnel organique présentant des fonctions hydroxy et amino et contenant une base essentiellement non dissoute ou
b2) en présence d'un solvant organique stable aux bases, à point d'ébullition élevé, ainsi que d'une base contenant un métal alcalin ou un métal alcalino-terreux et d'une base auxiliaire azotée,
en diimide d'acide pentarylènetétracarboxylique Ib.

7. Dérivés de bisterrylène de formule générale VII 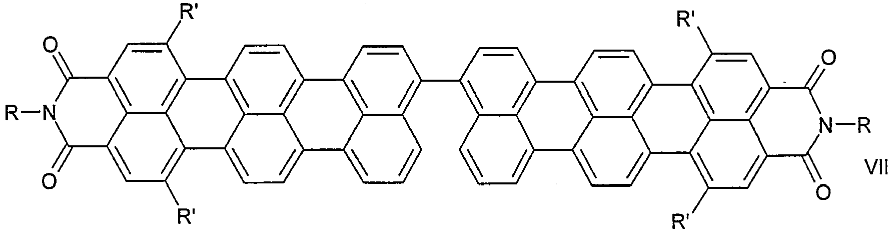 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

8. Imides d'acide 11-(imide d'acide 9-pérylène-3,4-dicarboxylique)terrylène-3,4-dicarboxylique de formule générale VIII 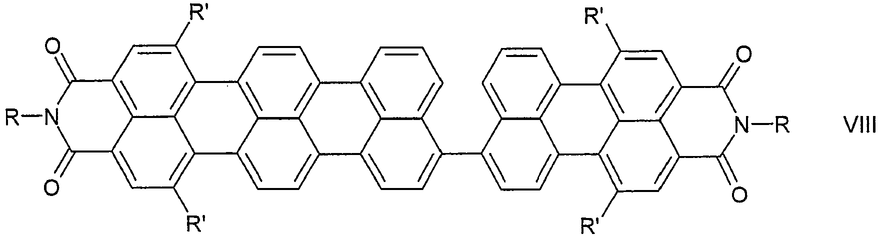 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

9. Imides d'acide terrylène-3,4-dicarboxylique de formule générale VI 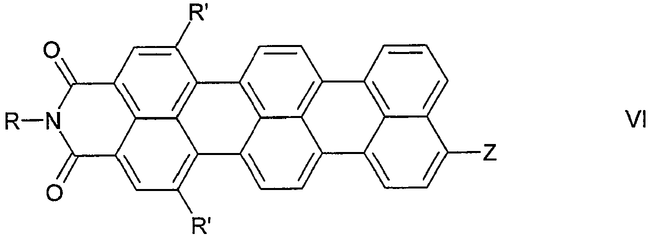 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
Z brome, iode, amino, nitro ou un radical 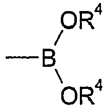
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle ;
R⁴ des radicaux identiques ou différents :
hydrogène, C₁-C₃₀-alkyle, C₅-C₈-cycloalkyle, aryle ou hétéroaryle, les deux radicaux R⁴ pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle.

10. Imides d'acide 9-(5-nitronaphtyl)pérylène-3,4-dicarboxylique de formule générale V 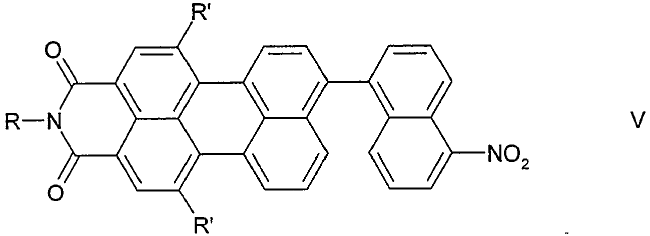 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents : aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

11. Procédé pour la préparation de diimides d'acide hexarylènetétracarboxylique de formule générale Ia 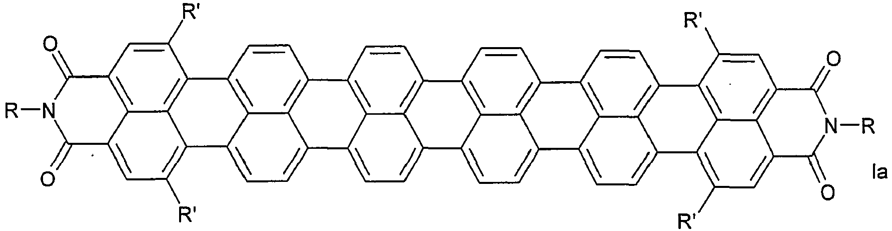 dans laquelle les variables présentent la signification mentionnée dans la revendication 1, **caractérisé en ce que**
a) un diborane de formule générale II 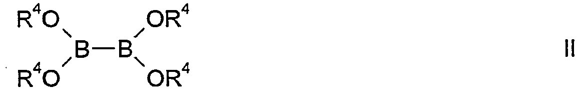 dans laquelle les radicaux R⁴ sont identiques ou différents et signifient, indépendamment les uns des autres, hydrogène, C₁-C₃₀-alkyle, C₅-C₈-cycloalkyle, aryle ou hétéroaryle, les radicaux R⁴ se trouvant sur à chaque fois un atome de bore pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle ;
est transformé, en présence d'un solvant organique aprotique, d'un catalyseur à base d'un métal de transition et d'une base, avec
a1) un imide d'acide 9-bromopérylène-3,4-dicarboxylique de formule générale IIIa 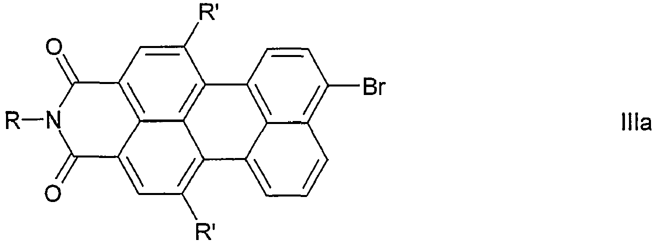 ou
a2) un dihalogénopérylène de formule générale IXa 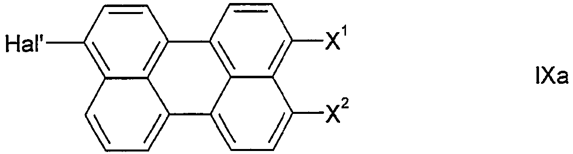 dans laquelle Hal' représente chlore ou brome et un des deux radicaux X¹ ou X² signifie également Hal' et l'autre radical signifie hydrogène,
b1) l'imide d'acide 9-(dioxaborolan-2-yl)pérylène-3,4-dicarboxylique formé dans l'étape a1) de formule générale IVa 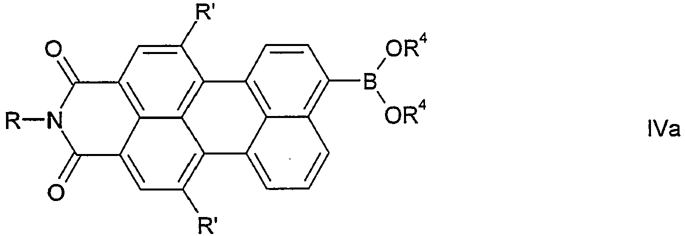 est soumis, en présence d'un solvant organique, si souhaité en mélange avec de l'eau, ainsi que d'un catalyseur à base d'un métal de transition et d'une base, à une réaction de couplage de Suzuki avec un dihalogénopérylène IXa dans un rapport molaire 2:1 à 6:1 ou
b2) le bis(dioxaborolan-2-yl)pérylène formé dans l'étape a2) de formule générale IXb 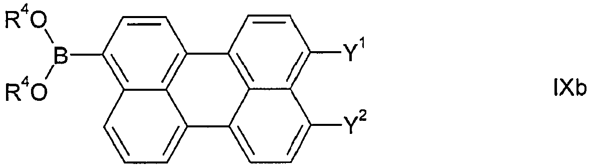 dans laquelle un des deux radicaux Y¹ ou Y² signifie également un radical 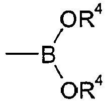 et l'autre radical signifie hydrogène est soumis, en présence d'un solvant organique, si souhaité en mélange avec de l'eau, ainsi que d'un catalyseur à base d'un métal de transition et d'une base, à une réaction de couplage de Suzuki avec un imide d'acide 9-bromopérylène-3,4-dicarboxylique IIIa dans un rapport molaire 2:1 à 6:1 et
c) le pérylène-3,9-bis(imide d'acide pérylène-3,4-dicarboxylique) formé dans l'étape b1) ou b2) de formule générale Xa 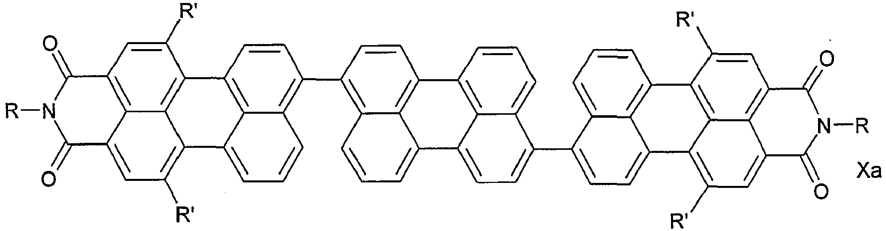 ou le pérylène-3,10-bis(imide d'acide pérylène-3,4-dicarboxylique) de formule générale Xb 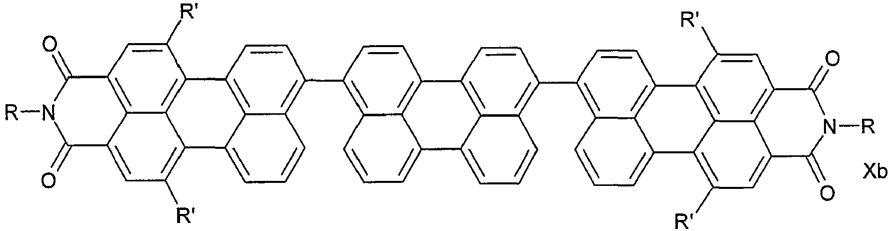
c1) est soumis à une cyclodéshydrogénation en une étape, en présence d'un acide de Lewis fort et d'un solvant organique inerte, directement en diimide d'acide hexarylènetétracarboxylique Ia ou
c2a) est mis en contact, dans une première étape, à température ambiante, en présence d'un solvant organique inerte, avec un acide de Lewis faible et
c2b) l'imide d'acide 13-(imide d'acide 9-pérylène-3,4-dicarboxylique)quaterrylène-3,4-dicarboxylique de formule générale XI 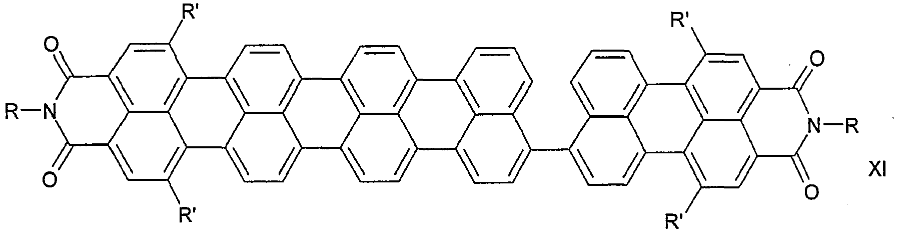 est ensuite cyclodéshydrogéné davantage, après isolement intermédiaire, dans une deuxième étape
c2bα) dans un milieu réactionnel organique présentant
des fonctions hydroxy et amino et contenant une base essentiellement non dissoute ou
c2bβ) en présence d'un solvant organique stable aux bases, à point d'ébullition élevé, ainsi que d'une base contenant un métal alcalin ou un métal alcalino-terreux et d'une base auxiliaire azotée,
en diimide d'acide hexarylènetétracarboxylique Ia.

12. Procédé pour la préparation de diimides d'acide pentarylènetétracarboxylique de formule générale Ib 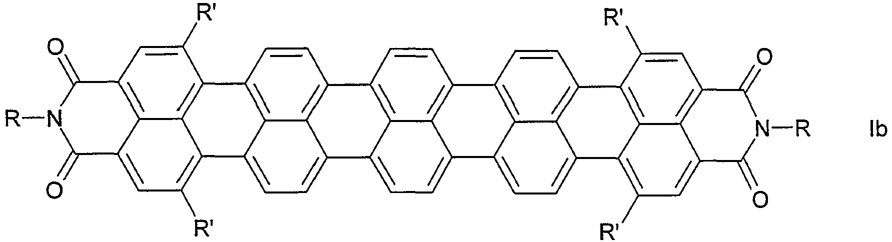 dans laquelle les variables présentent la signification mentionnée dans la revendication 1, **caractérisé en ce que**
a) un diborane de formule générale II 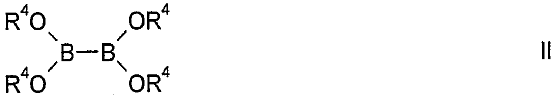 dans laquelle les radicaux R⁴ sont identiques ou différents et signifient, indépendamment les uns des autres, hydrogène, C₁-C₃₀-alkyle, C₅-C₈-cycloalkyle, aryle ou hétéroaryle, les radicaux R⁴ se trouvant sur à chaque fois un atome de bore pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle ;
est transformé, en présence d'un solvant organique aprotique, d'un catalyseur à base d'un métal de transition et d'une base, avec
a1) un imide d'acide 9-bromopérylène-3,4-dicarboxylique de formule générale IIIa 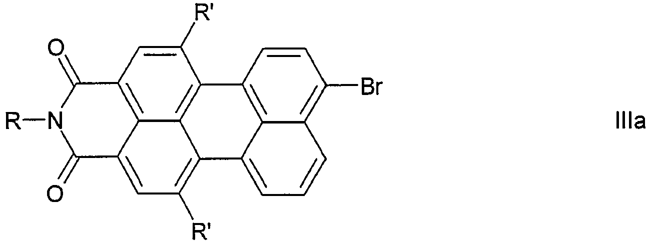 ou
a2) un dihalogénonaphtalène de formule générale IXc 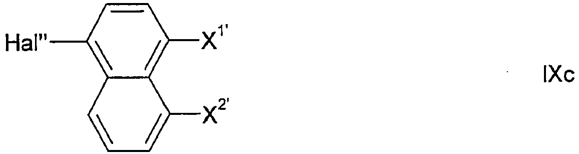 dans laquelle Hal" représente chlore, brome ou iode et un des deux radicaux X^{1'} ou X^{2'} signifie également Hal" et l'autre radical signifie hydrogène,
b1) l'imide d'acide 9-(dioxaborolan-2-yl)pérylène-3,4-dicarboxylique formé dans l'étape a1) de formule générale IVa 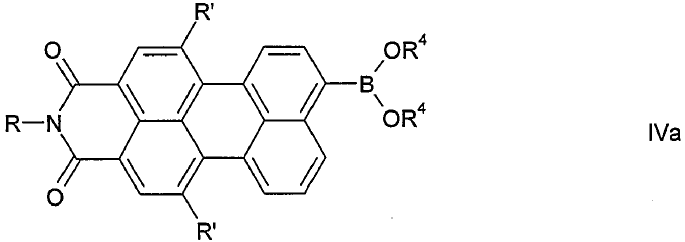 est soumis, en présence d'un solvant organique, si souhaité en mélange avec de l'eau, ainsi que d'un catalyseur à base d'un métal de transition et d'une base, à une réaction de couplage de Suzuki avec un dihalogénonaphtalène dans un rapport molaire 2:1 à 6:1 ou
b2) le bis(dioxaborolan-2-yl)naphtalène formé dans l'étape a2) de formule générale IXd 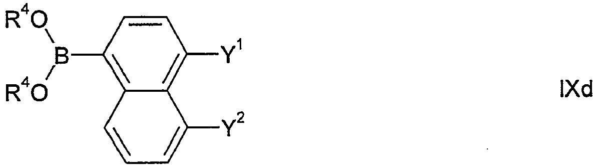 dans laquelle un des deux radicaux Y¹ ou Y² signifie également un radical 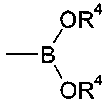 et l'autre radical signifie hydrogène est soumis, en présence d'un solvant organique, si souhaité en mélange avec de l'eau, ainsi que d'un catalyseur à base d'un métal de transition et d'une base, à une réaction de couplage de Suzuki avec un imide d'acide 9-bromopérylène-3,4-dicarboxylique IIIa dans un rapport molaire 2:1 à 6:1 et
c) le naphtalène-1,5-bis(imide d'acide pérylène-3,4-dicarboxylique) formé dans l'étape b1) ou b2) de formule générale XIIa 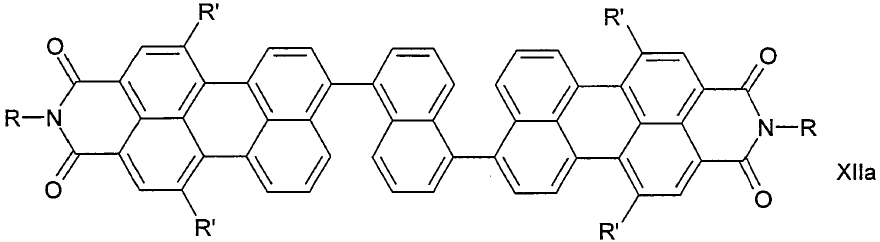 ou le naphtalène-1,4-bis(imide d'acide pérylène-3,4-dicarboxylique) de formule générale XIIb 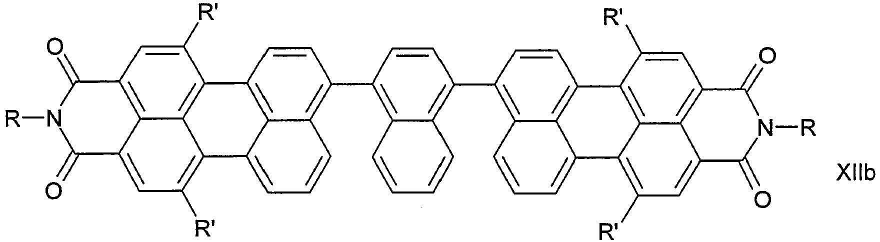
c1) est soumis à une cyclodéshydrogénation en une étape, en présence d'un acide de Lewis fort et d'un solvant organique inerte, directement en diimide d'acide pentarylènetétracarboxylique Ib ou
c2a) est mis en contact, dans une première étape, à température ambiante, en présence d'un solvant organique inerte, avec un acide de Lewis faible et
c2b) l'imide d'acide 11-(imide d'acide 9-pérylène-3,4-dicarboxylique)terrylène-3,4-dicarboxylique de formule générale VIII 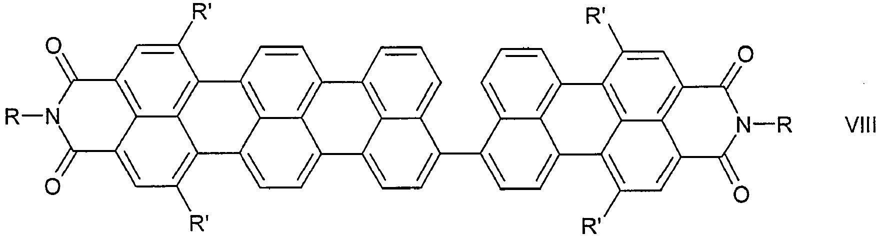 est ensuite cyclodéshydrogéné davantage, après isolement intermédiaire, dans une deuxième étape
c2bα) dans un milieu réactionnel organique présentant des fonctions hydroxy et amino et contenant une base essentiellement non dissoute ou
c2bβ) en présence d'un solvant organique stable aux bases, à point d'ébullition élevé, ainsi que d'une base contenant un métal alcalin ou un métal alcalino-terreux et d'une base auxiliaire azotée, en diimide d'acide pentarylènetétracarboxylique Ib.

13. Imides d'acide 13-(imide d'acide 9-pérylène-3,4-dicarboxylique)quaterrylène-3,4-dicarboxylique de formule générale XI 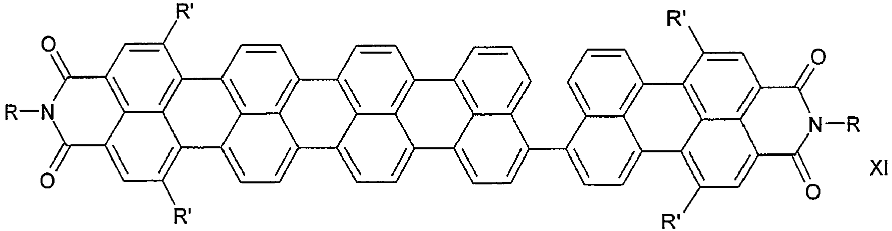 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

14. Pérylène-3,9-bis(imides d'acide pérylène-3,4-dicarboxylique) de formule générale Xa 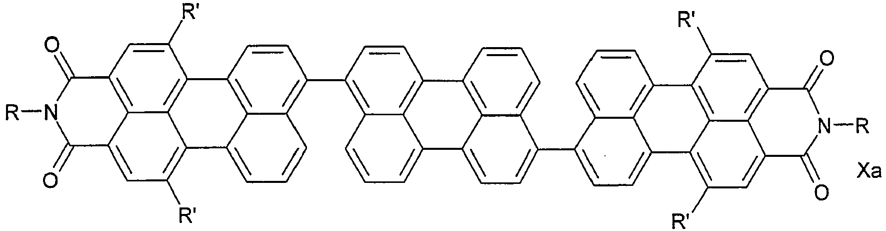 ou pérylène-3,10-bis(imides d'acide pérylène-3,4-dicarboxylique) de formule générale Xb 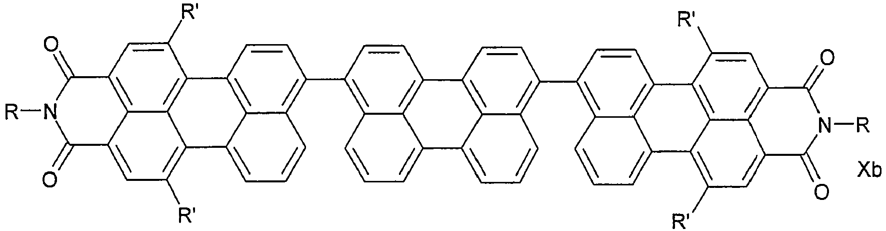 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par C₁-C₁₈-alkyle ; C₁-C₁₈-alcoxy ; cyano ; nitro ; halogéno ; -NR²R³ ; -CONR²R³ ; -SO₂R² ; et/ou arylazo ou hétéroarylazo, qui peut à chaque fois être substitué par C₁-C₁₈-alkyle, C₁-C₁₈-alcoxy ou cyano ; et sur lequel à chaque fois d'autres cycles de 5 à 7 chaînons, saturés ou insaturés peuvent être condensés, qui peuvent contenir -O-, -S-, -NR¹-, -CO- et/ou -SO₂-comme chaînons de cycle et/ou qui peuvent être substitués par un ou plusieurs radicaux R² identiques ou différents ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

15. Bis(dioxaborolan-2-yl)pérylènes de formule générale IXb 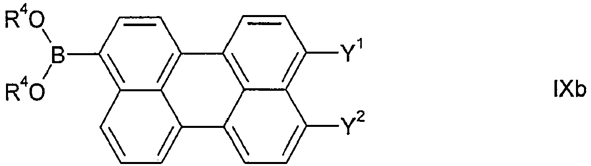 dans laquelle les variables ont la signification suivante :
R⁴ des radicaux identiques ou différents :
hydrogène, C₁-C₃₀-alkyle, C₅-C₈-cycloalkyle, aryle ou hétéroaryle, les deux radicaux R⁴ pouvant également être reliés l'un à l'autre en formant un cycle à cinq chaînons contenant les deux atomes d'oxygène ainsi que l'atome de bore, qui peut être substitué sur les atomes de carbone par jusqu'à 4 groupes C₁-C₃₀-alkyle, C₅-C₈-cycloalkyle, aryle ou hétéroaryle ;
un des deux radicaux Y¹ ou Y² signifiant également un radical 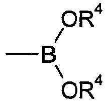
l'autre radical représentant hydrogène.

16. Naphtalène-1,5-bis(imides d'acide pérylène-3,4-dicarboxylique) de formule générale XIIa 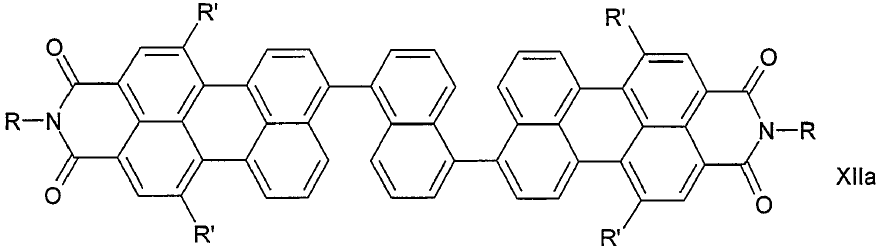 ou naphtalène-1,4-bis(imides d'acide pérylène-3,4-dicarboxyliques) de formule générale XIIb 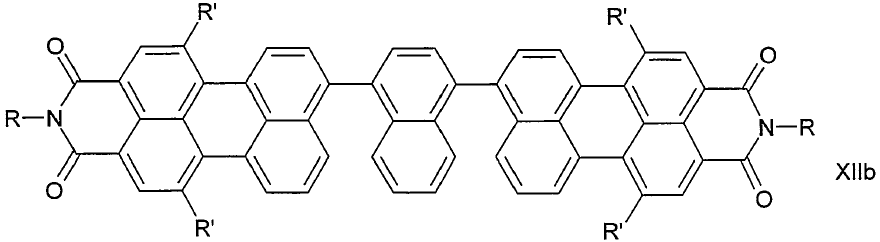 dans laquelle les variables ont la signification suivante :
R des radicaux identiques ou différents :
hydrogène ;
C₁-C₃₀-alkyle, dont la chaîne carbonée peut être interrompue par un ou plusieurs groupements -O-, -S-, -NR¹-, -CO- et/ou -SO₂- et qui peut être monosubstitué ou polysubstitué par cyano ; C₁-C₆-alcoxy ; aryle, qui peut être substitué par C₁-C₁₈-alkyle ou C₁-C₆-alcoxy ; et/ou un radical hétérocyclique lié via un atome d'azote, de 5 à 7 chaînons, qui peut contenir d'autres hétéroatomes et qui peut être aromatique ;
C₅-C₈-cycloalkyle, dont la structure carbonée peut être interrompue par un ou plusieurs groupements -O-, -S- et/ou -NR¹- et/ou qui peut être monosubstitué ou polysubstitué par C₁-C₁₈-alkyle ; aryle ou hétéroaryle, qui peut à chaque fois être monosubstitué ou polysubstitué par : C₁-C₁₈-alkyle ; C₁-C₁₈-alcoxy ; C₁-C₁₈-alcoxy ; cyano ; nitro ; halogéno ; -NR²R³, -CONR²R³ ; -SO₂R² ; et/ou arylazo et/ou hétéroarylazo, qui peut à chaque fois être substitué par C₁-C₁₂-alkyle, C₁-C₁₈-alcoxy ou cyano ;
R' des radicaux identiques ou différents :
hydrogène ;
aryloxy, arylthio, hétéroaryloxy ou hétéroarylthio, qui peut à chaque fois être monosubstitué ou polysubstitué par des radicaux alkyle R, des radicaux aryle R, C₁-C₁₂-alcoxy, cyano, halogéno, hydroxy, -COOR¹, -CONR²R³ et/ou -NHCOR² ;
R¹ hydrogène ou C₁-C₁₈-alkyle ;
R², R³ indépendamment l'un de l'autre, hydrogène ; C₁-C₁₈-alkyle, qui peut être substitué par C₁-C₆-alcoxy, halogéno, hydroxy, carboxy et/ou cyano ; aryle ou hétéroaryle, qui peut à chaque fois être substitué par les radicaux ci-dessus mentionnés pour alkyle ainsi que par C₁-C₆-alkyle.

17. Utilisation de diimides d'acide rylènetétracarboxylique de formule I selon la revendication 1 ou 2 pour teinter des matériaux organiques et inorganiques de haut poids moléculaire.

18. Utilisation selon la revendication 17, **caractérisée en ce que** les matériaux de haut poids moléculaire sont des laques, des encres d'imprimerie ou des matériaux synthétiques.

19. Utilisation de diimides d'acide rylènetétracarboxylique de formule I selon la revendication 1 ou 2 pour la préparation de dispersions aqueuses de polymères absorbant dans la plage infrarouge proche du spectre électromagnétique.

20. Utilisation de diimides d'acide rylènetétracarboxylique de formule I selon la revendication 1 ou 2 pour obtenir des marquages et des inscriptions absorbant la lumière infrarouge, invisibles pour l'oeil humain.

21. Utilisation de diimides d'acide rylènetétracarboxylique de formule I selon la revendication 1 ou 2 comme absorbant d'infrarouges pour la gestion de la chaleur.

22. Utilisation de diimides d'acide rylènetétracarboxylique de formule I selon la revendication 1 ou 2 comme matériaux absorbant les rayons laser IR lors du traitement par soudure de pièces en matériau synthétique.

23. Utilisation de diimides d'acide rylènetétracarboxylique de formule I selon la revendication 1 ou 2 comme composants actifs dans la photovoltaïque.
